(19) Europäisches Patentamt
European Patent Office
Office européen des brevets

(11) EP 0 848 957 A1

(12) **EUROPEAN PATENT APPLICATION**
published in accordance with Art. 158(3) EPC

(43) Date of publication:
**24.06.1998 Bulletin 1998/26**

(21) Application number: **96929510.4**

(22) Date of filing: **04.09.1996**

(51) Int. Cl.[6]: **A61K 45/00**, A61K 31/165, A61K 31/47, A61K 31/55, C07D 487/04, C07C 239/18

(86) International application number:
**PCT/JP96/02492**

(87) International publication number:
**WO 97/09066 (13.03.1997 Gazette 1997/12)**

(84) Designated Contracting States:
**AT BE CH DE DK ES FI FR GB GR IE IT LI LU NL PT SE**

(30) Priority: **08.09.1995 JP 256897/95**
**09.11.1995 JP 317136/95**

(71) Applicant: **KANEBO LTD.**
**Sumida-ku, Tokyo 131 (JP)**

(72) Inventors:
- **HIRANO, Takao**
  **Nakano-ku, Tokyo 165 (JP)**
- **YAGITA, Hideo**
  **Itabashi-ku, Tokyo 174 (JP)**
- **OKUMURA, Ko**
  **Chiba-shi, Chiba 260 (JP)**
- **HIRAYAMA, Ryoichi**
  **Akashi-shi, Hyogo 674 (JP)**
- **YAMAMOTO, Minoru**
  **Miyakojima-ku, Osaka-shi, Osaka 534 (JP)**
- **EBATA, Tomohiko**
  **Osaka-shi, Osaka-fu (JP)**
- **OHMOTO, Hiroshi**
  **Osaka-shi, Osaka 534 (JP)**
- **IKEDA, Shoji**
  **Kobe-shi, Hyogo 658 (JP)**
- **YOSHINO, Kohichiro**
  **Suita-shi, Osaka 565 (JP)**

(74) Representative:
**Kolb, Helga, Dr. Dipl.-Chem. et al**
**Hoffmann Eitle,**
**Patent- und Rechtsanwälte,**
**Arabellastrasse 4**
**81925 München (DE)**

# (54) Fas LIGAND SOLUBILIZATION INHIBITOR

(57) A Fas ligand solubilization inhibitor which comprises, as the active ingredient, a compound of the generic formula (I) having a matrix metalloproteinase inhibitory activity or its pharmaceutically acceptable salt. The drug is useful in the prevention or treatment of diseases caused by solubilized Fas ligand, such as hepatitis, GVHD, AIDS, autoimmune diseases, etc.

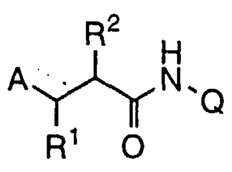

(I)

EP 0 848 957 A1

## Description

### TECHNICAL FIELD

Fas is a type I transmembrane protein which belongs to a TNF (tumor necrosis factor) receptor family [N. Itoh et al. Cell, 66, 233-243 (1991)] and is expressed at active leukocyte, liver, lung, intestine, skin, etc. [F. Leithhauser et al. Lab. Invest., 69, 415-429 (1993). S.Nagata, Adv. Immunol.,57, 129-144 (1994)], and it is a receptor which causes death of cells by apoptosis.

On the other hand, a ligand of Fas (Fas L) is a type II transmembrane protein which belongs to a TNF family [(T. Suda et al. Cell, 75, 1169-1178 (1995)] and is expressed at active T cell and NK cell. A soluble Fas L (sFas L) released from these cells binds with the Fas to cause apoptosis. This is one of mechanisms of cell cytotoxicity by T cell. [M. Tanaka et al. EMBO J., 14, 1129-1135 (1995)] The excessive apoptosis caused by the excessively activated T cell participates in the causes of fulminant hepatitis, graft-versus-host diseases (GVHD), acquired immunodeficiency syndrome (AIDS), autoimmune diseases, etc.

The present invention relates to a drug useful for the prevention and/or treatment of diseases caused by inhibiting the solubilization of Fas L and by preventing the release of sFas L, that is, to a Fas L solubilization inhibitor. In more detail, the present invention relates to a Fas L solubilization inhibitor which comprises a compound having a matrix metalloproteinase (MMP) inhibitory activity as an active ingredient.

On the other hand, an inhibitor for the production of sFas L is meaningful as a reagent in the study of the function of Fas L and the diagnosis or study of the above diseases. For example, a inhibitor for the production of sFas L is important as a reagent to make a quantitative analysis of the amount of Fas L which is expressed on the cell or to test its function. The present invention relates to a reagent for an inhibitor of solubilization of Fas L useful for such the diagnosis and study, too.

In this specification, both a Fas L solubilization inhibitor and a reagent consisting of a Fas L solubilization inhibitor are said a Fas L solubilization inhibitor, as long as not specified.

### BACKGROUND OF ART

Matrix metalloproteinases (MMPs) are proteases which participate in methabolization of connective tissue matrix, and many compounds which inhibit their proteases activity are known. The known compounds are illustrated as follows. The compound numbers are corresponding to ones of compounds mentioned later which are included in this invention.

Compound 1. [4-(N-hydroxyamino)-2(R)-isobutylsuccinyl]-L-phenylglycine-N-methylamide
Compound 2. [4-(N-hydroxyamino)-2(R)-isobutylsuccinyl]-L-p-hydroxyphenylglycine-N-methylamide
Compound 3. [4-(N-hydroxyamino)-2(R)-isobutyl-3 morpholinomethylsuccinyl]-L-phenylglycine-N-methylamide
Compound 4. [4-(N-hydroxyamino)-2(R)-isobutyl-3-methylsuccinyl]-L-phenylglycine-N-methylamide
Compound 5. [4-(N-hydroxyamino)-2(R)-isobutyl-3-methylaminomethylsuccinyl]-L-phenylglycine-N-methylamide
Compound 6. [4-(N-hydroxyamino)-2(R)-isobutylsuccinyl]-L-p-isopropylphenylglycine-N-methylamide
Compound 7. [4-(N-hydroxyamino)-2(R)-isobutylsuccinyl]-L-phenylglycine-N-ethylamide
Compound 8. [4-(N-hydroxyamino)-2(R)-isobutylsuccinyl]-L-p-fluorophenylglycine-N-methylamide

See PCT Publication No. WO 95/04715 on the above compound Nos. 1-8.

Compound 23. [4-(N-hydroxyamino)-2(R)-isobutylsuccinyl]-L-O-methyltyrosine-N-methylamide
Compound 24. [4-(N-hydroxyamino)-2(R)-isobutyl-3(S)-methylsuccinyl]-L-O-methyltyrosine-N-methylamide
Compound 25. [4-(N-hydroxyamino)-2(R)-cyclohexylmethylsuccinyl]-L-O-methyltyrosine-N-methylamide
Compound 26. [4-(N-hydroxyamino)-2(R)-isobutylsuccinyl]-L-phenylalanine-N-methylamide
Compound 27. [4-(N-hydroxyamino)-2(R)-isopropylsuccinyl]-L-tyrosine-N-methylamide

See US Patent No. 4,743,587 on the above compound Nos. 23-27.

Compound 28. [4-(N-hydroxyamino)-2(R)-isobutylsuccinyl]-L-tryptophan-N-methylamide
Compound 29. [4-(N-hydroxyamino)-2(R)-isobutylsuccinyl]-L-tryptophan-N-(1(S)-phenyl)ethylamide
Compound 30. [4-(N-hydroxyamino)-2(R)-isobutylsuccinyl]-L-tryptophan-N-(2-hydroxy)ethylamide

See PCT Publication No. WO 92/09556 on the above compound Nos. 28-30.

Compound 31. [4-(N-hydroxyamino)-3-methylthiomethyl-2-propoxymethylsuccinyl]-L-O-methyltyrosine-N-methylamide

Compound 32. [4-(N-hydroxyamino)-3-methyl-2-propoxymethylsuccinyl]-L-O-methyltyrosine-N-methylamide
Compound 33. [4-(N-hydroxyamino)-3-acetylthiomethyl-2-propoxymethylsuccinyl]-L-O-methyltyrosine-N-methylamide
Compound 34. [4-(N-hydroxyamino)-3-methyl-2-propylthiosuccinyl]-L-O-methyltyrosine-N-methylamide

See PCT Publication No. WO 93/09090 on the above compound Nos. 31-34.

Compound 35. [4-(N-hydroxyamino)-2(R)-(3-phenylpropyl)succinyl]-L-3-cyclohexylalanine-N-[(2-morpholinosulfonylamino)ethyl]amide
Compound 36. [4-(N-hydroxyamino)-2(R)-(3-(4-chlorophenyl)propyl) succinyl]-L-3-cyclohexylalanine-N-[(2-morpholinosulfonylamino)ethyl]amide
Compound 37. [4-(N-hydroxyamino)-2(R)-(3-(4-methylphenyl)propyl) succinyl]-L-3-cyclohexylalanine-N-[(2-morpholinosulfonylamino)ethyl]amide
Compound 38. [4-(N-hydroxyamino)-2(R)-(3-(2-phenoxyethyl) succinyl]-L-3-cyclohexylalanine-N-(2-phenylethyl)amide
Compound 39. [4-(N-hydroxyamino)-2(R)-(3-(4-pyridyl)propyl)succinyl]-L-3-cyclohexylalanine-N-(2-phenylethyl)amide
Compound 40. [4-(N-hydroxyamino)-2(R)-(3-phenylpropylsuccinyl]-L-leucine-N-(2-phenylethyl)amide
Compound 41. [4-(N-hydroxyamino)-2(R)-(3-phenylpropyl)succinyl]-L-tert-leucine-amide
Compound 42. [4-(N-hydroxyamino)-2(R)-(3-(4-chlorophenyl)propyl) succinyl]-L-tert-leucine-amide
Compound 43. [4-(N-hydroxyamino)-2(R)-(3-(4-methylphenyl)propyl) succinyl]-L-tert-leucine-amide
Compound 44. [4-(N-hydroxyamino)-2(R)-(3-(4-methoxyphenyl)propyl)succinyl]-L-tert-leucine-amide
Compound 45. [4-(N-hydroxyamino)-2(R)-(3-(4-trifluoromethylphenyl)-propyl)succinyl]-L-tert-leucine-amide
Compound 46. [4-(N-hydroxyamino)-2(R)-(3-(4-fluorophenyl)propyl) succinyl]-L-tert-leucine-amide
Compound 47. [4-(N-hydroxyamino)-2(R)-(3-(4-chlorophenyl)propyl) succinyl]-L-3-methylthiovaline-N-methylamide
Compound 48. [4-(N-hydroxyamino)-2(R)-(3-phenylpropyl)succinyl]-L-3-cyclohexylalanine-N-[2-(4-sulfonamidophenyl)ethyl]amide

See PCT Publication Nos. WO 93/24449, WO 95/04033 and European Patent No. 489579 on the above compound Nos. 35-48.

Compound 49. [4-(N-hydroxyamino)-2(R)-isobutylsuccinyl]-L-tert-leucine-N-methylamide
Compound 50. [4-(N-hydroxyamino)-2(R)-isobutyl-3-phthalimidomethylsuccinyl]-L-tert-leucine-N-methylamide
Compound 51. [4-(N-hydroxyamino)-2(R)-isobutyl-3(S)-methylsuccinyl]-L-tert-leucine-N-methylamide
Compound 52. [4-(N-hydroxyamino)-2(R)-isobutyl-3-succinylimidomethylsuccinyl]-L-tert-leucine-N-methylamide
Compound 53. [4-(N-hydroxyamino)-2(R)-isobutylsuccinyl]-L-leucylglycine ethyl ester
Compound 54. [4-(N-hydroxyamino)-2(R)-isobutyl-3(S)-phthalimidobutyl)succinyl-L-leucylglycine ethyl ester
Compound 55. [4-(N-hydroxyamino)-2(R)-nonylsuccinyl]-L-leucyl-L-leucine-N-ethylamide
Compound 56. [4-(N-hydroxyamino)-2(R)-heptyl-3-phthalimidomethylsuccinyl]-L-leucyl-L-leucine-N-ethylamide

See European Patent Nos. 236872, 497192 and 575844 on the above compound Nos. 49-56.

Compound 57. [4-(N-hydroxyamino)-2(R)-isobutyl-3(S)-(2-thienylthiomethyl)succinyl]-L-phenylalanine-N-methylamide
Compound 58. [4-(N-hydroxyamino)-2(R)-isobutyl-3(S)-phenylthiomethylsuccinyl]-L-phenylalanine-N-methylamide
Compound 59. [4-(N-hydroxyamino)-2(R)-isobutyl-3(S)-(4-hydroxyphenylthiomethyl)succinyl]-L-phenylalanine-N-methylamide
Compound 60. [4-(N-hydroxyamino)-2(R)-isobutyl-3-mercaptomethylsuccinyl]-L-phenylalanine-N-methylamide
Compound 61. [4-(N-hydroxyamino)-2(R)-isobutyl-3-(S)-phenylsulfinylmethylsuccinyl]-L-phenylalanine-N-methylamide
Compound 62. [4-(N-hydroxyamino)-2(R)-isobutyl-3(S)-phenylsulfonylmethylsuccinyl-L-phenylalanine-N-methylamide
Compound 63. [4-(N-hydroxyamino)-2(R)-isobutyl-3(S)-(2-thienylsulfonylmethyl)succinyl]-L-phenylalanine-N-methylamide
Compound 64. [4-(N-hydroxyamino)-2(R)-isobutyl-3(S)-(2-thienylsulfinylmethyl)succinyl]-L-phenylalanine-N-methylamide

Compound 65. [4-(N-hydroxyamino)-2(R)-isobutyl-3(S)-(2-propenyl)succinyl]-L-phenylalanine-N-methylamide
Compound 66. [4-(N-hydroxyamino)-2(R)-isobutyl-3(S)-(2-propenyl) succinyl]-L-leucine-N-methylamide
Compound 67. [4-(N-hydroxyamino)-2(R)-isobutyl-3(S)-(2-propenyl) succinyl]-L-3-(2-thienyl)alanine-N-methylamide
Compound 68. [4-(N-hydroxyamino)-2(R)-isobutyl-3(S)-(2-propenyl) succinyl]-L-2-(2-methoxycarbonylethyl)glycine-N-methylamide
Compound 69. [4-(N-hydroxyamino)-3-(S)-hydroxy-2(R)-isobutylsuccinyl]-L-phenylalanine-N-methylamide
Compound 70. [4-(N-hydroxyamino)-3(S)-hydroxy-2(R)-isobutylsuccinyl-L-3-cyclohexylalanine-N-methylamide
Compound 71. [4-(N-hydroxyamino)-3(S)-hydroxy-2(R)-isobutyl-succinyl]-L-phenylglycine-N-methylamide
Compound 72. [4-(N-hydroxyamino)-3(S)-hydroxy-2(R)-isobutyl-succinyl]-L-tert-leucine-N-methylamide
Compound 73. [4-(N-hydroxyamino)-3(S)-hydroxy-2(R)-isobutylsuccinyl]-L-3-cyclohexylalanine-N-(3-(2-pyrrolidone)propyl)amide
Compound 74. [4-(N-hydroxyamino)-3-(S)-hydroxy-2(R)-isobutylsuccinyl]-L-4-(N-hydroxyamino)glutamic acid N-methylamide
Compound 75. [4-(N-hydroxyamino)-3(S)-hydroxy-2(R)-isobutylsuccinyl-L-phenylalanylglycine-N-(2-acetamide-2-deoxy-β-D-glucopyranosil)amide
Compound 76. [4-(N-hydroxyamino)-2(R)-isobutylsuccinyl]-L-phenylalanylglycine-N-(2-acetamide-2-deoxy-β-D-glucopyranosil)-amide
Compound 77. [4-(N-hydroxyamino)-2(R)-isobutylsuccinyl]-O-[(2-acetamide-2-deoxy-β-D-glucopyranosil)carboxamidomethyl]-L-tyrosine-N-methylamide
Compound 78. [4-(N-hydroxyamino)-2(R)-isobutyl-3(S)-(2-propenyl) succinyl]-$N^4$-[(2-acetamide-2-deoxy-β-D-glucopyranosil)L-glutamine-N-methylamide

See PCT Publication Nos. WO 94/21625, WO 94/24140 and British Patent Publication No. 2268934 on the above compound Nos. 57-78.

Compound 79. 4(S)-[4-(N-hydroxyamino)-2(R)-isobutylsuccinyl]amino-1,2,4,5-tetrahydro-3H-2-benzoazepin-3-one

See Japanese Patent Publication No. 145148/1994 on compound 79.

Compound 84. 3(S)-[4-(N-hydroxyamino)-2(R)-isobutylsuccinyl]amino-3,4-dihydrocarbostyril
Compound 85. 3(S)-[4-(N-hydroxyamino)-2(R)-isobutyl-3-(S)-(2-thienylthiomethyl)succinyl]amino-3,4-dihydrocarbostyril
Compound 86. 3(S)-[4-(N-hydroxyamino)-2(R)-isobutyl-3(R or S)-phthalimidomethylsuccinyl]amino-3,4-dihydrocarbostyril
Compound 87. 3(S)-[4-(N-hydroxyamino)-2(R)-isobutylsuccinyl]amino-1-methoxy-3,4-dihydrocarbostyril

See Japanese Patent Publication (A) No. 157470/1995 on the above compound Nos. 84-87.

Recently, it is clarified that TNF-α (17 kD) which belongs to TNF family proteins as Fas L does, is processed from its precursor (26 kD) on the surface of cells by a methalloprotease. [K.M. Mohler et al., Nature, 370, 218-220 (1994)]

As mentioned above, as a compound having MMP inhibitory activity prohibits the activity of this methalloproteinase, the use of the MMP inhibitory compound for treatment of diseases mediated by TNF-α, for example, inflammatory, acute infectious disease, shock state, GVHD, AIDS, etc. was filed as a patent (PCT Patent Publication No. WO 94/10990).

It was suspected that Fas L on the surface of cells was processed by the enzyme to produce sFas L (M. Sara et al., Eur. J. Immunol., 25, 2303-2307 (1995)), but it has not been reported that a compound having the MMP inhibitory activity shows the inhibition of sFas L production, and the method for preventing the production of sFas L is not known.

DISCLOSURE OF INVENTION

The present invention relates to a drug for treating and/or preventing hepatitis, GVHD, AIDS, autoimmune diseases, etc. caused by inhibition of the production of sFas L. The present invention relates further to a reagent inhibiting the production of sFas L, which is useful for the test and study of Fas L.

The present inventors studied the inhibition of sFas L-production on the known compounds which have MMP inhibitory activity and found that these compounds prevents the processing of Fas L by the enzyme to inhibit the production of sFas L. Further, the present inventors synthesized new compounds having MMP inhibitory activity and found that these compounds have inhibitory activity of sFas L production as well. Furthermore, the present inventors recognized that the above compounds having MMP inhibitory activity show excellent therapeutic and prophylactic activity of GVHD

by using experimental animals and completed the present invention.

BEST MODE FOR PRACTICING INVENTION

The active ingredient of Fas L solubilization inhibitor preparation is a compound having MMP inhibitory activity, and includes, for example, a compound of the following generic formula (I), its pharmaceutically acceptable salt, its hydrate, its solvate and its prodrug. .

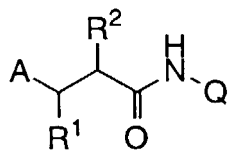

(I)

wherein Q is a group selected from the following formula (II)-(V)

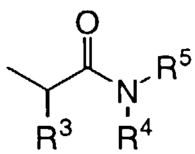

(II)

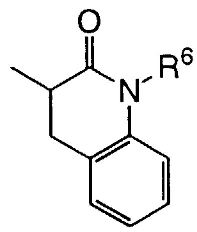

(III)

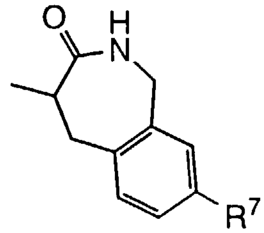

(IV)

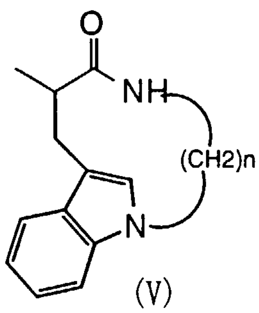

(V)

wherein A is N-hydroxyaminocarbonyl, carboxyl, mercapto, or phosphono; $R^1$ is hydrogen, amino, hydroxy, mercapto, $C_1$-$C_6$ alkoxy, $C_1$-$C_6$ alkylthio, $C_1$-$C_6$ alkyl, $C_2$-$C_6$ alkenyl, $C_6$-$C_{12}$ aryl, $C_6$-$C_{12}$ aryl($C_1$-$C_6$ alkyl), ($C_1$-$C_6$ alkyl)thio($C_1$-$C_6$ alkyl), ($C_1$-$C_6$ alkanoyl)thio($C_1$-$C_6$ alkyl), $C_6$-$C_{12}$ arylthio(1-C6 alkyl), heterocyclylthio($C_1$-$C_6$ alkyl), ($C_1$-$C_6$ alkyl)sulfinyl($C_1$-$C_6$ alkyl), $C_6$-$C_{12}$ arylsulfinyl($C_1$-$C_6$ alkyl), heterocyclylsulfinyl ($C_1$-$C_6$ alkyl), ($C_1$-$C_6$ alkyl)sulfonyl($C_1$-$C_6$ alkyl), ($C_6$-$C_{12}$ aryl)sulfonyl($C_1$-$C_6$ alkyl), heterocyclylsulfonyl($C_1$-$C_6$ alkyl), $C_1$-$C_6$ alkyl substituted by -$NR^{11}R^{12}$, wherein $R^{11}$ and $R^{12}$ are the same or different and are hydrogen, or $C_1$-$C_6$ alkyl, or may constitute a saturated or unsaturated heterocyclic ring with the adjacent nitrogen atom, or $C_1$-$C_6$ alkyl which may be substituted by a radical selected from a group consisting of amino, hydroxy, mercapto and carboxyl of which the radical may be chemically protected; $R^2$ is hydrogen, $C_1$-$C_6$ alkyl, $C_2$-$C_6$ alkenyl, $C_6$-$C_{12}$ aryl, $C_6$-$C_{12}$ aryl($C_1$-$C_6$ alkyl), heterocyclyl($C_1$-$C_6$ alkyl, cycloalkyl($C_1$-$C_6$ alkyl), $C_1$-$C_6$ alkylthio, $C_1$-$C_6$ alkylsulfinyl, $C_1$-$C_6$ alkylsulfonyl, $C_6$-$C_{12}$ aryl($C_1$-$C_6$ alkyl)thio, ($C_1$-$C_6$ alkyl)thio($C_1$-$C_6$ alkyl), or ($C_1$-$C_6$ alkoxy) $C_1$-$C_6$ alkyl; $R^3$ is $C_1$-$C_6$ alkyl, $C_2$-$C_6$ alkenyl, $C_6$-$C_{12}$ aryl($C_1$-$C_6$ alkyl), $C_6$-$C_{12}$ aryl, heterocyclyl, heterocyclyl($C_1$-$C_6$ alkyl), bicycloaryl, bicycloaryl($C_1$-$C_6$ alkyl), cycloalkyl($C_1$-$C_6$ alkyl), cycloalkyl, glycosyloxy($C_1$-$C_6$ alkyl), or tetrahydronaphthylmethyl; $R^4$ is hydrogen, $C_1$-$C_6$ alkyl, $C_1$-$C_6$ alkoxy($C_1$-$C_6$ alkyl), [di($C_1$-$C_6$ alkoxy)$C_1$-$C_6$ alkyl]methyl, [tri($C_1$-$C_6$ alkoxy)$C_1$-$C_6$ alkyl]methyl, hydroxy($C_1$-$C_6$ alkyl), [dihydroxy($C_1$-$C_6$ alkyl)]methyl, [trihydroxy($C_1$-$C_6$ alkyl)]methyl, amino($C_1$-$C_6$ alkyl), substituted amino($C_1$-$C_6$ alkyl), carboxy($C_1$-$C_6$ alkyl), $C_1$-$C_6$ alkox-

ycarbonyl($C_1$-$C_6$ alkyl), carbamoyl($C_1$-$C_6$ alkyl, substituted carbamoyl($C_1$-$C_6$ alkyl), dihydroxyphosphonyl($C_1$-$C_6$ alkyl), di($C_1$-$C_6$ alkoxy)phosphonyl($C_1$-$C_6$)alkyl, $C_6$-$C_{12}$ aryl, $C_6$-$C_{12}$ aryl($C_1$-$C_6$ alkyl), pyrrolidino($C_1$-$C_6$ alkyl), piperidino($C_1$-$C_6$ alkyl), morpholino($C_1$-$C_6$ alkyl), glycosyloxy($C_1$-$C_6$ alkyl), - CH($R^{13}$)COOR$^{14}$, wherein $R^{13}$ is a side chain of $\alpha$-amino acid, and $R^{14}$ is $C_1$-$C_6$ alkyl, -CH($R^{13}$)CONR$^{15}$R$^{16}$, wherein $R^{13}$ is as defined above, $R^{15}$ and $R^{16}$ are the same or different and are hydrogen, or $C_1$-$C_6$ alkyl, $C_1$-$C_6$ alkoxy which may be substituted by hydroxy, amino or carboxyl, morpholinosulfonylamino($C_1$-$C_6$ alkyl), or ($C_1$-$C_6$ alkyl)aminosulfonylamino($C_1$-$C_6$ alkyl), di($C_1$-$C_6$ alkyl) aminosulfonylamino($C_1$-$C_6$ alkyl), aminosulfonyl $C_6$-$C_{12}$ aryl ($C_1$-$C_6$ alkyl; $R^5$ is hydrogen, or $C_1$-$C_6$ alkyl, and $R^4$ and $R^5$ may constitute a saturated or unsaturated cyclic amine with the adjacent nitrogen atom, of which the cyclic amine may contain an oxygen or sulfur atom; $R^6$ is hydrogen, hydroxy, $C_1$-$C_6$ alkoxy, or ($C_1$-$C_6$ alkoxy)$C_1$-$C_6$ alkoxy; and $R^7$ is hydrogen, hydroxy, or methoxy; and n is integer 5-7

Substituents of the compound (I) are illustrated as follows.

Examples of $C_1$-$C_6$ alkyl are methyl, ethyl, propyl, isopropyl, n-butyl, isobutyl, sec-butyl, tert-butyl, n-pentyl, isopentyl, n-hexyl, isohexyl, etc..

Examples of a saturated or unsaturated cyclic amine which may be contain a sulfur or oxygen atom are piperidine, morpholine, etc. $C_1$-$C_6$ alkyl substituted by -NR$^{11}$R$^{12}$, wherein $R^{11}$ and $R^{12}$ are the same or different and are hydrogen or $C_1$-$C_6$ alkyl, or may constitute a saturated or unsaturated heterocyclic ring with the adjacent nitrogen atom means $C_1$-$C_6$ alkyl substituted by mono($C_1$-$C_6$ alkyl)amino or di($C_1$-$C_6$ alkyl)amino in which the alkyls may be different, or by a saturated or unsaturated 5 or 6 membered ring which may be fused with a benzene ring, at its N atom. The examples are methylaminomethyl, dimethylaminomethyl, pyrrolidinomethyl which may be substituted, piperadinomethyl, piperidinomethyl, 4-morpholinomethyl, 1,2,3,4-tetrahydroisoquinolin-2-yl methyl, phthalimidomethyl, etc.

Heterocyclyl means a saturated or unsaturated 5 or 6 membered heterocyclic ring which contains at least an atom among nitrogen, oxygen and sulfur atoms. As it are illustrated furyl, thienyl, morpholino, pyridinyl, imidazolidinyl, pyrimidinyl, and pyridazinyl.

Cycloalkyl means cyclopentyl, cyclohexyl, and cycloheptyl.

Bicycloaryl means a bicycle which may contain one or more hetero atoms selected from sulfur, nitrogen and oxygen atoms and is aromatic, and is fused or combined with others. As the fused bicycle are illustrated naphthyl, indolyl, benzothienyl, quinolinyl, isoquinolinyl, etc. As the combined bicycle are illustrated biphenyl, 4-phenylpyrimidyl, 3-phenylpyridyl, etc.

As $C_6$-$C_{12}$ aryl are illustrated phenyl, 1-naphthyl, 2-naphthyl, etc.

The above mentioned heterocycle, cycloalkyl, bicycloaryl and $C_6$-$C_{12}$ aryl may be substituted and the substituents means halogen such as chloro, bromo, fluoro, etc., hydroxy, amino, dimethylamino, methoxy, trifluoromethyl, and $C_1$-$C_4$ alkyl, as long as defined otherwise.

Each a stereoisomer is contained in a compound of the generic formula (I)-, and these isomers and mixture thereof should be included in this invention, as long as described otherwise.

The optically active compounds of the present invention (I) are illustrated below.

Compound 1. [4-(N-hydroxyamino)-2(R)-isobutylsuccinyl]-L-phenylglycine-N-methylamide
Compound 2. [4-(N-hydroxyamino)-2(R)-isobutylsuccinyl]-L-p-hydroxyphenylglycine-N-methylamide
Compound 3. [4-(N-hydroxyamino)-2(R)-isobutyl-3-morpholinomethylsuccinyl]-L-phenylglycine-N-methylamide
Compound 4a. [4-(N-hydroxyamino)-2(R)-isobutyl-3(S)-methylsuccinyl]-L-phenylglycine-N-methylamide
Compound 5. [4-(N-hydroxyamino)-2(R)-isobutyl-3-methylaminomethylsuccinyl]-L-phenylglycine-N-methylamide
Compound 6. [4-(N-hydroxyamino)-2(R)-isobutylsuccinyl]-L-p-isopropylphenylglycine-N-methylamide
Compound 7. [4-(N-hydroxyamino)-2(R)-isobutylsuccinyl]-L-phenylglycine-N-ethylamide
Compound 8. [4-(N-hydroxyamino)-2(R)-isobutylsuccinyl]-L-p-fluorophenylglycine-N-methylamide
Compound 9. [4-(N-hydroxyamino)-2(R)-isobutylsuccinyl]-L-cyclohexylglycine-N-methylamide
Compound 10. [4-(N-hydroxyamino)-2(R)-isobutyl-3-methylsuccinyl]-L-cyclohexylglycine-N-methylamide
Compound 11. [4-(N-hydroxyamino)-2(R)-isobutylsuccinyl]-L-2-naphthylglycine-N-methylamide
Compound 12. [4-(N-hydroxyamino)-2(R)-isobutyl-3-morpholinomethylsuccinyl]-L-cyclohexylglycine-N-methylamide
Compound 13. [4-(N-hydroxyamino)-2(R)-isobutylsuccinyl]-L-3-(1-naphthyl)alanine-N-methylamide
Compound 14. [4-(N-hydroxyamino)-2(R)-isobutylsuccinyl]-L-3-(2-naphthyl)alanine-N-methylamide
Compound 15. [4-(N-hydroxyamino)-2(R)-isobutyl-3-(1,2,3,4-tetrahydroisoquinolin-2-yl) methylsuccinyl]-L-3-(1-naphthyl)alanine-N-methylamide
Compound 16. [4-(N-hydroxyamino)-2(R)-isobutyl-3-morpholinomethylsuccinyl]-L-3-(1-naphthyl)alanine-N-methylamide
Compound 17. [4-(N-hydroxyamino)-2(R)-isobutyl-3-methylsuccinyl]-L-3-(1-naphthyl)alanine-N-methylamide
Compound 18. [4-(N-hydroxyamino)-2(R)-isobutyl-3-methylsuccinyl]-L-3-(5,6,7,8-tetrahydro-1-naphthyl)alanine-N-methylamide

Compound 19. [4-(N-hydroxyamino)-2(R)-isobutylsuccinyl]-L-homophenylalanine-N-methylamide
Compound 20. [4-(N-hydroxyamino)-2(R)-isobutylsuccinyl]-L-p-fluorophenylalanine-N-methylamide
Compound 21. [4-(N-hydroxyamino)-2(R)-isobutylsuccinyl]-L-3-(3-benzothienyl)alanine-N-methylamide
Compound 22. [4-(N-hydroxyamino)-2(R)-isobutyl-3-phenylthiomethylsuccinyl]-L-phenylglycine-N-methylamide
Compound 23. [4-(N-hydroxyamino)-2(R)-isobutylsuccinyl]-L-O-methyltyrosine-N-methylamide
Compound 24. [4-(N-hydroxyamino)-2(R)-isobutyl-3(S)-methylsuccinyl]-L-O-methyltyrosine-N-methylamide
Compound 25. [4-(N-hydroxyamino)-2(R)-cyclohexylmethylsuccinyl]-L-O-methyltyrosine-N-methylamide
Compound 26. [4-(N-hydroxyamino)-2(R)-isobutylsuccinyl]-L-phenylalanine-N-methylamide
Compound 27. [4-(N-hydroxyamino)-2(R)-isopropylsuccinyl]-L-tyrosine-N-methylamide
Compound 28. [4-(N-hydroxyamino)-2(R)-isobutylsuccinyl]-L-tryptophan-N-methylamide
Compound 29. [4-(N-hydroxyamino)-2(R)-isobutylsuccinyl]-L-tryptophan-N-(1(S)-phenyl)ethylamide
Compound 30. [4-(N-hydroxyamino)-2(R)-isobutylsuccinyl]-L-tryptophan-N-(2-hydroxy)ethylamide
Compound 31. [4-(N-hydroxyamino)-3-methylthio-2-propoxymethylsuccinyl]-L-O-methyltyrosine-N-methylamide
Compound 32. [4-(N-hydroxyamino)-3-methyl-2-propoxymethylsuccinyl]-L-O-methyltyrosine-N-methylamide
Compound 33. [4-(N-hydroxyamino)-3-acetylthiomethyl-2-propoxymethylsuccinyl]-L-O-methyltyrosine-N-methylamide
Compound 34. [4-(N-hydroxyamino)-3-methyl-2-propylthiosuccinyl]-L-O-methyltyrosine-N-methylamide
Compound 35. [4-(N-hydroxyamino)-2(R)-(3-phenylpropyl)succinyl]-L-3-cyclohexylalanine-N-[(2-morpholinosulfonylamino)ethyl]amide
Compound 36. [4-(N-hydroxyamino)-2(R)-(3-(4-chlorophenyl)propyl) succinyl]-L-3-cyclohexylalanine-N-[(2-morpholinosulfonylamino)ethyl]amide
Compound 37. [4-(N-hydroxyamino)-2(R)-(3-(4-methylphenyl)propyl) succinyl]-L-3-cyclohexylalanine-N-[(2-morpholinosulfonyl)ethyl]amide
Compound 38. [4-(N-hydroxyamino)-2(R)-(3-(2-phenoxyethyl) succinyl]-L-3-cyclohexylalanine-N-(2-phenylethyl)amide
Compound 39. [4-(N-hydroxyamino)-2(R)-(3-(4-pyridyl)propyl)succinyl]-L-3-cyclohexylalanine-N-(2-phenylethyl)amide
Compound 40. [4-(N-hydroxyamino)-2(R)-(3-phenylpropyl)succinyl]-L-leucine-N-(2-phenylethyl)amide
Compound 41. [4-(N-hydroxyamino)-2(R)-(3-phenylpropyl)succinyl]-L-tert-leucine-amide
Compound 42. [4-(N-hydroxyamino)-2(R)-(3-(4-chlorophenyl)propyl) succinyl]-L-tert-leucine-amide
Compound 43. [4-(N-hydroxyamino)-2(R)-(3-(4-methylphenyl)propyl) succinyl]-L-tert-leucine-amide
Compound 44. [4-(N-hydroxyamino)-2(R)-(3-(4-methoxyphenyl)propyl)succinyl]-L-tert-leucine-amide
Compound 45. [4-(N-hydroxyamino)-2(R)-(3-(4-trifluoromethylphenyl)propyl)succinyl]-L-tert-leucine-amide
Compound 46. [4-(N-hydroxyamino)-2(R)-(3-(4-fluorophenyl)propyl) succinyl]-L-tert-leucine-amide
Compound 47. [4-(N-hydroxyamino)-2(R)-(3-(4-chlorophenyl)propyl) succinyl]-L-3-methylthiovaline-N-methylamide
Compound 48. [4-(N-hydroxyamino)-2(R)-(3-phenylpropyl)succinyl]-L-3-cyclohexylalanine-N-[2-(4-sulfonylamidophenyl)ethyl]amide
Compound 49. [4-(N-hydroxyamino)-2(R)-isobutylsuccinyl]-L-tert-leucine-N-methylamide
Compound 50. [4-(N-hydroxyamino)-2(R)-isobutyl-3-phthalimidomethylsuccinyl]-L-tert-leucine-N-methylamide
Compound 51. [4-(N-hydroxyamino)-2(R)-isobutyl-3(S)-methylsuccinyl]-L-tert-leucine-N-methylamide
Compound 52. [4-(N-hydroxyamino)-2(R)-isobutyl-3-succinylimidomethylsuccinyl]-L-tert-leucine-N-methylamide
Compound 53. [4-(N-hydroxyamino)-2(R)-isobutylsuccinyl]-L-leucylglycine ethyl ester
Compound 54. [4-(N-hydroxyamino)-2(R)-isobutyl-3(S)-(4-phthalimidobutyl)succinyl-L-leucylglycine ethyl ester
Compound 55. [4-(N-hydroxyamino)-2(R)-nonylsuccinyl]-L-leucyl-L-leucine-N-ethylamide
Compound 56. [4-(N-hydroxyamino)-2(R)-heptyl-3-phthalimidomethylsuccinyl]-L-leucyl-L-leucine-N-ethylamide
Compound 57. [4-(N-hydroxyamino)-2(R)-isobutyl-3(S)-(2-thienylthiomethyl)succinyl]-L-phenylalanine-N-methylamide
Compound 58. [4-(N-hydroxyamino)-2(R)-isobutyl-3(S)-phenylthiomethyl)succinyl]-L-phenylalanine-N-methylamide
Compound 59. [4-(N-hydroxyamino)-2(R)-isobutyl-3(S)-(4-hydroxyphenylthiomethyl)succinyl]-L-phenylalanine-N-methylamide
Compound 60. [4-(N-hydroxyamino)-2(R)-isobutyl-3(S)-mercaptomethylsuccinyl]-L-phenylalanine-N-methylamide
Compound 61. [4-(N-hydroxyamino)-2(R)-isobutyl-3-(S)-phenylsulfinylmethylsuccinyl]-L-phenylalanine-N-methylamide
Compound 62. [4-(N-hydroxyamino)-2(R)-isobutyl-3(S)-phenylsulfonylmethylsuccinyl-L-phenylalanine-N-methylamide
Compound 63. [4-(N-hydroxyamino)-2(R)-isobutyl-3(S)-(2-thienylsulfonylmethyl)succinyl]-L-phenylalanine-N-methylamide

Compound 64. [4-(N-hydroxyamino)-2(R)-isobutyl-3(S)-(2-thienylsulfinylmethyl)succinyl]-L-phenylalanine-N-methylamide

Compound 65. [4-(N-hydroxyamino)-2(R)-isobutyl-3(S)-(2-propenyl)succinyl]-L-phenylalanine-N-methylamide

Compound 66. [4-(N-hydroxyamino)-2(R)-isobutyl-3(S)-(2-propenyl) succinyl]-L-leucine-N-methylamide

Compound 67. [4-(N-hydroxyamino)-2(R)-isobutyl-3(S)-(2-propenyl) succinyl]-L-3-(2-thienyl)alanine-N-methylamide

Compound 68. [4-(N-hydroxyamino)-2(R)-isobutyl-3(S)-(2-propenyl) succinyl]-L-2-(2-methoxycarbonylethyl)glycine-N-methylamide

Compound 69. [4-(N-hydroxyamino)-3-(S)-hydroxy-2(R)-isobutylsuccinyl]-L-phenylalanine-N-methylamide

Compound 70. [4-(N-hydroxyamino)-3(S)-hydroxy-2(R)-isobutylsuccinyl-L-3-cyclohexylalanine-N-methylamide

Compound 71. [4-(N-hydroxyamino)-3(S)-hydroxy-2(R)-isobutyl-succinyl]-L-phenylglycine-N-methylamide

Compound 72. [4-(N-hydroxyamino)-3(S)-hydroxy-2(R)-isobutyl-succinyl]-L-tert-leucine-N-methylamide

Compound 73. [4-(N-hydroxyamino)-3(S)-hydroxy-2(R)-isobutylsuccinyl]-L-3-cyclohexylalanine-N-(3-(2-pyrrolidone)propyl)amide

Compound 74. [4-(N-hydroxyamino)-3-(S)-hydroxy-2(R)-isobutylsuccinyl]-L-4-(N-hydroxyamino)glutamic acid N-methylamide

Compound 75. [4-(N-hydroxyamino)-3(S)-hydroxy-2(R)-isobutylsuccinyl]-L-phenylalanylglycine-N-(2-acetamide-2-deoxy-β-D-glucopyranosil)-amide

Compound 76. [4-(N-hydroxyamino)-2(R)-isobutylsuccinyl]-L-phenylalanylglycine-N-(2-acetamide-2-deoxy-β-D-glucopyranosil)-amide

Compound 77. [4-(N-hydroxyamino)-2(R)-isobutylsuccinyl]-O-[(2-acetamide-2-deoxy-β-D-glucopyranosil)carboxamidemethyl]-L-tyrosine-N-methylamide

Compound 78. [4-(N-hydroxyamino)-2(R)-isobutyl-3(S)-(2-propenyl) succinyl]-$N^4$-[(2-acetamide-2-deoxy-β-D-glucopyranosil)-L-glutamine-N-methylamide

Compound 79. 4(S)-[4-(N-hydroxyamino)-2(R)-isobutylsuccinyl]amino1,2,4,5-tetrahydro-3H-2-benzoazepin-3-one

Compound 80. 4(S)-[4-(N-hydroxyamino)-2(R)-isobutyl-3-morpholinomethylsuccinyl]amino-1,2,3,4,5-tetrahydro-3H-2-benzoazepin-3-one

Compound 81. 4(S)-[4-(N-hydroxyamino)-2(R)-isobutyl-3-(1,2,3,4-tetrahydroisoquinolin-2-yl)methylsuccinyl]amino-1,2,4,5-tetrahydro-3H-2-benzoazepin-3-one

Compound 82. 4(S)-[4-(N-hydroxyamino)-2(R)-isobutyl-3-methylsuccinyl]amino-1,2,4,5-tetrahydro-3H-2-benzoazepin-3-one

Compound 83. 4(S)-[4-(N-hydroxyamino)-2(R)-isobutyl-3-phenylthiomethylsuccinyl]amino-1,2,4,5-tetrahydro-3H-2-benzoazepin-3-one

Compound 84. 3(S)-[4-(N-hydroxyamino)-2(R)-isobutylsuccinyl]amino-3,4-dihydrocarbostyril

Compound 85. 3(S)-[4-(N-hydroxyamino)-2(R)-isobutyl-3(S)-(2-thienylthiomethyl)succinyl]amino-3,4-dihydrocarbostyril

Compound 86. 3(S)-[4-(N-hydroxyamino)-2(R)-isobutyl-3(R or S)-phthalimidemethylsuccinyl]amino-3,4-dihydrocarbostyril

Compound 87. 3(S)-[4-(N-hydroxyamino)-2(R)-isobutylsuccinyl]amino-1-methoxy-3,4-dihydrocarbostyril

Compound 88. [4-(N-hydroxyamino)-2(R)-phenylethylsuccinyl]-L-phenylglycine-N-methylamide

Compound 89. [4-(N-hydroxyamino)-2(R)-phenylethylsuccinyl]-L-3-(1-naphthyl)alanine-N-methylamide

Compound 90. [4-(N-hydroxyamino)-2(R)-phenylpropylsuccinyl]-L-3-(1-naphthyl)alanine-N-methylamide

Compound 91. [4-(N-hydroxyamino)-2(R)-phenylpropylsuccinyl]-L-phenylglycine-N-methylamide

A Fas L solubilization inhibitor of the present invention is not limited, as long as it shows MMP inhibitory activity, and a compound of the following formula

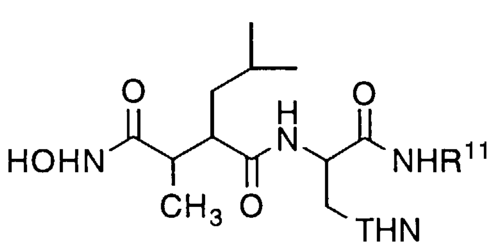
(VIa)

wherein $R^{11}$ is a $C_1$-$C_6$ alkyl, and THN is 5,6,7,8-tetrahydro-1-naphthyl or 5,6,7,8-tetrahydro-2-naphthyl, or its salt is preferably used.

The following compounds are illustrated:

[4-(N-hydroxyamino)-2(R)-isobutyl-3(R)-methylsuccinyl]-L-3-(5,6,7,8-tetrahydro-1-naphthyl)alanine-N-methylamide;
[4-(N-hydroxyamino)-2(R)-isobutyl-3(S)-methylsuccinyl]-L-3-(5,6,7,8-tetrahydro-1-naphthyl)alanine-N-methylamide;
[4-(N-hydroxyamino)-2(S)-isobutyl-3(R)-methylsuccinyl]-L-3-(5,6,7,8-tetrahydro-1-naphthyl)alanine-N-methylamide;
[4-(N-hydroxyamino)-2(S)-isobutyl-3(S)-methylsuccinyl]-L-3-(5,6,7,8-tetrahydro-1-naphthyl)alanine-N-methylamide.

Among them, [4-(N-hydroxyamino)-2(R)-isobutyl-3(S)-methylsuccinyl]-L-3-(5,6,7,8-tetrallydro-1-naphthyl)alanine-N-methylamide is especially preferable.

The processes for these compounds are explained as follows.

⟨Process 1⟩

A compound of formula (I), wherein Q is a group (II) and A is N-hydroxyaminocarbonyl; $R^1$ is hydrogen, methyl, C1-6 alkylaminomethyl, morpholinomethyl, piperazinomethyl, piperidinomethyl, or (1,2,3,4-tetrahydroisoquinolin-2-yl)methyl; $R^2$ is C1-6 alkyl; $R^3$ is phenyl which may be substituted; $R^4$ and $R^5$ are the same or different and are hydrogen, or $C_1$-$C_6$ alkyl,
for example a compound of compounds 1-8, is prepared by the method described in Japanese Patent Publication (A) No. 101925/1995 (Kanebo Ltd.) or the method according to that publication.

To obtain a stereoisomer, it is possible to separate and purify its diastereomer and prepare selectively a specific stereoisomer. An example on compound 4a is explained as follows.

A compound 4a is prepared by separating the diastereomer according to A method or B method, as shown in the following Scheme 1.

Scheme 1

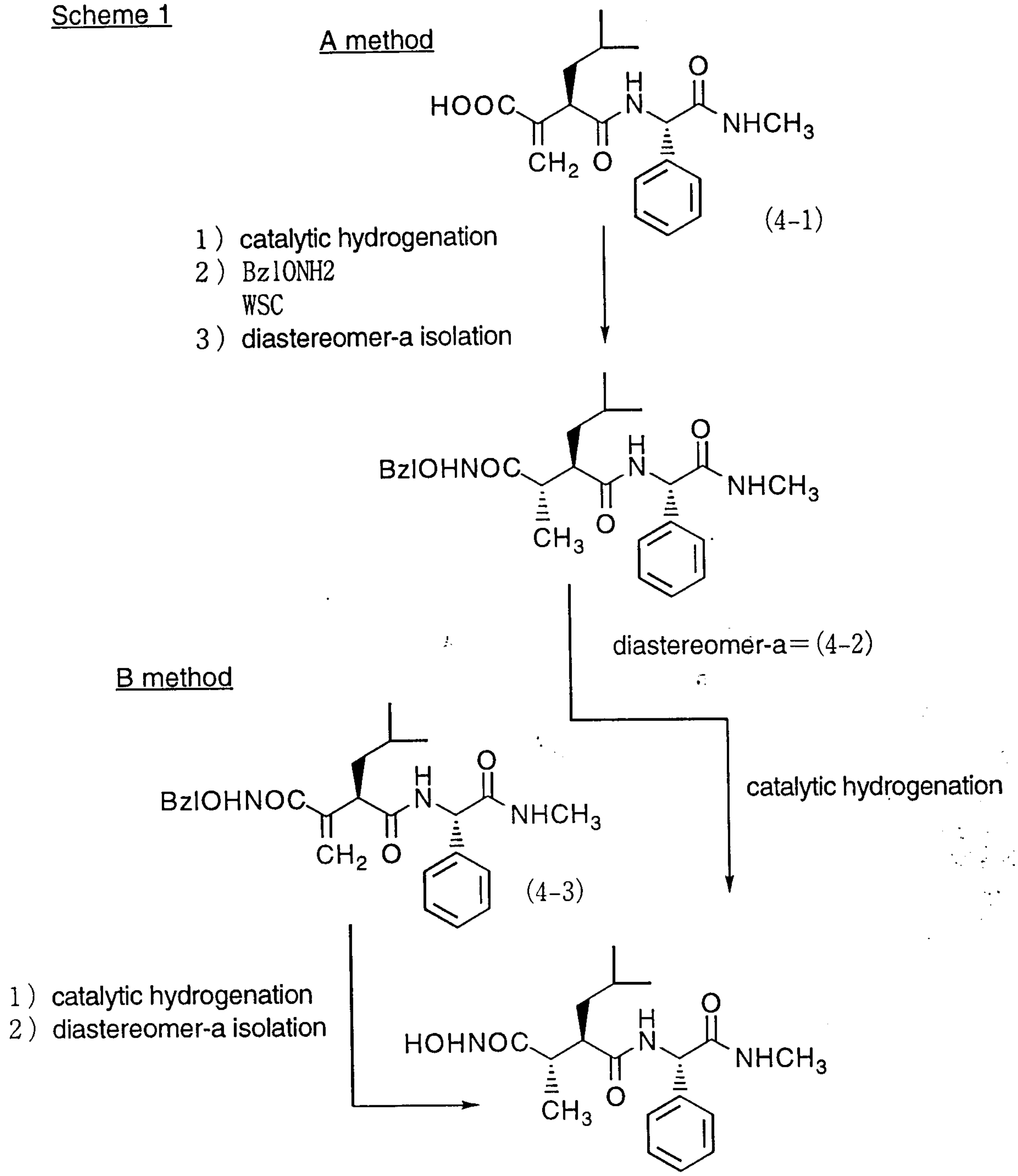


diastereomer-c=Compound 4a

wherein Bzl is benzyl, and WSC is 1-ethyl-3-(3-dimethylaminopropyl)carbodiimide hydrochloride.

A method: Compound (4-1) (see PCT Patent Publication No. WO 95/04715) is subjected to catalytic hydrogenation and is condensed with benzyloxyamine to give a mixture of diastereomer a and diastereomer b. A main product, diastereomer a (4-2) is obtainable, for example, by recrystallizing it in the N,N-dimethylformamide (DMF) solution, and then the diastereomer is subjected to catalytic hydrogenation to give compound 4a.

B method: Compound (4-3) is subjected to catalytic hydrogenation to give a mixture of diastereomer c and diastereomer d and its main product (diastereomer c) is compound 4a, and the compound is separated and purified, for example, by using high performance liquid chromatography (HPLC), etc.

On the other hand, compound 4a can be selectively prepared by using an optically active carboxylic acid derivative as shown in the following Scheme 2.

Scheme 2

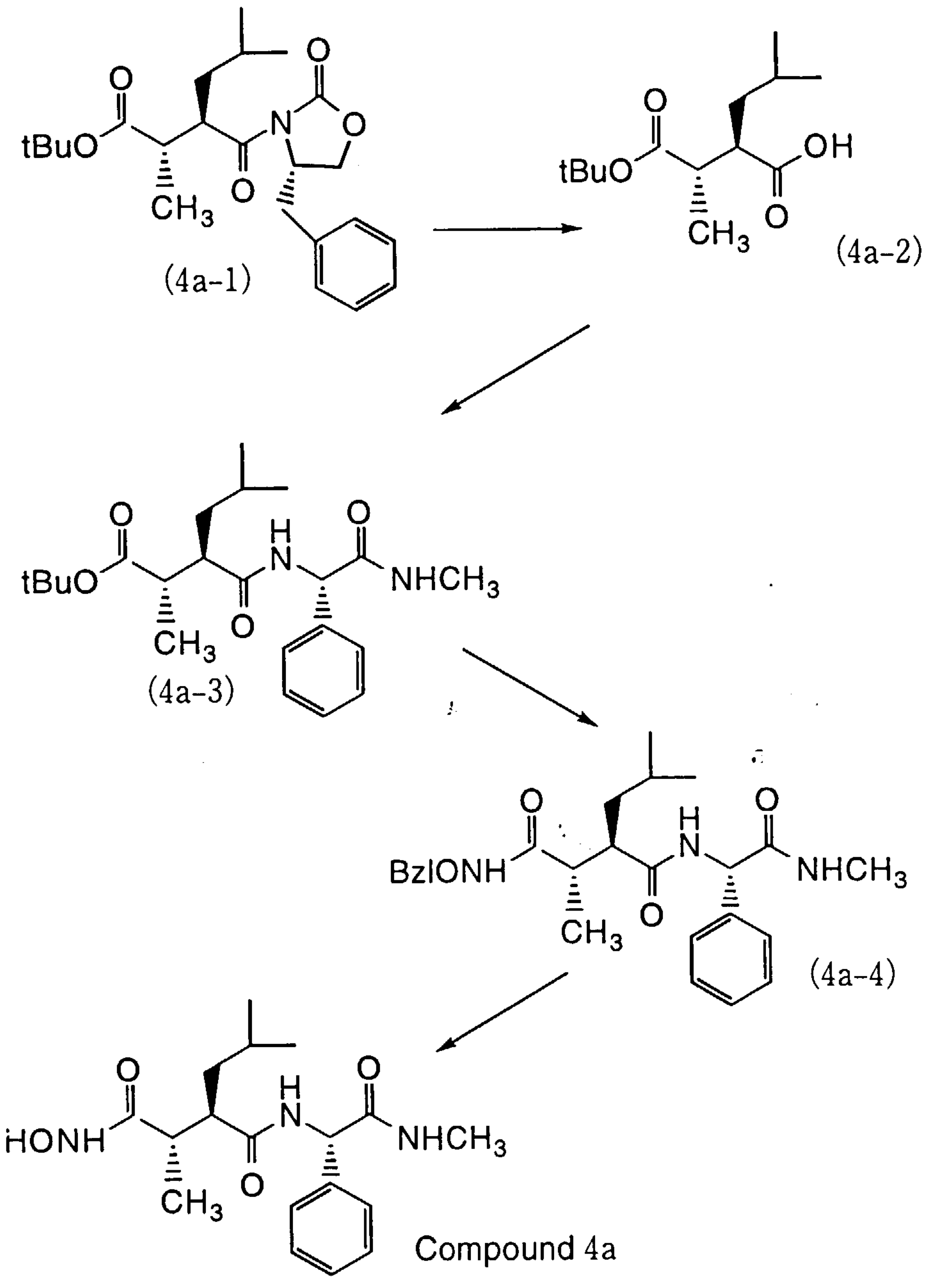

(4a-1) (4a-2) (4a-3) (4a-4) Compound 4a

An oxazolidinone derivative (4a-1) [Decicco et al., J. Org. Chem., 1995, 60, 4782-4785] is subjected to oxidative degradation by using lithium hydride and hydrogen peroxide to give an optically active carboxylic acid derivative (4a-2).

Then, a carboxylic acid (4a-2) and L-phenylglycine-N-methylamide are condensed by using a water-soluble carbodiimide [e.g. 1-ethyl-3-(3-dimethylaminopropyl)carbodiimide hydrochloride (abbreviated as WSC)] and N-hydroxybenztriazol (abbreviated as HOBt) to give a compound (4a-3).

And then, after acid-degradation of the tert-butyl group on a compound (4a-3) by formic acid, the product is condensed with O-benzylhydroxyamine by using WSC and HOBt to give an optically active compound (4a-4).

Finally, a compound (4a-4) is hydrogenated under an atmosphere of hydrogen with Pd/C 10% to give [4-(N-hydroxyamino)-2(R)-isobutyl-3(S)-methylsuccinyl]-L-phenylglycine-N-methylamide (compound 4a).

〈Process 2〉

A compound of formula (I), wherein Q is a group (II) and A is N-hydroxyaminocarbonyl; $R^1$ is hydrogen, C1-6 alkyl, phenyl, or phenyl($C_1$-$C_6$ alkyl); $R^2$ is hydrogen, $C_1$-$C_6$ alkyl, phenyl, phenyl($C_1$-$C_6$ alkyl), cycloalkyl, or cycloalkyl($C_1$-$C_6$); $R^3$ is $C_1$-$C_6$ alkyl, benzyloxy($C_1$-$C_6$ alkyl), benzyl, benzyloxybenzyl, or ($C_1$-$C_6$ alkoxy)benzyl; $R^4$ is $C_1$-$C_6$ alkyl; and $R^5$ is hydrogen,
for example a compound of compounds 23-27, is prepared by the method described in US Patent 4743587 (Seale) or the method according to that publication.

〈Process 3〉

A compound of formula (I), wherein Q is a group (II) and A is N-hydroxyaminocarbonyl; $R^1$ and $R^2$ are the same or different and are hydrogen, or $C_1$-$C_6$ alkyl; $R^3$ is substituted or unsubstituted bicycloarylmethyl, $R^4$ is hydrogen, substituted or unsubstituted $C_1$-$C_{12}$ alkyl, or $C_6$-$C_{12}$ aryl, and $R^5$ is hydrogen, or -$NR^4R^5$ is amino acid residue, its amide, cyclic amine, or heterocyclic amine,
for example a compound of compounds (28-30), is prepared by the method described in PCT Patent Publication WO 92/09556 (Glycomed) or the method according to that publication.

〈Process 4〉

A compound of formula (I), wherein Q is a group (II) and A is N-hydroxyaminocarbonyl; $R^1$ is $C_1$-$C_6$ alkyl which may be substituted by a member selected from a group consisting of mercapto, $C_1$-$C_6$ alkylthio, $C_6$-$C_{12}$ arylthio, or acylthio; $R^2$ is $C_1$-$C_6$ alkylthio, $C_1$-$C_6$ alkylsulfinyl, $C_1$-$C_6$ alkylsulfonyl, $C_1$-$C_6$ alkylthioalkyl, or $C_1$-$C_6$ alkoxyalkyl; $R^3$ is $C_1$-$C_6$ alkoxybenzyl; $R^4$ is $C_1$-$C_6$ alkyl; and $R^5$ is hydrogen,
for example a compound of compounds 31-34, is prepared by the method described in PCT Patent Publication WO 93/09090 (Yamanouchi) or the method according to that publication.

〈Process 5〉

A compound of formula (I), wherein Q is a group (II) and A is N-hydroxyaminocarbonyl, phosphono, or carboxylyl; $R^1$ is hydrogen, alkyl, alkenyl, $C_6$-$C_{12}$ aryl, heteroaralkyl, or hetero $C_6$-$C_{12}$ arylthioalkyl; $R^2$ is optionally substituted alkyl, alkenyl, cycloalkyl, cycloalkylalkyl, $C_6$-$C_{12}$ aryl, or aralkyl; $R^3$ is alkyl, cycloalkylalkyl, or cycloalkenylalkyl; $R^4$ is alkyl, alkanoyl, $C_6$-$C_{12}$ aryl, aralkyl, morpholinosulfonylaminoalkyl, alkylaminosulfonylaminoalkyl, or aminosulfonyl $C_6$-$C_{12}$ aryl($C_1$-$C_6$ alkyl); and $R^5$ is hydrogen,
for example a compound of compounds 35-48, is prepared by the method described in PCT Patent Publications WO 93/24449 and WO 95/04033 (Celltech) and European Patent 489579 (Celltech), or the method according to these publications.

〈Process 6〉

A compound of formula (I), wherein Q is a group (II) and A is N-hydroxyaminocarbonyl; $R^1$ is hydrogen, amino, hydroxy, mercapto, $C_1$-$C_6$ alkyl, $C_1$-$C_6$ alkoxy, $C_1$-$C_6$ alkylamino, $C_1$-$C_6$ alkylthio, $C_6$-$C_{12}$ aryl($C_1$-$C_6$ alkyl), amino($C_1$-$C_6$ alkyl) optionally protected, hydroxy($C_1$-$C_6$ alkyl) optionally protected, mercapto($C_1$-$C_6$ alkyl) optionally protected, or carboxyl($C_1$-$C_6$ alkyl) optionally protected; $R^2$ is $C_2$-$C_6$ alkyl; $R^3$ is $\alpha$-amino acid residue; $R^4$ is hydrogen, $C_1$-$C_6$ alkyl, alkoxyalkyl, carboxyalkyl, alkoxycarbonylalkyl, alkylaminocarbonylalkyl, or $C_6$-$C_{12}$ arylaminocarbonylalkyl; and $R^5$ is hydrogen, or methyl,
for example a compound of compounds (49-56), is prepared by the method described in European Patent 236872, 497192 or 575844 (Roche) or the method according to these patents.

〈Process 7〉

A compound of formula (I), wherein Q is a group (II) and A is N-hydroxyaminocarbonyl, or carboxyl; $R^1$ is $C_1$-$C_6$ alkylthio $C_1$-$C_6$ alkyl, ($C_6$-$C_{12}$ arylthio($C_1$-$C_6$ alkyl), phenyl($C_1$-$C_6$ alkyl)thio $C_1$-$C_6$ alkyl, heterocyclylthio $C_1$-$C_6$ alkyl, $C_6$-$C_{12}$ aryl, hydroxy, $C_1$-$C_6$ alkylsulfinylalkyl, $C_6$-$C_{12}$ arylsulfinyl $C_1$-$C_6$ alkyl, phenyl($C_1$-$C_6$ alkyl)sulfinyl$C_1$-$C_6$ alkyl, heterocyclylsulfinyl $C_1$-$C_6$ alkyl, $C_1$-$C_6$ alkylsulfonyl $C_1$-$C_6$ alkyl, ($C_6$-$C_{12}$ arylsulfonyl)$C_1$-$C_6$ alkyl, phenyl $C_1$-$C_6$ alkylsulfonyl$C_1$-$C_6$ alkyl, or heterocyclylsulfonyl($C_1$-$C_6$ alkyl); $R^2$ is hydrogen, $C_1$-$C_6$ alkyl, $C_1$-$C_6$ alkenyl, phenyl $C_1$-$C_6$

alkyl, cycloalkyl($C_1$-$C_6$ alkyl), or cycloalkenyl($C_1$-$C_6$ alkyl); $R^3$ is $C_1$-$C_6$ alkyl, $C_2$-$C_6$ alkenyl, substituted phenylmethyl, cycloalkyl, glycosyloxy $C_1$-$C_6$ alkyl, or N-glycosylcarbamoyl $C_1$-$C_6$ alkyl; $R^4$ is hydrogen, $C_1$-$C_6$ alkyl optionally substituted, $C_6$-$C_{12}$ aryl $C_1$-$C_6$ alkyl, ($C_1$-$C_6$ alkoxycarbonyl)$C_1$-$C_6$ alkyl, or N-substituted carbamoyl $C_1$-$C_6$ alkyl; and $R^5$ is hydrogen, or methyl,
for example, a compound of compounds 57-78, is prepared by the method described in PCT Patent Publication WO 94/21625, WO 94/24140, British Patent Publication GB 2268934 (British Bio-Technology) or the method according to these publications.

⟨Process 8⟩

A compound of formula (I), wherein Q is a group (IV) and A is N-hydroxyaminocarbonyl; $R^1$ is hydrogen; and $R^2$ is $C_1$-$C_6$ alkyl, for example compound 79, is prepared by the method described in Japanese Patent Publication (A) No. 145148/1994 (Kanebo Ltd.).

⟨Process 9⟩

A compound of formula (I), wherein Q is a group (III) and A is N-hydroxyaminocarbonyl; $R^1$ is hydrogen, amino $C_1$-$C_6$ alkyl, phthalimido $C_1$-$C_6$ alkyl, thienylthio $C_1$-$C_6$ alkyl, phenylthio $C_1$-$C_6$ alkyl, $C_2$-$C_6$ alkanoylthio $C_1$-$C_6$ alkyl, mercapto $C_1$-$C_6$ alkyl; $R^2$ is $C_1$-$C_6$ alkyl, $C_1$-$C_6$ alkoxy $C_1$-$C_6$ alkyl, or phenyl $C_1$-$C_6$ alkyl which may be substituted; and $R^6$ is hydrogen, hydroxy, $C_1$-$C_6$ alkoxy, or ($C_1$-$C_6$ alkoxy) $C_1$-$C_6$ alkoxy,
for example a compound of compounds 84-87, is prepared by the method described in Japanese Patent Publication (A) No. 157470/1995 (Otsuka Seiyaku) or the method according to that publication.

⟨Process 10⟩

A compound of formula (I), wherein Q is a group (V) and A is N-hydroxyaminocarbonyl, or carboxyl; $R^1$ is hydrogen, $C_1$-$C_6$ alkyl, hydroxy, amino, aminomethyl, or $C_1$-$C_6$ alkylsulfonyl; and $R^2$ is $C_1$-$C_6$ alkyl, cycloalkyl, cycloalkyl $C_1$-$C_6$ alkyl, or aralkyl,
is prepared by the method described in PCT Patent Publication WO 95-047535 or a literature [Bioorganic & Medicinal Chemistry Letters, Vol.5, No. 13, pp. 1415-1420 (1995)] or the method according to these publications.

⟨Process 11⟩

A compound of formula (I), wherein Q is a group (IV) and A is N-hydroxyaminocarbonyl; $R^1$ is $C_1$-$C_6$ alkyl, heterocyclylalkyl, or ($C_6$-$C_{12}$ arylthio)$C_1$-$C_6$ alkyl; and $R^2$ is $C_1$-$C_6$ alkyl,
for example, a compound of compounds 80-83, can be prepared by the method shown in the following Scheme 3 or the method according to this Scheme.

Scheme 3

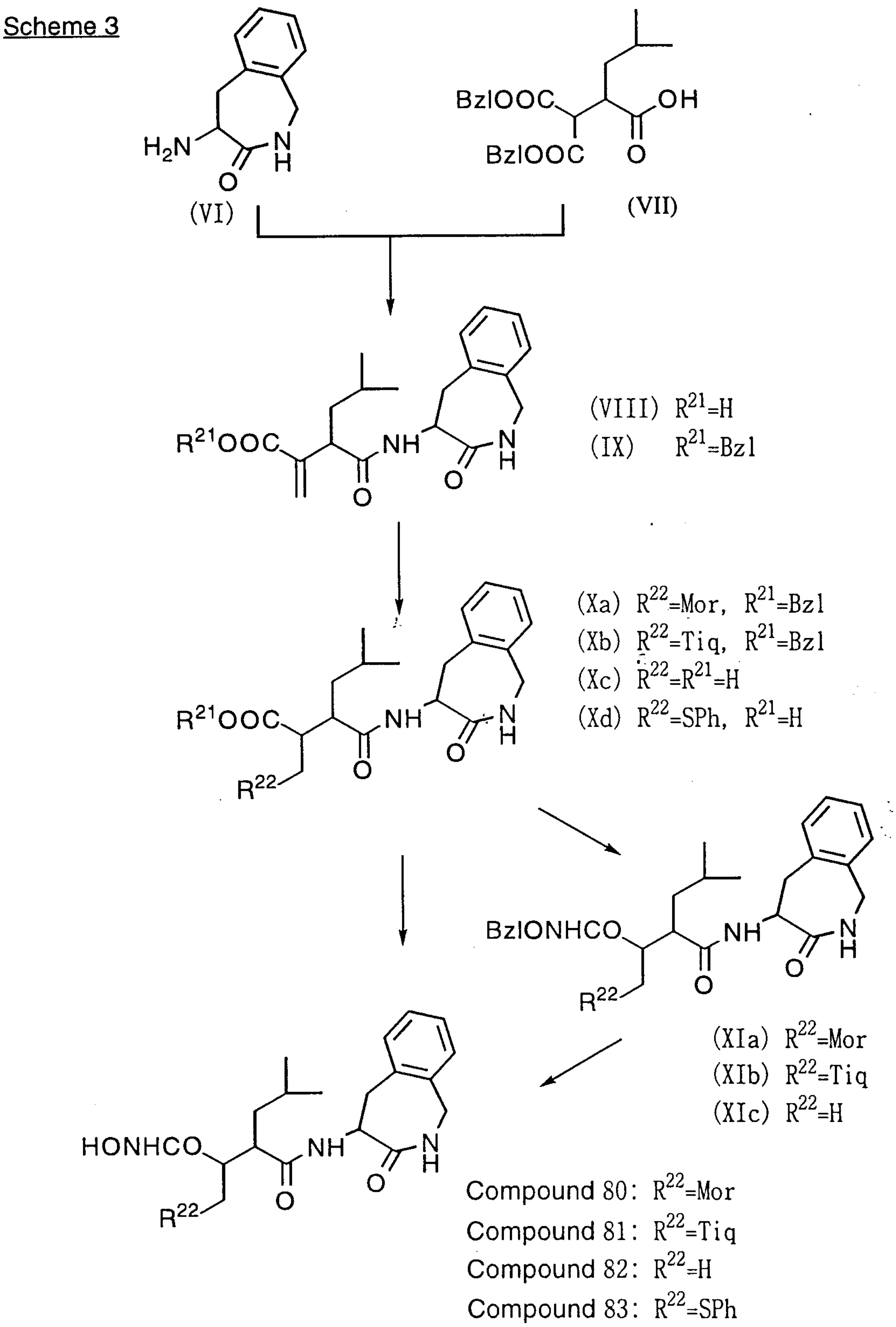

(VI)

(VII)

(VIII) $R^{21}$=H

(IX) $R^{21}$=Bzl

(Xa) $R^{22}$=Mor, $R^{21}$=Bzl

(Xb) $R^{22}$=Tiq, $R^{21}$=Bzl

(Xc) $R^{22}$=$R^{21}$=H

(Xd) $R^{22}$=SPh, $R^{21}$=H

(XIa) $R^{22}$=Mor

(XIb) $R^{22}$=Tiq

(XIc) $R^{22}$=H

Compound 80: $R^{22}$=Mor

Compound 81: $R^{22}$=Tiq

Compound 82: $R^{22}$=H

Compound 83: $R^{22}$=SPh

wherein Bzl is benzyl; Mor is morpholino; Tiq is 1,2,3,4-tetrahydro-2-isoquinolyl; and SPh is phenylthio.

Compounds 80 and 81 are, for example, prepared by the following method.

First, a compound (VI) (see Japanese Patent Publication (A) No. 145148/1994) is condensed with a compound (VII) (see PCT Patent Publication WO 95/04715). This reaction is carried out in an aprotic solvent, such as DMF, tetrahydrofuran (THF), dichloromethane etc. in the presence of condensing agents which are usually used in the peptide synthesis, such as dicyclohexylcarbodiimide (DCC), WSC, etc., and further preferably in the presence of additives, such as HOBt etc. This reaction is usually at 0°C-room temperature for 2-24 hours. The mole ratio per 1.0 mol of a compound (VII) is 0.8-1.5 mol of a compound (VII), 1.0-1.5 mol of a condensing agent, and 0.1-1.5 mol of an additive. And then the resulting condensed product is subjected to hydrogenolysis according to the scheme described in Japanese Patent Publication (A) No. 101925/1995, and the product is reacted with formaldehyde to give a compound (VIII).

A α-methlenecarboxylic acid derivative (IX) is allowed to react with benzylbromide (one to 5 mols) in an aprotic solvent such as DMF etc. in the presence of an inorganic base (e.g. sodium hydrogen carbonate, potassium hydrogen carbonate, etc.) at room temperature for 2-24 hours, to give a benzyl ester (IX).

Next, a compound (IX) is allowed to react with morpholine or 1,2,3,4-tetrahydroisoquinoline to give a compound (Xa) or a compound (Xb). This reaction is preferably carried out in a solvent such as THF etc. or without a solvent, at room temperature - 100°C for several hours-24 hours, by using one to 30 mol of morpholine or 1,2,3,4-tetrahydroisoquinoline, per one mol of a compound (IX).

Next, a compound (Xa) or a compound (Xb) is subjected to hydrogenolysis to give a corresponding carboxylic acid derivative, and the compound is condensed with O-benzyl hydroxylamine to give a compound (XIa) or a compound (XIb).

The hydrogenolysis is usually carried out under an atomosphere of hydrogen at pressure or without pressure at room temperature -60°C with a hydrogenation catalyst, such as Pd/C et al. in a solvent of a lower alkanol, such as methanol, ethanol, etc. or a mixture of an alcohol and 1,4-dioxane, and if necessary, by adding water, hydrochloric acid, acetic acid , etc. In this reaction, formic acid or ammonium formate instead of hydrogen gas can be used.

Compound 80 or 81 is obtained by hydrogenolyzing under the condition mentioned above, a compound (XIa) or a compound (XIb) which are obtained by the above condensation reaction.

Compound 82 is prepared by, for example, the following method.

First, a compound (VIII) can be subjected to catalytic reduction. This reduction is carried out by applying the same reaction condition as the reaction condition on the above mentioned hydrogenolysis. Then, the resulting compound is condensed with O-benzyl hydroxylamine in the same manner as in preparation of compound 80 or 81, followed by hydrogenolysis to give compound 82.

Compound 83 is prepared by, for example, the following method.

First, a compound (Xd) is prepared by reacting a compound (VIII) and thiophenol. This reaction is preferably carried out by using 5 mol equivalent-50 mol equivalent of thiophenol, without a solvent at room temperature -60°C overnight.

Then, the compound (Xd) is condensed with hydroxylamine under a condensing agent, such as DCC, WSC, etc. to afford compound 83.

To get an optically active compound, the above reaction is carried out by using an optically active compound (VIII) or (IX) as a starting material to obtain a mixture of steric isomers of an intermediate (Xa)-(Xd) or (XIa)-(XIc), or a final compound 80-83, and then, the mixture is separated and purified by recrystallization or various column chromatographies (HPLC, preparative thin-layer chromatography, etc.)

A compound of the generic formula (I) includes a compound of the following formula (VI),

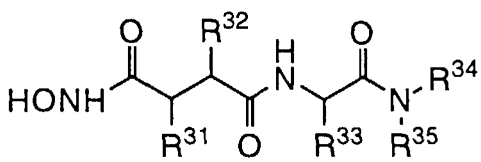

(VI)

wherein $R^{31}$ is hydrogen, methyl, heterocyclylmethyl, or phenylthiomethyl; $R^{32}$ is $C_1$-$C_6$ alkyl, or ($C_6$-$C_{12}$ aryl)$C_1$-$C_6$ alkyl; $R^{33}$ is cyclohexyl, 1-naphthyl, 2-naphthyl, 1-naphthylmethyl, 2-naphthylmethyl, 1-(5,6,7,8-tetrahydronaphthyl)methyl, phenethyl, substituted benzyl, 3-benzothienyl, or phenyl which may be substituted; $R^{34}$ and $R^{35}$ are the same or different and are hydrogen, or $C_1$-$C_6$ alkyl, for example compounds 9-22, 88-91.

These compounds can be prepared by scheme 4 or scheme 5 below or according to these Schemes.

〈Process 12〉

Compounds 9-22 are prepared by, for example the following Scheme 4.

Scheme 4

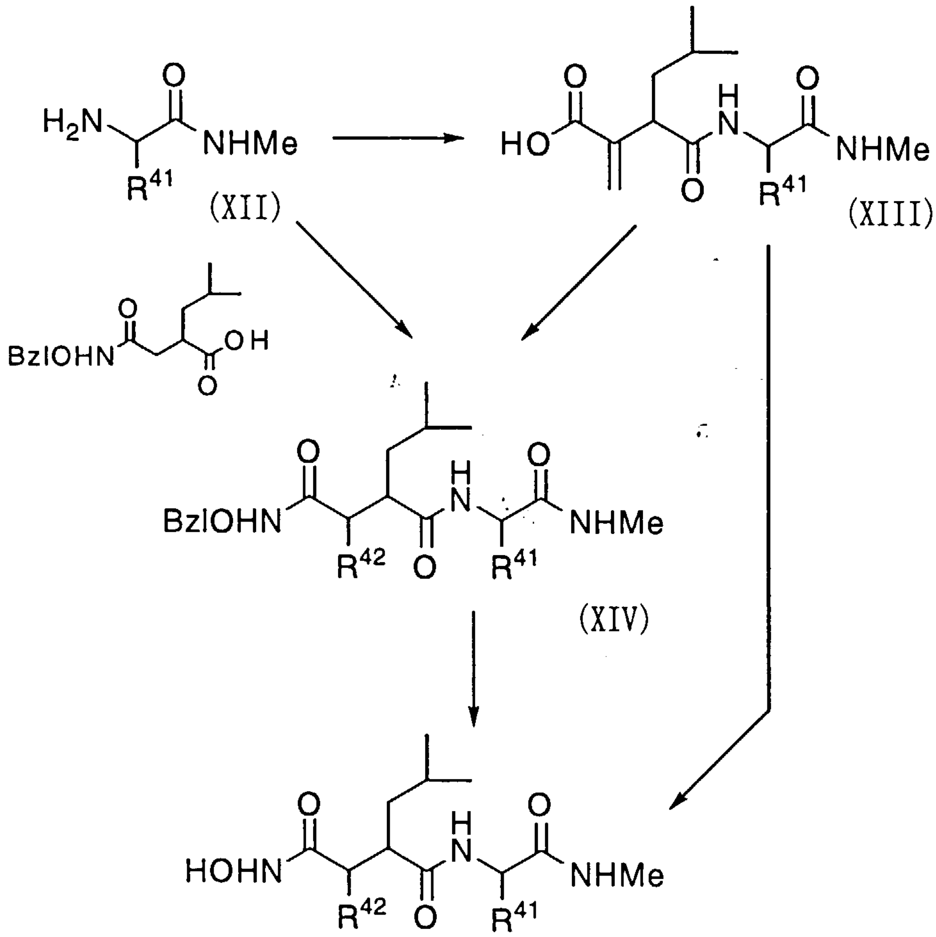

| $R^{42}$ | $R^{41}$ |
|---|---|
| compound 9:H | cyclohexyl |
| compound 10:methyl | cyclohexyl |
| compound 11:H | 2-naphthyl |
| compound 12:morpholinomethyl | cyclohexyl |
| compound 13:H | 1-naphthylmethyl |
| compound 14:H | 2-naphthylmethyl |
| compound 15:TIM | 1-naphthylmethyl |
| compound 16:morpholinomethyl | 1-naphthylmethyl |
| compound 17:methyl | 1-naphthylmethyl |
| compound 18:methyl | TNM |
| compound 19:H | phenethyl |

(continued)

| $R^{42}$ | $R^{41}$ |
|---|---|
| compound 20:H | p-fluorobenzyl |
| compound 21:H | 3-benzothienylmethyl |
| compound 22:phenylthiomethyl | phenyl |

wherein $R^{41}$ is cyclohexyl, phenyl, 1-naphthyl, 2-naphthyl, 1-naphthylmethyl, 2-naphthylmethyl, 5,6,7,8-tetrahydro-2-naphthylmethyl(TNM), 3-benzothienylmethyl, phenethyl, or p-fluorobenzyl; and $R^{42}$ is hydrogen, methyl, morpholinomethyl, (1,2,3,4-tetrahydroisoquinolin-2-yl)methyl(TIM), or phenylthiomethyl.

Compounds 9, 11, 13, 14, 19-21 are prepared by condensing a amino acid derivative (XII) and 3-benzyloxyaminocarbonyl-2-(2-methylpropyl)propionic acid (see Japanese Patent Publication (A) No. 154148/1994), followed by hydrogenlyzing the resulting compound (XIV).

Compounds 10, 12, 15-18 are obtained by preparing a compound (XIV) via an α-methylene derivative (XIII) from an amino acid derivative (XII), followed by hydrogenolysis of the compound (XIV).

In any route, the condition of the condensing reaction, the synthesis of α-methylene derivative and hydrogenolysis are same as the condition in case of preparing compounds 80-83 mentioned above.

Compound 22 is prepared, in the same manner as in preparing compound 83, by reacting an α-methylene derivative (XIII) and thiophenol and then condensing the resulting carboxylic acid derivative with N-hydroxylamine.

A compound of the generic formula (VI) mentioned above includes a novel compound of the following formula (VIa)

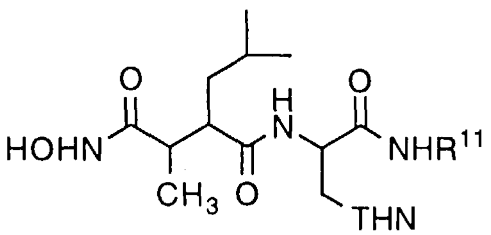

(VIa)

wherein $R^{11}$ is $C_1$-$C_6$ alkyl; and THN is 5,6,7,8-tetrahydro-1-naphthyl, or 5,6,7,8-tetrahydro-2-naphthyl.

A compound (VIa) can be prepared according to the method in Scheme 4, and can be also prepared by the following Scheme 4-1.

Scheme 4-1

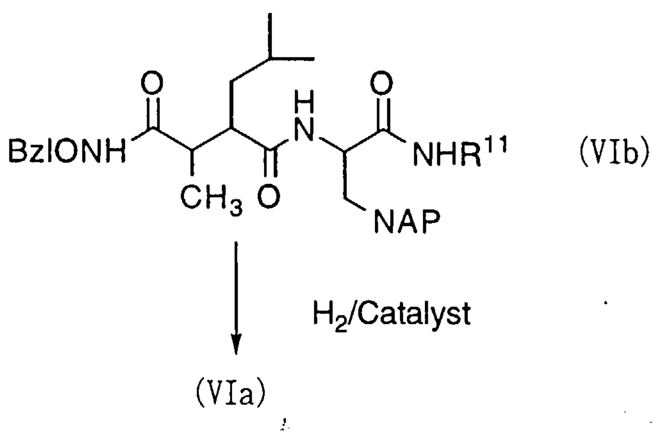


wherein NAP is 1-naphthyl or 2-naphthyl, and $R^{11}$ and Bzl are as defined above.

A compound (VIa) is obtained by stirring a naphthyl derivative (VIb) in acetic acid under an atmosphere of hydrogen with a reduction catalyst, such as Pd/C 10%, Pt, etc. under the pressure or without pressure at room temperature - 60°C for 5-24 hours.

〈Process 13〉

Compounds 88-91 are prepared according to the following Scheme 5.

Scheme 5

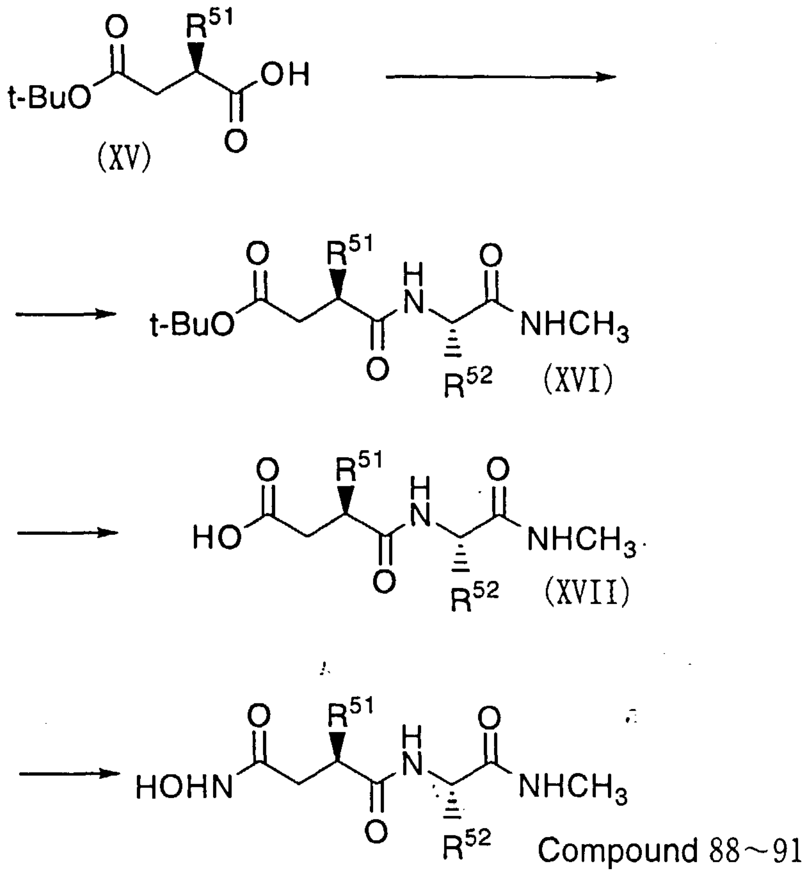

(XV)

(XVI)

(XVII)

Compound 88~91

wherein $R^{51}$ is 2-phenylethyl or 3-phenylpropyl; $R^{52}$ is phenyl or 1-naphthylmethyl; and t-But is tert-butyl.

A compound (XVI) is prepared by condensing an optically active carboxylic acid derivative (XV) and L-phenylglycine-N-methylamide or L-3-(1-naphthyl)alanine-N-methylamide in the presence of a condensing agent, such as DCC, WSC, etc.(in the same manner as in compounds 80-83).

Then, tert-butyl ester on the compound (XVI) is subjected to acidlysis by using trifluoroacetic acid etc. to give a carboxylic acid (XVII).

And then, after preparing a mixed anhydride of the carboxylic acid, it is reacted with hydroxylamine to give compounds 88-91.

The mixed anhydride is prepared by dissolving a compound (XVII) in an aprotic solvent such as THF etc. and adding an equivalent tertiary amine such as triethylamine, N-methylmorpholine, etc. to it, and further adding to the mixture, 1.0-1.2 equivalents of a chlorocarbonate, such as ethyl chloroformate etc., or an acid chloride, such as pivaloylchloride etc. at preferably -20- -5°C.

An optically active carboxylic acid (XV) used as a starting material in the above reaction, is prepared as the following Scheme.

Scheme 6

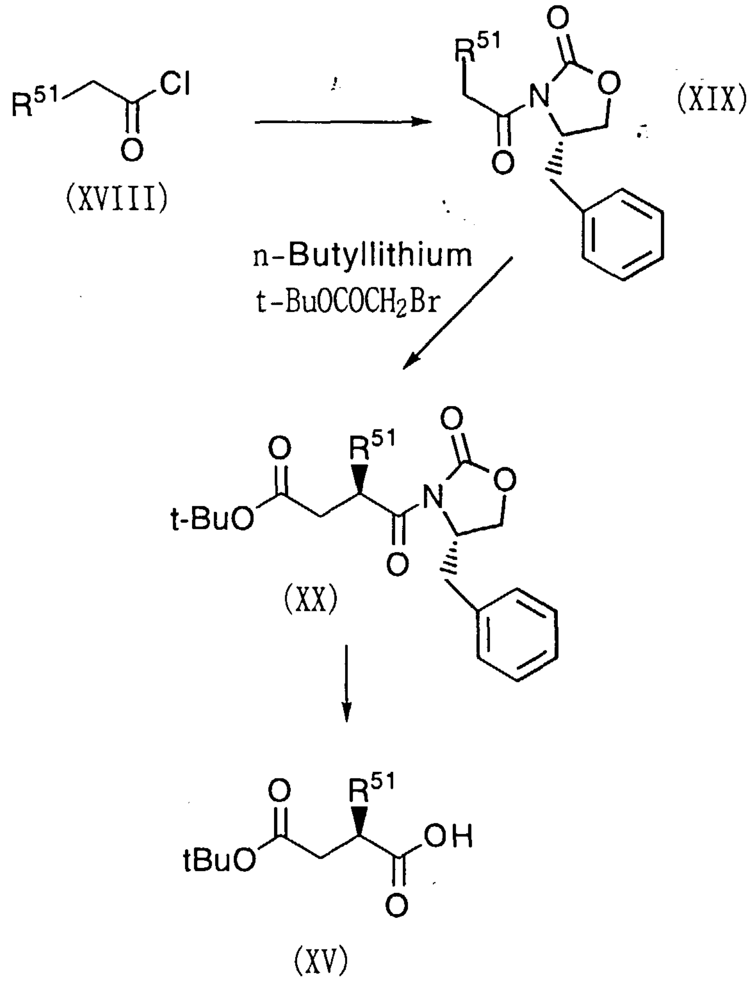


(XVIII)

(XIX)

(XX)

(XV)

wherein Oxz(S) is 4-(S)-benzyl-oxazolidinone, and t-Bu and $R^{51}$ are as defined above.

4-(S)-Benzyl-oxazolidinone is dissolved in an anhydrous aprotic solvent such as THF etc., the solution is reacted with a strong base such as n-butyllithium and then is reacted with an acid chloride (XVIII) to give a compound (XIX).

And then the compound (XIX) is reacted with a strong base such as n-butyllithium etc. and is reacted with tert-butyl bromoacetate to give a compound (XX). The compound (XX) is subjected to oxidative degradation with 30% hydrogen peroxide in an alkali such as lithium hydride to give an optically active carboxylic acid (XV).

Other compounds included in a generic formula (I) can be prepared according to the methods mentioned above.

The Fas L solubilization inhibitor of the present invention comprising, as an active ingredient, a compound of the generic formula (I) including its pharmaceutically acceptable salt, its hydrate, its solvate and its prodrug, is administered orally or parenterally, according to the conventional method to human being for the treatment and prevention of diseases caused by the excessively activated T cell, for example fulminant hepatitis, graft-versus-host diseases (GVHD), acquired immunodeficiency syndrome (AIDS), autoimmune diseases, etc.

Preparations for oral administration include solid preparations such as tablets, granules, powders, subtilized granules, hard capsules, etc., and liquids such as syrups, soft capsules etc. Such preparations are prepared by the conventional method, and for example, tablets, granules, powders and subtilized granules are prepared by mixing a compound of the generic formula (I) and pharmaceutical additives conventionally used, such as lactose, starch, crystalline cellulose, magnesium stearate, hydroxypropylcellulose, talc, etc. The hard capsules are prepared by filling the above granules or powders into a capsule.

Syrups are prepared, for example, by dissolving or suspending a compound of the generic formula (I) in an aqueous solution containing sucrose, carboxycellulose, etc. Soft capsules are prepared, for example, by dissolving or suspending a compound of the generic formula (I) in a lipid excipient, such as plant oil, oil emulsion, glycol, etc. and filling it into a soft capsule.

Preparations for parenteral administration are illustrated as injections, suppositories, such as suppositories, vaginal

suppositories, etc., and pernasal preparations such as aerosol. These preparations are prepared by the conventional method. For example, injections are prepared by dissolving or suspending a compound of the generic formula (I) in sterilized water and by adding to it a tonicity agent, such as mannitol, sodium chloride, glucose, sorbit, glycerol, xylitol, fructose, maltose, etc., and if necessary, by adding a stabilizing agent, such as sodium sulfite, albumin etc., and a preservative such as benzylalcohol etc. and then by sealing aseptically the solution or suspension into an ampoule or a vial.

A dosage of a compound of the generic formula (I) or its pharmaceutically acceptable salt varies depending on age, sex, body weight, and disease of patients, but 0.1-100 mg / kg is suitable, when its amount is calculated in a compound of the generic formula (I) without salts or solvates, and its amount is administered orally or parenterally once a day, or its amount is dividedly administered two to three times a day.

EFFECT OF INVENTION

A compound of the generic formula (I) or its pharmaceutically acceptable salt showed inhibitory activity of Fas L solubilization, and also exhibited the excellent prophylaxis and treating effect in the test using GVHD induced animals.

And a compound of the generic formula (I) or its pharmaceutically acceptable salt was low in toxicity. For example, when compound 4a ( 3,000mg/kg:body weight) was orally administered, the mortality was not found. Therefore, a compound of the generic formula (I) or its pharmaceutically acceptable salt is useful for preventing and treating the diseases in which apoptosis caused by binding the solubilized Fas L with Fas is considered to participate, for example, fulminant hepatitis, GVHD, AIDS, autoimmune diseases, etc.

A compound of the generic formula (I) or its salt shows excellent inhibitory activity of Fas L solubilization and therefore, is useful for a reagent on the test and study related to Fas L, too.

The inhibitory activity of Fas L solubilization on a compound of the generic formula (I) is explained by following experiments.

〈Test 1〉Test for inhibition of Fas L solubilization

(1) Test sample

Test sample A: [4-(N-hydroxyamino)-2(R)-isobutylsuccinyl]-L-O-methyltyrosine-N-methylamide (compound 23)
Test sample B: [4-(N-hydroxyamino)-2(R)-isobutyl-3(S)-phenylthiomethylsuccinyl]-L-phenylalanine-N-methylamide (compound 58)
Test sample C: [4-(N-hydroxyamino)-2(R)-isobutyl-3(S)-methylsuccinyl]-L-O-methylthylosine-N-methylamide (compound 24)
Test sample D: [4-(N-hydroxyamino)-2(R)-isobutyl-3(S)-(2-thienylthiomethyl)succinyl]-L-phenylalanine-N-methylamide (compound 57)
Test sample E: [4-(N-hydroxyamino)-2(R)-isobutyl-3(S)-methylsuccinyl]-L-phenylglycine-N-methylamide (compound 4a)
Test sample F: [4-(N-hydroxyamino)-2(R)-isobutylsuccinyl]-L-2-naphthylglycine-N-methylamide (compound 11: compound of preparation 2)
Test sample G: [4-(N-hydroxyamino)-2(R)-(3-phenylpropylsuccinyl]-L-3-cyclohexylalanine-N-[2-(4-sulfonamidephenyl)ethyl]amide (compound 48)
Test sample H: [4-(N-hydroxyamino)-2(R)-isobutyl-3-methylsuccinyl]-L-3-(5,6,7,8-tetrahydro-1-naphthyl)alanine-N-methylamide (compound 18: compound of preparation 10)
Test sample I: [4-(N-hydroxyamino)-2(R)-isobutyl-3-methylsuccinyl]-L-3-(1-naphthyl)alanine-N-methylamide (compound 17: compound of preparation 9)

(2) Test method

hFas L/BHK-21 (BHK-21 cells in which human Fas L cDNA was transfected)(3.5 x $10^5$) was suspended in 1ml of RPMI 1640 culture medium containing 10% fetal bovine serum, and in 24 wells culture plate (Falcon 3047, Becton Dickenson Co., Ltd.) each test sample adjusted with RPMI 1640 containing 10% fetal bovine serum at the final concentration 5, 2.5, 1.25, and 0.625 μM was incubated under 5% carbon dioxide atmosphere at 37°C for 24 hours, to obtain a culture supernatant.

The concentration of sFas L in the supernatant thus obtained was quantitatively analyzed by the sandwich-ELISA method as shown below.

The concentration of anti Fas L antibody (NOK3)(see preparation 17) was adjusted to 10μg/ml with 0.05M sodium carbonate-sodium hydrogen carbonate buffer solution (pH 9.6), and each 50μl thereof was poured in each well of a 96-wells microplate (Immunolon 2, Dinatech Co., Ltd.) and left it overnight at 4°C to make the solid phase. Each well was washed twice with 300 μl of a washing solution consisting of a physiological saline solution adjusted at pH 7.4 with a phosphate buffer solution, containing 0.05% of Tween 20 (polyoxyethylene sorbitan monolaurate)(abbreviated as T-PBS), and then to it was added 300μl of PBS (pH 7.4) containing bovine serum albumin (abbreviated as BSA, Wako Chemical Co., Ltd), and it was left for 2 hours at 37°C to remove the supernatant.

Then, the culture supernatant (50μl) containing a test sample were added to each well, and incubated for one hour at room temperature. After incubation, each well was washed twice with 300μl of T-PBS (pH7.4).

The concentration of anti Fas L antibody (NOK1) labeled with biotin (see preparation 18) was adjusted to 5μg/ml by diluting with PBS (pH 7.4) containing 1% BSA, and 50μl thereof was added to the solution prepared above. The solution was reacted for one hour at room temperature, and washed twice with 300μl of T-PBS (pH 7.4).

And then, streptoavidin-peroxidase (Amasham Lab.) was diluted 1000 times with PBS (pH 7.4) containing 1% of BSA, and 50μl thereof was added to it. The solution was incubated for 30 minutes at room temparature and was washed twice with 300μ of T-PBS (pH 7.4).

Further, 100μl of 0.15M citric acid-sodium phosphate buffer solution (pH 5.0) containing hydrogen peroxide and o-phenylenediamine (former concentration:0.015 w/v%, latter concentration:0.2mg/ml) was added to each well and incubated for 10 minutes at room temperature.

After incubation, 50μl of 2 N sulfuric acid was added to each well to quench the reaction, and the absorbance at 490 nm was measured by Softmax (Molecular Device Co., Ltd.)

On the other hand, by measuring the absorbance of the sFas L solution of which the concentration was known, as a sample instead of the culture supernatant, the calibration curve was detected based on 4-logistic parameter analysis, and based on it the concentration (X) of sFas L in the culture supernatant was calculated.

As a control, by using the concentration (A) of sFas L in the culture supernatant in case that the test sample was not added, based on the concentration (X) of sFas L in the culture supernatant in case that the sample was added, sFas secretion inhibition rate (%) at 5μM was calculated by using the following formula

$$\text{sFas L secretion inhibition rate (\%)} = (1-X/A) \times 100$$

Then, in cases of test samples B, C, D and G that showed the inhibition rate more than 70%, sFas L secretion inhibitory rate (%) at 2.5, 1.25, and 0.625 μM as well, was measured, and IC50 (μM) was calculated by the probit method.

(3) Test result

sFas L secretion inhibitory rate (%) at 5μM is shown in Table 1.

Table 1

| Test sample | aFas L secretion inhibition rate (%) |
|---|---|
| Test sample A | 47 |
| Test sample B | 73 |
| Test sample C | 72 |
| Test sample D | 71 |
| Test sample E | 29 |
| Test sample F | 35 |
| Test sample G | 72 |
| Test sample H | 57 |
| Test sample I | 50 |

IC50 on test samples B, C, D and G (μM) is shown in Table 2.

Table 2

| Test sample | IC50 (μM) |
|---|---|
| Test sample B | 2.2 |
| Test sample C | 1.5 |
| Test sample D | 1.6 |
| Test sample G | 1.1 |

〈Test 2〉 Recovery test of GVHD induced mouse

(1) Test sample

Test sample E: [4-(N-hydroxyamino)-2(R)-isobutyl-3(S)-methylsuccinyl]-L-phenylglycine-N-methylamide (compound 4a)

(2) Test method

CBF1 mice (F1 mice of BALB/c and C57BL/6) were injected with splenocytes ($5x10^7$) of C57BL/6 mouse, and a week later were again injected with splenocytes $5x10^7$ to induce GVHD into a mouse.

A test sample was suspended in 0.5% CMC solution (test sample 10mg vs the CMC solution 1ml), 2mg of the test sample / mouse / day was subcutaneously administered every day for 2 weeks beginning from injection of splenocytes to the above GVHD induced mice, and this group was compared with the GVHD induced group in which any test sample was not administered (no drug administered group) and the GVHD non-induced group (non-treated group).

The body weight of mice (g) was measured every week a week later after the first injection of splenocytes, for 6 weeks, and the total IgE amount (ng/ml) was measured every week a week later after the first injection of splenocytes, for 9 weeks, and the degree of GVHD was evaluated by change of day.

(3) Test result

The decrease of body weight and the increase of total IgE amount caused by the induced GVHD were clearly prohibited by test samples.

〈Test 3〉 Prevention of the mortality of GVHD induced mouse

(1) Test sample

Test sample: [4-(N-hydroxyamino)-2(R)-isobutyl-3(S)-methylsuccinyl]-L-phenylglycine-N-methylamide (compound 4a)

(2) Test method

GVHD was introduced into CBF1 mouse in the same manner as in Test example 2, except for the numbers of the injected splenocytes, $1x10^8$, and the test sample administered group was compared with the drug non-administered group and the non-treated group.

The effect of test samples was evaluated by the mortality of mouse for 3 weeks after the first introduction of the splenocytes.

(3) Test result

The survival rate on the drug administered group was 90%, after the death of all mice of the drug non-administered group.

EXAMPLE

The present invention is further explained by illustrating Preparations and Examples.

Preparation 1

Preparation for [4-(N-hydroxyamino)-2(R)-isobutylsuccinyl]-L-cyclohexylglycine-N-methylamide (Compound 9)

(1) L-cyclohexylglycine-N-methylamide:

Phenylglycine-N-methylamide (1.15g) which was prepared by the method described in Japanese Patent Publication (A) 101925/1995 (as abbreviated as JP-A-101925/1995) was dissolved in ethanol (50ml), and rhodium chloride (3.0g) was added to the solution and the resulting solution was stirred at 30 °C for 2 hours. To the solution, $NaBH_4$ (2.5g) in ethanol (100ml) was added dropwise over a 35 minutes period and the solution was stirred for 4 hours. After the reaction was completed, the insoluble materials were filtered off and the filtrate was concentrated in vacuo. The residue was dissolved in 1N HCl solution and the solution was washed with ethyl acetate. The aqueous layer was neutralized with sodium carbonate and the solution was extracted with ethyl acetate. The organic layer was dried over magnesium sulfate and concentrated in vacuo to give L-cyclohexylglycine-N-methylamide (700mg) as an oil.

(2) [4-(N-Benzyloxyamino)-2(R)-isobutylsuccinyl]-L-cyclohexylglycine-N-methylamide:

To DMF (20ml) were dissolved L-cyclohexylglycine-N-methylamide (444mg) and [4-(N-benzyloxyamino)-2(R)-isobutylsuccinic acid (730mg) which were prepared by the method described in JP-A-101925/1995, and WSC (750mg) was added to the solution under ice cooling and the solution was stirred overnight. The resulted crystals were filtered to give [4-(N-benzyloxyamino)-2(R)-isobutylsuccinyl]-L-cyclohexylglycine-N-methylamide (725mg) as a colorless solid.
$^1H$-NMR(DMSO-$d_6$) $\delta$ : 0.79 (3H, d, J=6.2Hz), 0.84 (3H, d, J=6.2Hz), 0.85-1.20 (6H, m), 1.32-1.72 (8H, m), 1.97 (1H, dd, J=7.0Hz, 14.4Hz), 2.15 (1H, dd, J=7.4Hz, 14.4Hz), 2.55 (3H, d, J=4.5Hz), 2.75-2.85 (1H, m), 4.05 (1H, t , J=8.3Hz), 4.72 (1H, d, J=10.9Hz), 4.77 (1H, d, J=10.9Hz), 7.37 (5H, s), 7.74-7.85 (2H, m),10.99 (1H, s).

(3) [4-(N-Hydroxyamino)-2(R)-isobutylsuccinyl]-L-cyclohexylglycine-N-methylamide:

Methanol (40ml) and 10% Pd/C (40mg) were added by stirring to [4-(N-benzyloxyamino)-2(R)-isobutylsuccinyl]-L-cyclohexylglycine-N-methylamide (400mg) under hydrogen atmosphere. After the reaction was completed, the catalyst was filtered off and the filtrate was concentrated in vacuo, and the resulted crystals were recrystallized from ethyl acetate/acetone/ethanol to give [4-(N-hydroxyamino)-2(R)-isobutylsuccinyl]-L-cyclohexylglycine-N-methylamide (160mg).
$^1H$-NMR(DMSO-$d_6$) $\delta$ : 0.79 (3H, d, J=6.2Hz), 0.83 (3H, d, J=6.2Hz), 0.80-1.23 (6H, m), 1.33-1.72 (8H, m), 1.99 (1H, dd, J=7.7Hz, 14.3Hz), 2.14 (1H, dd, J=6.7Hz, 14.3Hz), 2.55 (3H, d, J=4.5Hz), 2.70-2.82 (1H, m), 4.03 (1H, t, J=8.1Hz), 7.73-7.83 (2H, m), 8.71 (1H, s), 10.37 (1H, s).

| Analysis for $C_{17}H_{31}N_3O_4$ | | | |
|---|---|---|---|
| Calcud: | C, 59.80; | H, 9.15; | N, 12.31 |
| Found: | C, 59.12; | H, 9.12; | N, 12.15 |

Preparation 2

Preparation for [4-(N-hydroxyamino)-2(R)-isobutylsuccinyl]-L-2-naphthylglycine-N-methylamide (Compound 11)

(1) 2(S)-[(2-Hydroxy-l(R)-phenylethyl)amino]-2-(2-naphthyl)ethanenitrile:

2-Naphthaldehyde (6.26g) was suspended in water (150ml), and $NaHSO_3$ (4.2g) and KCN (2.7g) were added to the suspension under ice cooling. A solution of (R)-2-amino-2-phenylethanol (5.0g) in methanol was added to the mixture and the mixture was stirred for 2 hours and refluxed for 1 hour. The reaction mixture was cooled, extracted with chloroform and the extract was dried over magnesium sulfate and concentrated in vacuo. The residue was purified by silica gel column chromatography to give 2(S)-[(2-hydroxy-1(R)-phenylethyl)amino]-2-(2-naphthyl)ethanenitrile (3.6g) as crystals.
$^1H$-NMR($CDCl_3$) $\delta$ :2.0 (1H, broad s), 2.6 (1H, broad s), 3.8-4.0 (2H, m), 4.2-4.5 (1H, m), 4.6 (1H, s), 7.2-8.1 (12H, m).

(2) N-[2-Hydroxy-l(R)-phenylethyl]-L-(2-naphthyl)glycine methyl ester:

Concentrated hydrochloric acid (45ml) was added to 2(S)-[2-hydroxy-l(R)-phenylethyl) amino]-2-(2-naphthyl)ethanenitrile. The solution was stirred at 100°C for one hour, cooled to room temperature and extracted with chloroform / methanol(10:1). The extract was washed with a saturated aqueous sodium chloride solution, dried over magnesium sulfate, and concentrated in vacuo to give an oil. The oil was dissolved in methanol (50ml), the solution was added to a solution which was prepared by adding dropwise thionyl chloride (4.7g) into methanol (50ml) cooled to -75°C, and then warmed gradually to room temperature under stirring and refluxed further for 1 hour. About two third solvent was removed under reduced pressure and the residue was neutralized with a saturated aqueous sodium hydrogencarbonate solution and extracted with chloroform. The organic layer was dried over magnesium sulfate, concentrated in vacuo and the residue was purified by silica gel column (solvent: ethyl acetate/n-hexane) to give N-[2-hydroxy-1(R)-phenylethyl]-L-(2-naphthyl)glycine methyl ester (3.38g) as an oil.
$^1$H-NMR($CDCl_3$) δ : 2.7 (2H, s), 3.6-4.1 (2H, m), 3.7 (3H, s), 4.5 (1H, s), 7.2-8.0 (12H, m).

(3) L-(2-Naphthyl)glycine:

N-[2-Hydroxy-l(R)-phenylethyl]-L-(2-naphthyl)glycine methyl ester (3.3g) was dissolved in a mixture of chloroform (50ml) and methanol (15ml). Lead tetraacetate (5.3g) was added in portions to the solution at -5-0°C and the solution was stirred for 6 minutes. A saturated aqueous sodium hydrogen carbonate solution (60ml) was added to the solution. After separating the organic layer, the aqueous layer was extracted with chloroform and the extract was combined with the organic layer. The solution was dried over magnesium sulfate and concentrated in vacuo. 6N Hydrochloric acid was added to the residue and the solution was stirred at room temperature overnight, and further stirred for 2 hours at 60°C and for 2 hours at 110°C. After the reaction mixture was cooled, the mixture was washed with ether and neutralized with sodium hydrogen carbonate. The resulting crystals were filtered to give L-(2-naphthyl) glycine (1.6g).

(4) N-Benzyloxycarbonyl-L-(2-naphthyl)glycine-N-methylamide:

L-(2-Naphthyl)glycine (1.6g) was dissolved in 0.1N sodium hydroxide (100ml) and dioxane (20ml). Sodium carbonate (3.37g) and benzyloxycarbonyl chloride (1.63g) were added to the solution under ice cooling and the solution was stirred for 4 hours. The reaction mixture was washed with ether and chloroform was added thereto. The solution was adjusted to pH 1 with conc.hydrochloric acid with stirring and then, the organic layer was separated. The organic layer was dried over magnesium sulfate and concentrated in vacuo to give an oil. The oil was dissolved in tetrahydrofuran (100ml), and triethylamine (1.1g) was added thereto. The solution was cooled to 0°C and ethyl chlorocarbonate (1.16g) was added thereto. The solution was stirred for 1 hour and a methylamine/methanol solution was added thereto and the solution was stirred for 3.5 hours. After the reaction was completed, chloroform was added thereto. The solution was washed with 1N hydrochloric acid and water, dried over magnesium sulfate and concentrated in vacuo to give N-benzyloxycarbonyl-L-(2-naphthyl)glycine-N-methylamide (1.78g).
$^1$H-NMR(DMSO-$d_6$) δ : 2.6 (3H, d, J=4.8Hz), 5.0 (2H, s), 5.3 (1H, d, J=8Hz), 7.2-8.3 (12H, m).

(5) [4-(N-Benzyloxyamino)-2(R)-isobutylsuccinyl]-L-(2-naphthyl)glycine-N-methylamide:

L-(2-Naphthyl)glycine-N-methylamide which was prepared by subjecting N-benzyloxycarbonyl-L-(2-naphthyl)glycine-N-methylamide (500mg) to hydrogenolysis in methanol under hydrogen atmosphere in the presence of 10% Pd/C, was reacted in accordance with the same method as is Preparation 1 (2) to give [4-(N-benzyloxyamino)-2(R)-isobutylsuccinyl]-L-(2-naphthyl)glycine-N-methylamide (308mg) as crystals.
$^1$H-NMR(DMSO-$d_6$) δ : 0.83 (3H, d, J=6.3Hz), 0.89 (3H, d, J=6.3Hz), 1.03-1.16 (1H, m), 1.4-1.58 (2H, m), 1.99 (1H, dd, J=6.7Hz, 14.5Hz), 2.15 (1H, dd, J=7.6Hz, 14.5Hz), 2.60 (3H, d, J=4.5Hz), 2.90-3.01 (1H, m), 4.62-4.70 (2H, m), 5.56 (1H, d, J=7.8Hz), 7.43-7.56 (3H, m), 7.82-7.90 (4H, m), 8.18-8.23 (1H, m), 8.57 (1H, d, J=7.8Hz), 10.97 (1H, s).

(6) [4-(N-Hydroxyamino)-2(R)-isobutylsuccinyl]-L-(2-naphthyl)glycine-N-methylamide:

The crystalline compound obtained above (250mg) was reacted in the same manner as is Preparation 1 (3) to give [4-(N-hydroxyamino)-2(R)-isobutylsuccinyl]-L-(2-naphthyl)glycine-N-methylamide (143mg) as colorless crystals.
$^1$H-NMR(DMSO-$d_6$) δ : 0.83 (3H, d, J=6.3Hz), 0.88 (3H, d, J=6.3Hz), 1.04-1.16 (1H, m), 1.4-1.58 (2H, m), 1.98 (1H, dd, J=7.4Hz, 14.5Hz), 2.15 (1H, dd, J=7.0Hz, 14.5Hz), 2.59 (3H, d, J=4.5Hz), 2.87-2.98 (1H, m), 5.54 (1H, d, J=7.7Hz), 7.46-7.57 (3H, m), 7.82-7.92 (4H, m), 8.17-8.25 (1H, m), 8.56 (1H, d, J=7.7Hz), 8.73 (1H, bs), 10.37 (1H, s).

| Analysis for $C_{21}H_{27}N_3O_4$ | | | |
|---|---|---|---|
| Calcd: | C, 65.44; | H, 7.06; | N, 10.90 |
| Found: | C, 65.28; | H, 7.06; | N, 10.73 |

Preparation 3

Preparation of [4-(N-hydroxyamino)-2(R)-isobutyl-3-morpholinomethylsuccinyl]-L-cyclohexylglycine-N-methylamide (Compound 12)

(1) [4-Benzyloxyamino-2(R)-isobutyl-3-morpholinomethylsuccinyl]-L-cyclohexylglycine-N-methylamide:

[4-(N-Benzyloxyamino)-2(R)-isobutyl-3-morpholinomethylsuccinyl]-L-cyclohexylglycine-N-methylamide (126mg) was prepared from 2-benzyloxy-3(R)-hydroxycarbonyl-5-methyhexanoic acid benzyl ester (see JP-A-101925/1995) (1.75g) and cyclohexylglycine-N-methyamide (cf. Preparation 1 (1) described above) (684g) in the same manner as described in JP-A-101925/1995.
$^1$H-NMR(DMSO-$d_6$) $\delta$ : 0.73 (3H, d, J=6.4Hz), 0.79 (3H, d, J=6.4Hz), 0.8-1.8 (14H, m), 1.96 (1H, dd, J=2.6Hz, 11.8Hz), 2.0-2.10 (2H, m), 2.27-2.68 (5H, m), 2.54 (3H, d, J=4.5Hz), 3.40-3.53 (4H, m), 4.06 (1H, t, J=8.3Hz), 4.77 (1H, d, J=11.4Hz), 4.82 (1H, d, J=11.4Hz), 7.32-7.45 (5H, m), 7.72-7.79 (1H, m), 8.06 (1H, d, J=8.4Hz), 11.04 (1H, s).

(2) [4-(N-Hydroxyamino)-2(R)-isobutyl-3-morpholinomethylsuccinyl]-L-cyclohexylglycine-N-methylamide:

[4-(N-Hydroxyamino)-2(R)-isobutyl-3-morpholinomethylsuccinyl]-L-cyclohexylglycine-N-methylamide (65mg) was produced by subjecting [4-(N-benzyloxyamino)-2(R)-isobutyl-3-morpholinomethylsuccinyl]-L-cyclohexylglycine-N-methylamide (95mg) to hydrogenolysis likewise.
$^1$H-NMR(DMSO-$d_6$) $\delta$ : 0.75 (3H, d, J=6.4Hz), 0.79 (3H, d, J=6.4Hz), 0.85-1.8 (14H, m), 1.93 (1H, dd, J=2.5z, 11.9z), 2.0-2.10 (2H, m), 2.30-2.69 (5H, m), 2.52 (3H, d, J=4.5Hz), 3.43-3.56 (4H, m), 4.06 (1H, t, J=8.3Hz), 7.72-7.78 (1H, m), 8.03 (1H, d, J=8.5Hz), 8.80 (1H, s), 10.39 (1H, s).

| Analysis for $C_{22}H_{40}N_4O_5 \cdot H_2O$ | | | |
|---|---|---|---|
| Calcd: | C, 57.62; | H, 9.23; | N, 12.22 |
| Found: | C, 57.74; | H, 9.15; | N, 12.18 |

Preparation 4

Preparation of [4-(N-hydroxyamino)-2(R)-isobutylsuccinyl]-L-3-(1-naphthyl)alanine-N-methylamide (compound 13)

(1) Boc-L-3-(1-naphthyl)alanine-N-methylamide:

N-Boc-L-3-(1-naphthyl)alanine-N-methylamide (Synthetech Co., Ltd., Oregon U.S.A.) (2g) was dissolved in tetrahydrofuran (50ml), and triethylamine (0.77g) was added, and ethyl chlorocarbonate (0.826g) was added with stirring under ice cooling, and the mixture was stirred for 5 minutes. To the mixture a 40% solution of methylamine in methanol (2ml) was added dropwise, and the mixture was stirred for 3 hours. To the reaction solution, ethyl acetate (100ml) was added, and the mixture was washed with 1N hydrochloric acid, a saturated aqueous sodium bicarbonate and water successively, and was dried over anhydrous magnesium sulfate. It was concentrated in vacuo to give Boc-L-3-(1-naphthyl)alanine-N-methylamide (1.67g) as colorless crystals.
$^1$H-NMR (DMSO-$d_6$) $\delta$ : 1.25 (9H, s), 2.58 (3H, d), 3.11 (1H, dd), 3.51 (1H, dd), 4.2-4.3 (1H, m), 6.98 (1H, d), 7.91 (1H, d), 7.4-8.2 (7H, m).

(2) [4-(N-Benzyloxyamino)-2(R)-isobutylsuccinyl]-L-3-(1-naphthyl)alanine-N-methylamide:

To Boc-L-3-(1-naphthyl)alanine-N-methylamide (0.8g) was added 95% aqueous trifluoroacetic acid (10ml), and the mixture was stirred for 30 minutes. Trifluoroactic acid was distilled off under reduced pressure, and chloroform was added, and the mixture was washed with a saturated aqueous sodium hydrogencarbonate. The mixture was dried over anhydrous magnesium sulfate and concentrated under reduced pressure. The resulting oil was dissolved in DMF (25ml), and thereto were added [4-(N-benzyloxyamino-2(R)-isobutylsuccinic acid (670mg), 1-ethyl-3-(3-dimethylaminopropyl)carbodiimide hydrochloride (WSC) (552mg), and the mixture was stirred at room temperature for 6.5 hours. To the reaction mixture was added chloroform, and the mixture was washed with 1N hydrochloric acid, a saturated aqueous sodium bicarbonate, and water, dried over anhydrous magnesium sulfate, and concentrated under reduced pressure to give [4-(N-benzyloxyamino)-2(R)-isobutylsuccinyl]-L-3-(1-naphthyl)alanine-N-methylamide (680mg) as colorless crystals. $^1$H-NMR(DMSO-$d_6$) $\delta$ : 0.72 (3H, d, J=6.3Hz), 0.77 (3H, d, J=6.3Hz), 0.90-1.02 (1H, m), 1.20-1.38 (2H, m), 1.91 (1H, dd, J=7.1Hz, 14.4Hz), 2.07 (1H, dd, J=7.5Hz, 14.4Hz), 2.55 (3H, d, J=4.5Hz), 2.6-2.7 (1H, m), 3.27 (1H, dd, J=8.7Hz, 13.9Hz), 3.53 (1H, dd, J=5.5Hz, 13.9Hz), 4.48-4.58 (1H, m), 4.76 (2H, s), 7.32-7.42 (7H, m), 7.48-7.59 (2H, m), 7.76 (1H, d, J=7.6Hz), 7.83-7.92 (2H, m), 8.12-8.25 (2H, m), 11.03 (1H, s).

(3) [4-(N-Hydroxyamino)-2(R)-isobutylsuccinyl]-L-3-(1-naphthyl)alanine-N-methylamide:

The crystals obtained in the above (2) (250mg) was dissolved in methanol (50ml), and 10% Pd/C (70mg) was added thereto, and the mixture was stirred under hydrogen atmosphere. The catalyst was removed by filtration and the mixture was concentrated under reduced pressure. The residue was purified by HPLC to give [4-(N-hydroxyamino)-2(R)-isobutylsuccinyl]-L-3-(1-naphthyl)alanine-N-methylamide (100mg).
$^1$H-NMR(DMSO-$d_6$) $\delta$ : 0.71 (3H, d, J=6.4Hz), 0.75 (3H, d, J=6.4Hz), 0.92-1.03 (1H, m), 1.15-1.38 (2H, m), 1.91 (1H, dd, J=7.1Hz, 14.3Hz), 2.08 (1H, dd, J=7.6Hz, 14.3Hz), 2.50-2.60 (1H, m), 2.57 (3H, d, J=4.5Hz), 3.25 (1H, dd, J=9.2Hz, 13.9Hz), 3.58 (1H, dd, J=5.0Hz, 13.9Hz), 4.45-4.56 (1H, m), 7.32-7.42 (2H, m), 7.48-7.60 (2H, m), 7.77 (1H, d, J=6.8Hz), 7.91 (1H, d, J=7.9Hz), 7.93-8.0 (1H, m), 8.16 (1H, d, J=8.2Hz), 8.21 (1H, d, J=8.2Hz), 8.80 (1H, bs), 10.44 (1H, s).

| Analysis for $C_{22}H_{29}N_3O_4$ | | | |
|---|---|---|---|
| Calcd: | C, 66.14; | H, 7.32; | N, 10.52 |
| Found: | C, 66.05; | H, 7.34; | N, 10.57 |

Preparation 5

Preparation of [4-(N-hydroxyamino)-2(R)-isobutylsuccinyl]-L-homophenylalanine-N-methylamide (compound 19)

(1) [4-(N-Benzyloxyamino)-2(R)-isobutylsuccinyl]-L-homophenylalanine-N-methylamide:

In the same manner as described in Preparation 4, [4-benzyloxyamino-2(R)-isobutylsuccinyl]-L-homophenylalanine-N-methylamide (283mg) was prepared by using N-Boc-L-homophenylalanine (Synthetech Co., Ltd., Oregon, USA) (1.0g).
$^1$H-NMR(DMSO-$d_6$) $\delta$ : 0.83 (3H, d, J=6.5Hz), 0.88 (3H, d, J=6.5Hz), 1.01-1.15 (1H, m), 1.40-1.55 (2H, m), 1.75-2.0 (2H, m), 2.04 (1H, dd, J=6.4Hz, 14.6Hz), 2.24 (1H, dd, J=8.2Hz, 14.6Hz), 2.57 (3H, d, J=4.5Hz), 2.43-2.68 (2H, m), 2.70-2.83 (1H, m), 4.12-4.22 (1H, m), 4.75 (2H, s), 7.12-7.28 (5H, m), 7.35 (5H, s), 7.70-7.77 (1H, m), 8.08 (1H, d, J=8.1Hz), 11.06 (1H, s).

(2) [4-(N-Hydroxyamino)-2(R)-isobutylsuccinyl]-L-homophenylalanine-N-methylamide:

The compound of the above (1) (220mg) was subjected to hydrogenolysis and purified by HPLC to give [4-(N-hydroxyamino)-2(R)-isobutylsuccinyl]-L-(homophenylalanine-N-methylamide (87mg).
$^1$H-NMR(DMSO-$d_6$) $\delta$ : 0.82 (3H, d, J=6.2Hz), 0.87 (3H, d, J=6.2Hz), 1.02-1.16 (1H, m), 1.40-1.57 (2H, m), 1.75-2.0 (2H, m), 2.04 (1H, dd, J=6.9Hz, 14.5Hz), 2.24 (1H, dd, J=7.7Hz, 14.5Hz), 2.57 (3H, d, J=4.5Hz), 2.42-2.68 (2H, m), 2.68-2.80 (1H, m), 4.07-4.17 (1H, m), 7.13-7.30 (5H, m), 7.73-7.81 (1H, m), 8.06 (1H, d, J=8.0Hz), 8.76 (1H, s), 10.44 (1H, s).

Preparation 6

Preparation of [4-(N-hydroxyamino)-2(R)-isobutylsuccinyl]-L-p-fluorophenylalanine-N-methylamide (compound 20)

(1) [4-(N-Benzyloxyamino)-2(R)-isobutylsuccinyl]-L-p-fluorophenylalanine-N-methylamide:

In the same manner as described in Preparation 4, [4-(N-benzyloxyamino)-2(R)-isobutylsuccinyl]-L-p-fluorophenylalanine-N- methylamide (434mg) was prepared by using N-Boc-L-p-fluorophenylalanine (Synthetech Co., Ltd., Oregon, USA) (800mg).
$^1$H-NMR(DMSO-$d_6$) $\delta$ : 0.72 (3H, d, J=6.3Hz), 0.78 (3H, d, J=6.3Hz), 0.88-1.00 (1H, m), 1.15-1.35 (2H, m), 1.90 (1H, dd, J=7.5Hz, 14.4Hz), 2.04 (1H, dd, J=7.2Hz, 14.4Hz), 2.54-2.68 (1H, m), 2.57 (3H, d, J=4.5Hz), 2.80 (1H, dd, J=9.8Hz, 13.7Hz), 2.99 (1H, dd, J=5.0Hz, 13.7Hz), 4.34-4.43 (1H, m), 4.75 (2H, s), 7.00-7.10 (2H, m), 7.19-7.27 (2H, m), 7.37 (5H, s), 7.84-7.91 (1H, m), 8.07 (1H, d, J=8.3Hz), 11.01 (1H, s).

(2) [4-(N-Hydroxyamino)-2(R)-isobutylsuccinyl]-L-p-fluorophenylalanine-N-methylamide:

The above-mentioned compound (1) (350mg) was subjected to hydrogenolysis, and the product was recrystallized from a mixed solvent of ethanol and diethyl ether to give [4-(N-hydroxyamino)-2(R)-isobutylsuccinyl]-L-p-fluorophenylalanine-N-methylamide (64mg).
$^1$H-NMR(DMSO-$d_6$) $\delta$ : 0.71 (3H, d, J=6.4Hz), 0.77 (3H, d, J=6.4Hz), 0.90-1.00 (1H, m), 1.12-1.35 (2H, m), 1.91 (1H, dd, J=7.5Hz, 14.3Hz), 2.04 (1H, dd, J=7.2Hz, 14.3Hz), 2.5-2.65 (1H, m), 2.57 (3H, d, J=4.5Hz), 2.80 (1H, dd, J=10Hz, 13.7Hz), 3.02 (1H, dd, J=4.9Hz, 13.7Hz), 4.32-4.42 (1H, m), 7.02-7.10 (2H, m), 7.19-7.27 (2H, m), 7.89-7.96 (1H, m), 8.04 (1H, d, J=8.4Hz), 8.78 (1H, d, J=1.5Hz), 10.42 (1H, d, J=1.5Hz).

Preparation 7

Preparation of hydrochloride of [4-(N-hydroxyamino)-2(R)-isobutyl-3-(1,2,3,4-tetrahydroisoquinolin-2-yl)methylsuccinyl]-L-3-(1-naphthyl)alanine-N-methylamide (Compound 15).

(1) [4-Benzyloxy-3-methylene-2(R)-isobutylsuccinyl]-L-3-(1-naphthyl)alanine-N-methylamide:

[4-Benzyloxy-3-benzyloxycrbonyl-2(R)-isobutylsuccinyl]-L-3-(1-naphthyl)alanine-N-methylamide was produced by condensation reaction of 4-benzyloxy-3-benzyloxycarbonyl-2(R)-isobutylsuccinic acid (cf. JP-A-101925/1995) and L-3-(1-naphthyl)alanine-N-methylamide (described in Preparation 4) using WSC and HOBt, and the compound (4.6g) and ammonium formate (2.4g) were suspended into ethanol (20ml), and 10%Pd/C (0.5g) was thereto, and the mixture is stirred at room temperature for 6.5 hours. After the catalyst was removed by filtration, piperidine (0.7g) was added thereto, and the solution was stirred at room temperature for 15 minutes, and thereto 37% aqueous formaldehyde solution (3.1g) was added, and the mixture was stirred at room temperature overnight. After the mixture was refluxed for 1 hour, the solvent was distilled off under the reduced pressure. To the residue was added ethyl acetate, and extracted with aqueous potassium carbonate solution. The aqueous layer was adjusted to pH 1 with conc. hydrochloric acid and extracted with chloroform. The organic layer was dried over anhydrous magnesium sulfate and concentrated under reduced pressure to give [4-hydroxy-3-methylene-2(R)-isobutylsuccinyl]-L-3-(1-naphthyl)alanine-N-methylamide (1.34g) as an oil. The oily substance was dissolved in DMF (50ml) and thereto were added sodium hydrogen carbonate (0.42g) and benzyl bromide (2.1g), and the mixture was stirred at room temperature overnight. To the reaction mixture was added chloroform, and the mixture was washed with water, dried over anhydrous magnesium sulfate and concentrated under reduced pressure. The residue was purified by silica gel column chromatography (ethyl acetate:hexane=1:3) to give [4-benzyloxy-3-methylene-2(R)-isobutylsuccinyl]-L-3-(1-naphthyl)alanine-N-methylamide (0.94g).
$^1$H-NMR(DMSO-$d_6$) $\delta$ : 0.76 (3H, d, J=6.4Hz), 0.79 (3H, d, J=6.4Hz), 1.2-1.4 (2H, m), 1.5-1.58 (1H, m), 2.53 (3H, d, J=4.5Hz), 3.15-3.25 (1H, m), 3.34-3.45 (1H, m), 3.48-3.54 (1H, m), 4.5-4.6 (1H, m), 5.13 (1H, d, J=12.6Hz), 5.18 (1H, d, J=12.6Hz), 5.43 (1H, s), 6.02 (1H, s), 7.2-7.4 (7H, m), 7.46-7.58 (2H, m), 7.74 (1H, d, J=7.5Hz), 7.77-7.84 (1H, m), 7.89 (1H, d, J=7.8Hz), 8.13 (1H, J=8.3Hz), 8.18 (1H, d, J=8.3Hz).

(2) [4-(N-Benzyloxyamino-2(R)-isobutyl-3-(1,2,3,4-tetrahydroisoquinolin-2-ylmethyl)succinyl]-L-3-(1-naphthyl)alanine-N-methylamide:

[4-Benzyloxy-3-methylene-2(R)-isobutyl)succinyl]-L-3-(1-naphthyl)alanine-N-methylamide (0.5g) and 1, 2, 3, 4-tetrahydroisoquinoline (0.411g) were stirred at 60°C for one hour. The reaction mixture was purified by thin layer chromatography to give [4-(benzyloxy-2(R)-isobutyl-3-(1, 2, 3, 4-tetrahydroisoquinolin-2-ylmethyl)succinyl]-L-3-(1-naphthyl)alanine-N-methylamide (0.445g) as crystals. The crystals were dissolved in methanol (30ml) and thereto was added 10% Pd-C (100mg), and the mixture was stirred under hydrogen atmosphere to remove the benzyl group. After removing the catalyst by filtration, the filtrate was concentrated under reduced pressure. The residue was dissolved in DMF (25ml) and thereto were added O-benzylhydroxylamine hydrochloride (0.122g), hydroxybenzotriazole (90mg) and triethylamine (77mg), and thereto further added WSC (127mg) under ice cooling, and the mixture was stirred at room temperature overnight. To the reaction mixture was added chloroform (80ml), and the mixture was washed with water, saturated aqueous sodium hydrogen carbonate and aqueous sodium chloride, and dried over anhydrous magnesium sulfate, and concentrated under reduced pressure to give [4-(N-benzyloxyamino-2(R)-isobutyl-3-(1,2,3,4-tetrahydroisoquinolin-2-ylmethyl)succinyl]-L-3-(1-naphthyl)alanine-N-methylamide (280mg) as colorless crystals.
$^1$H-NMR(DMSO-$d_6$) $\delta$ : 0.74 (3H, d, J=6.4Hz), 0.83 (3H, d, J=6.4Hz), 1.3-1.5 (3H, m), 2.0-2.1 (1H, m), 2.2-2.3 (1H, m), 2.35-2.45 (2H, m), 2.54 (3H, d, J=4.5Hz), 2.6-2.65 (2H, m), 2.8-3.1 (3H, m), 3.2-3.5 (3H, m), 4.6-4.7 (3H, m), 6.9-7.1 (3H, m), 7.15-7.3 (5H, m), 7.4-7.6 (5H, m), 7.7-7.9 (3H, m), 7.95 (1H, s), 8.23 (1H, d, J=8.3Hz), 8.37 (1H, d, J=9Hz), 10.93 (1H.s).

(3) [4-(N-Hydroxyamino)-2(R)-isobutyl-3-(1,2,3,4-tetrahydroisoquinolin-2-yl)methylsuccinyl]-L-3-(1-naphthyl)alanine-N-methylamide hydrochloride:

[4-(N-Benzyloxyamino-2(R)-isobutyl-3-(1,2,3,4-tetrahydroisoquinolin-2-ylmethyl)succinyl]-L-3-(1-naphthyl)alanine-N-methylamide (250mg) was dissolved in methanol (15ml) and thereto was added 10% Pd/C. The mixture was stirred under hydrogen atmosphere. After removing the catalyst by filtration, the mixture was concentrated under reduced pressure and the residue was purified by silica gel column chromatography (solvent, chloroform:methanol=20:1) to give an oily substance. The oily substance was dissolved in a mixed solvent of methanol-ethyl acetate (1:2) and thereto was added 4N solution of hydrochloric acid in dioxane (0.1ml) and thereto was further added ether to crystalize the product. The resulting crystals were separated by filtration to give [4-(N-hydroxyamino)-2(R)-isobutyl-3-(1,2,3,4-tetrahydroisoquinolin-2-yl)methylsuccinyl]-L-3-(1-naphthyl)alanine-N-methylamide hydrochloride (52mg).
$^1$H-NMR(DMSO-$d_6$) $\delta$ : 0.76 (3H, d, J=6.4Hz), 0.79 (3H, d, J=6.4Hz), 0.9-1.05 (1H, m), 1.2-1.8 (4H, m), 2.50 (3H, d, J=4.7Hz), 2.5-3.3 (8H, m), 3.8-4.1 (1H, broad), 4.1-4.3 (1H, m), 4.65 (1H, dd, J=8.0Hz, 8.1Hz), 6.8-8.0 (10H, m), 8.28 (1H, d, J=8.3Hz), 8.62 (1H, broad), 9.09 (1H, broad), 10.43 (1H, broad), 10.89 (1H, broad).

Preparation 8

Preparation of hydrochloride of [4-(N-hydroxyamino)-2(R)-isobutyl-3-morpholinomethylsuccinyl]-(1-naphthyl)alanine-N-methylamide (compound 16)

(1) [4-(N-Benzyloxyamino)-2(R)-isobutyl-3-morpholinomethylsuccinyl]-L-3-(1-naphthyl)alanine-N-methylamide:

[4-Benzyloxy-3-methylene-2(R)-isobutylsuccinyl]-L-3-(1-naphthyl)alanine-N-methylamide (0.5g) obtained in Preparation 7 (1) and morpholine (2ml) were dissolved in methylene chloride (4ml). The mixture was reacted in the same manner as is Preparation 7 (2) to give [4-(N-benzyloxyamino)-2(R)-isobutyl-3-morpholinomethylsuccinyl]-L-3-(1-naphthyl)alanine-N-methylamide (258mg).
$^1$H-NMR(DMSO-$d_6$) $\delta$ : 0.72 (3H, d, J=6.4Hz), 0.81 (3H, d, J=6.4Hz), 0.8-0.9 (1H, m), 1.2-1.35 (3H, m), 1.78 (2H, broad), 2.1-2.35 (6H, m), 2.57 (3H, d, J= 4.5Hz), 3.3-3.45 (8H, m), 4.6-4.8 (3H, m), 7.3-7.6 (9H, m), 7.78 (2H, d, J=7.5Hz), 7.92 (1H, dd, J=9.2Hz, 1.4Hz), 8.22 (1H, d, J=8.1Hz), 8.35 (1H, d, J=8.2Hz), 10.93 (1H, broad).

(2) [4-(N-Hydroxyamino)-2(R)-isobutyl-3-morpholinomethylsuccinyl]-L-3-(1-naphthyl)alanine-N-methylamide hydrochloride:

[4-(N-Benzyloxyamino)-2(R)-isobutyl-3-morpholinomethylsuccinyl]-L-3-(1-naphthyl)alanine-N-methylamide (260mg) was reacted in the same manner as is Preparation 7 (3) to give [4-(N-hydroxyamino)-2(R)-isobutyl-3-morpholinomethylsuccinyl]-L-3-(1-naphthyl)alanine-N-methylamide hydrochloride (143mg) as colorless crystals.
$^1$H-NMR(DMSO-$d_6$) $\delta$ : 0.74 (3H, d, J=6.4Hz), 0.78 (3H, d, 7=6.4Hz), 0.9-1.0 (1H, m), 1.2-1.5 (2H, m), 2.5-2.8 (4H, m), 2.55 (3H, d, J=4.4Hz), 3.0-3.4 (4H, m), 3.6-3.9 (4H, m), 4.6-4.7 (1H, m), 7.4-7.6 (4H, m), 7.9-8.0 (1H, m), 7.93 (1Hz, d, 8Hz), 7.97 (1H, d, J=4.6Hz), 8.28 (1H, d, 7=8.1Hz), 8.56 (1H, d, J=8.2Hz), 9.03 (1H, broad), 10.40 (1H, broad), 10.85 (1H, s).

Preparation 9

Preparation of [4-(N-hydroxyamino)-2(R)-isobutyl-3-methylsuccinyl]-L-3-(1-naphthyl)alanine-N-methylamide (Compound 17).

(1) [4-(N-Benzyloxyamino)-2(R)-isobutyl-3-methylsuccinyl]-L-3(1-naphthyl)alanine-N-methylamide:

[4-Benzyloxy-3-methylene-2(R)-isobutylsuccinyl]-L-3-(1-naphthyl)alanine-N-methylamide (0.5g) obtained in Preparation 7 (1) was dissolved in Methanol (30ml), and 10% Pd/C (100mg) was added to the solution, and the mixture was stirred under hydrogen atmosphere. The catalyst was removed by filtration, and the mixture was dissolved in DMF (30ml), and O-benzyloxyamine hydrochloride (246mg), triethylamine (156mg), hydroxybenzotriazol (180mg) and WSC (256mg) were added thereto, and the mixture was stirred at room temperature overnight. The reaction mixture was concentrated in vacuo, and the residue was dissolved in chloroform, and washed with water and a saturated aqueous sodium bicarbonate, and was dried over anhydrous magnesium sulfate. It was concentrated in vacuo to give [4-(N-benzyloxyamino)-2(R)-isobutyl-3-methylsuccinyl]-L-3-(1-naphthyl)alanine-N-methylamide (329mg) as colorless crystals.

(2) [4-(N-Hydroxyamino)-2(R)-isobutyl-3-methylsuccinyl]-L-3-(1-naphthyl) alanine-N-methylamide:

[4-(N-Benzyloxyamino)-2(R)-isobutyl-3-methylsuccinyl]-L-3-(1-naphthyl)alanine-N-methylamide (328mg) was suspended in methanol (30ml), and 10% Pd/C (60mg) was added thereto, and the mixture was stirred under hydrogen atmosphere (3kg/$m^2$). The catalyst was removed by filtration, and the mixture was concentrated under reduced pressure. The residue was purified by high performance liquid chromatography (HPLC, column: Inertsil PREP-ODS (20×250mm), mobile phase: 0.1% TFA water:acetonitrile=6:4) to give [4-(N-hydroxyamino)-2(R)-isobutyl-3-methylsuccinyl]-L-3-(1-naphthyl)alanine-N-methylamide (74mg) as colorless crystals.
$^1$H-NMR(DMSO-$d_6$) $\delta$ : 0.39 (3H, d, J=6.7Hz), 0.73 (3H, d, J=6.4), 0.81 (3H, d, J=6.4Hz), 0.8-0.9 (1H, m), 1.2-1.45 (2H, m), 1.85-2.0 (1H, m), 2.3-2.5 (1H, m), 2.57 (3H, d, J=4.5Hz), 3.2-3.6 (2H, m), 4.6-4.75 (1H, m), 7.3-7.65 (4H, m), 7.7-7.8 (2H, m), 7.9 (1H, d, J=7.9Hz), 8.22 (1H, d, J=8.8Hz), 8.34 (1H, d, 8.4Hz), 8.7 (1H, broad), 10.35 (1H, s).

Preparation 10

Preparation of [4-(N-hydroxyamino)-2(R)-isobutyl-3-methylsuccinyl] -L-3-(5,6,7,8-tetrahydro-1-naphthyl)alanine-N-methylamide (Compound 18).

[4-(N-Benzyloxyamino)-2(R)-isobutyl-3-methylsuccinyl]-L-3-(1-naphthyl)alanine-N-methylamide (350mg) obtained in Preparation 9 (1) was suspended in acetic acid (20ml), and 10% Pd/C (100mg) was added to the suspension. The mixture was stirred under hydrogen atmosphere (3kg/$m^2$). The catalyst was removed by filtration, and the mixture was concentrated under reduced pressure. The residue was purified by high performance liquid chromatography (HPLC, column: Inertsil PREP-ODS (20×250mm), mobile phase: 0.1% TFA water:acetonitrile=6:4) to give [4-(N-hydroxyamino)-2(R)-isobutyl-3-methylsuccinyl]-L-3-(5,6,7,8-tetrahydro-1-naphthyl)alanine-N-methylamide (103mg) as colorless crystals.
$^1$H-NMR(DMSO-$d_6$) $\delta$ : 0.59 (3H, d, J=6.7Hz), 0.74 (3H, d, J=6.4Hz), 0.80 (3H, d, J=6.4Hz), 0.8-0.9 (1H, m), 1.2-1.45 (2H, m), 1.6-1.85 (4H, m), 1.95-2.1 (1H, m), 2.3-2.5 (1H, m), 2.54 (3H, d, J=4.6Hz), 2.6-2.95 (6H, m), 4.45-4.6 (1H, m), 6.8-7.05 (3H, m), 7.67 (1H, q, J=4.6Hz), 8.25 (1H, d, J=8.6Hz), 8.72 (1H, s), 10.39 (1H, s).

Preparation 11

Preparation of [4-(N-hydroxyamino)-2(R)-isobutyl-3-phenylthiomethylsuccinyl]-L-phenylglycine-N-methylamide (Compound 22)

To [4-hydroxy-2(R)-isobutyl-3-methylenesuccinyl]-L-phenylglycine-N-methylamide (700mg) which was prepared by the method described in JA-P-101925/1995) was added thiophenol (5ml) and the mixture was stirred at 60°C under argon atmosphere for 3 days. To the reaction mixture was added ether/hexane (60ml/20ml), and the precipitated solid material was filtered and washed with ether-ethyl acetate to give [4-hydroxy-2(R)-isobutyl-3-phenylthiomethylsuccinyl-L-phenylglycine-N-methylamide (380mg). Then, this solid material was dissolved in DMF (5ml), and N-hydroxybenzotriazole (145mg) and WSC (180mg) were added thereto and the mixture was stirred under ice cooling for two hours. To this reaction mixture were added hydroxylamine hydrochloride (82mg) and a solution of N-methylmorpholine (120mg) in DMF (3ml), and the solution was stirred at room temparature overnight. To the reaction mixture was added ethyl acetate (40ml), and the solution was washed with 1N hydrochloric acid, dried over magnesium sulfate, and concentrated in

vacuo. The residue was recrystallized from isopropyl alcohol to give [4-(N-hydroxyamino)-2(R)-isobutyl-3-phenylthiomethylsuccinyl]-L-phenylglycine-N-methylamide (84mg) as colorless crystals.
$^1$H-NMR (DMSO-$d_6$) δ : 0.80 (3H, d, J=6.4Hz), 0.85 (3H, d, J=6.4Hz), 0.88-1.00 (1H, m), 1.32-1.53 (2H, m), 2.26-2.37 (1H, m), 2.53-2.61 (1H, m), 2.57 (3H, d, J=4.5Hz), 2.72-2.83 (1H, m), 2.93-3.93 (1H, m), 5.53 (1H, d, J=8.2Hz), 6.92-6.97 (2H, m), 7.09-7.32 (6H, m), 7.43-7.49 (2H, m), 8.12-8.18 (1H, m), 8.91 (1H, s), 8.95 (1H, d, J=8.2Hz), 10.58 (1H, s).

Preparation 12

Preparation of [4-(N-hydroxyamino)-2(R)-isobuthylsuccinyl]-L-3-(3-benzothienyl)alanine-N-methylamide (Compound 21).

(1) [4-(N-Benzyloxyamino)-2(R)-isobutylsuccinyl]-L-3-(3-benzothienyl)alanine-N-methylamide:

In the same manner as described in Preparation 4, [4-benzyloxyamino-2(R)-isobutylsuccinyl]-L-3-(3-benzothienyl)alanine-N-methylamide (350mg) was prepared by using N-Boc-L-3-(3-benzothienyl)alanine (Synthetech Co., Ltd., Oregon, USA) (1.0g).
$^1$H-NMR(DMSO-$d_6$) δ : 0.72 (3H, d, J=6.2Hz), 0.79 (3H, d, J=6.2Hz), 0.9-1.0 (1H, m), 1.30-1.40 (2H, m), 1.93 (1H, dd, J=7.2Hz, 14.4Hz), 2.08 (1H, dd, J=7.3Hz, 14.4Hz), 2.57 (3H, d, J=4.5Hz), 2.6-2.7 (1H, m), 3.11 (1H, dd, J=9.0Hz, 14.7Hz), 3.23 (1H, dd, J=5.1Hz, 14.7Hz), 4.5-4.6 (1H, m), 4.75 (2H, s), 7.3-7.5 (8H, m), 7.6-8.0 (3H, m), 8.18 (1H, d, J=8.1Hz), 11.02 (1H, s).

(2) [4-(N-Hydroxyamino)-2(R)-isobuthylsuccinyl]-L-3-(3-benzothienyl)alanine-N-methylamide:

The above-mentioned compound (1) (300mg) was subjected to hydrogenolysis, and the product was recrystallized from a mixed solvent of ethyl acetate and methanol to give [4-(N-hydroxyamino)-2(R)-isobutylsuccinyl]-L-3-(3-benzothienyl)alanine-N-methylamide (85mg).
$^1$H-NMR(DMSO-$d_6$) δ : 0.71 (3H, d, J=6.2Hz), 0.77 (3H, d, J=6.2Hz), 0.9-1.0 (1H, m), 1.20-1.40 (2H, m), 1.92 (1H, dd, J=7.2Hz, 14.4Hz), 2.05 (1H, dd, J=7.3Hz, 14.4Hz), 2.54 (3H, d, J=5.2Hz), 2.55-2.7 (1H, m), 3.11 (1H, dd, J=9.3Hz, 14.7Hz), 3.26 (1H, dd, J=4.8Hz, 14.7Hz), 4.54-4.6 (1H, m), 7.3-7.5 (3H, m), 7.6-8.0 (3H, m), 8.16 (1H, d, J=8.1Hz), 8.77 (1H, d, J=1.6Hz), 10.44 (1H, d, J=1.5Hz).

| Analysis for $C_{20}H_{27}N_3O_4S$ | | | |
|---|---|---|---|
| Calcd: | C, 59.24; | H, 6.71; | N, 10.36 |
| Found: | C, 59.18; | H, 6.81; | N, 10.34 |

Preparation 13

Preparation of 4(S)-[4-N-hydroxyamino)-2(R)-isobutyl-3-morpholinosuccinyl]amino-1,2,4,5-tetrahydro-3H-2-benzazepin-3-one (Compound 80)

(1) 4(S)-[4-Benzyloxy-3-benzyloxycarbonyl-2(R)-isobutylsuccinyl]amino-1,2,4,5-tetrahydro-3H-2-benzazepin-3-one:

To a mixture of Compound (VII) in optically active form (11.25g), Compound (VI) hydrochloride (6.0g), HOBt (3.8g), DMF (50ml) and triethylamine (3.9ml) was added to WSC (6.5g) under ice cooling, and the solution was stirred overnight. The reaction mixture was concentrated in vacuo, and the residue was dissolved in ethyl acetate (500ml), washed with diluted hydrochloric acid, water, a saturated aqueous sodium bicarbonate and a saturated aqueous sodium chloride solution, and dried over anhydrous magnesium sulfate. It was concentrated in vacuo to give the title compound (16.0g)
$^1$H-NMR(DMSO-$d_6$) δ : 0.76 (3H, d), 0.83 (3H, d), 0.97-1.10 (1H, m), 1.42-1.53 (1H, m), 1.56-1.70 (1H, m), 2.86 (1H, dd), 2.97 (1H, dd), 3.13 (1H, dt), 3.72 (1H, d), 3.89 (1H, dd), 4.74 (1H, dd), 5.00-5.10 (1H, m), 5.10-5.23 (4H, m), 7.00-7.40 (14H, m), 8.18-8.26 (2H, m).

(2) Preparation of 4(S)-[4-hydroxy-2(R)-isobutyl-3-methylenesuccinyl]amino-1,2,4,5-tetrahydro-3H-2-benzazepin-3-one [Compound (VIII) in optically active form]:

To a mixture of the compound in the above (1) (16g), ammonium formate (9.5g) and ethanol (300ml) was added a mixture of 10% Pd-C (3g) and 1,4-zioxane (10ml), and the mixture was stirred for three hours at room temperature. The catalyst was removed by filtering the reaction mixture, and piperidine (2.5g) and 37% formaldehyde solution (16ml) were added thereto, and the mixture was stirred at room temparature overnight. After the reaction mixture was refluxed for another 1.5 hour, the mixture was concentrated to dryness in vacuo. 10% Citric acid solution was added to the residue, and it was extracted by ethyl acetate. After the organic layer was extracted by 10% aqueous potassium carbonate solution and the aqueous layer was adjusted to pH 4 with diluted hydrochloric acid, and extracted with methlene chloride. The methylene chloride solution was dried over magnesium sulfate and concentrated in vacuo. The product was crystallized by adding ether to the residue to give the title compound (5.18g).
m.p.: 139°C-145°C
$^1$H-NMR(DMSO-$d_6$) $\delta$ : 0.85 (3H, d), 0.89 (3H, d), 1.33-1.43 (1H, m), 1.45-1.60 (1H, m), 1.63-1.74 (1H, m), 2.85 (1H, dd), 3.11 (1H, dd), 3.58 (1H, dd), 3.92 (1H, dd), 4.80 (1H, dd), 5.02-5.12 (1H, m), 5.79 (1H, s), 6.20 (1H, s), 7.10-7.23 (4H, m), 7.85 (1H, d), 8.27-8.34 (1H, m), 12.5 (1H, bs).

(3) 4(S)-[4-Benzyloxy-2(R)-isobutyl-3-methylsuccinyl]amino-1,2,4,5-tetrahydro-3H-2-benzazepin-3-one [Compound (IX) in optically active form]:

A mixture of Compound (VIII) in optically active form (3.0g), sodium hydrogen carbonate (1.1g), benzyl bromide (3.0g) and DMF (20ml) was stirred at room temperature overnight, and the reaction mixture was concentrated in vacuo. The residue was dissolved in ethyl acetate (200ml), washed with diluted hydrochloric acid, dried over anhydrous magnesium sulfate and concentrated in vacuo. The residue was purified with the medium pressure silica gel column chromatography (eluent:cyclohexane/ethyl acetate=1/1) to give the title compound (2.0g).
$^1$H-NMR($CDCl_3$) $\delta$ : 0.89 (3H, d), 0.92 (3H, d), 1.5 (2H, m), 1.63 (1H, m), 2.79 (1H, dd), 3.32 (1H, dd), 3.60 (1H, t), 3.97 (1H, dd), 4.88 (1H, dd), 5.22 (1H, m), 5.23 (2H, s), 5.91 (1H, s), 6.46 (1H, s), 6.50 (1H, t), 7.06 (1H, m), 7.20 (9H, m).

(4) 4(S)-[4-Benzyloxy-2(R)-isobutyl-3-morpholinomethylsuccinyl]amino-1,2,4,5-tetrahydro-3H-2-benzazepin-3-one [Compound (Xa)]:

A mixture of Compound (IX) (0.67g) and morpholine (2ml) was stirred at 50-60°C for 17 hours, and concentrated in vacuo. The residue was purified with the medium pressure silica gel column chromatography (chloroform/ethanol=100/1) to give the title compound (0.53g) as a mixture of two kinds of isomer. These stereoisomers were eluted at a retention time of 15 minutes and 19 minutes respectively in the following HPLC analysis, wherein the peak area ratio was about 1:2.

⟨HPLC conditionss⟩

Column: YMC-Pack ODS-A A-312 (4.6×150mm)
Mobile phase: 0.1% TFA solution: acetonitrile=7:3
Flow rate: 1 ml/min
The mixture of the stereoisomers showed the following properties.
m.p.: 75°C-78°C
$^1$H-NMR(DMSO-$d_6$) $\delta$ : 0.8-0.9 (6H, m), 1.0 (1H, m), 1.2-1.5 (1H, m), 1.7 (1H, m), 2.2 (1H, m), 2.3 (1H, m), 2.3-2.4 (1H, m), 2.5 (2H, m), 2.6 (1H, m), 2.7 (1H, m), 2.9 (1H, m), 3.0 (1H, m), 3.4 (1H, m), 3.5-3.6 (4H, m), 3.9 (1H, dd), 4.9 (1H, dd), 5.2 (2H, m), 5.3 (1H, m), 6.3-6.6 (1H, m), 7.2 (1H, m), 7.5 (10H, m).

(5) 4(S)-[4-(N-Benzyloxyamino)-2(R)-isobutyl-3-morpholinomethylsuccinyl]amino-1,2,4,5-tetrahydro-3H-2-benzazepin-3-one [Compound (XIa) in optically active form]:

A mixture of the mixed stereoisomers obtained in the above (4) (0.5g), 10% Pd-C (0.1g) and methanol (20ml) was stirred under hydrogen atmosphere for two hours and the catalyst was filtered off, and the filtrate was concentrated in vacuo. To the residue were added DMF (10ml), HOBt (0.2g), triethylamine (0.12g) and O-benzylhydroxylamine hydrochloride (0.19g), and the solution was ice-cooled. After WSC (0.28g) was added, the mixture was stirred at room temperature overnight. The reaction mixture was concentrated in vacuo and the residue was dissolved in ethyl acetate (150ml). The solution was washed with a saturated aqueous sodium bicarbonate, dried over anhydrous magnesium sulfate and distilled under reduced pressure to remove the solvent to give Compound (XIa) as a mixture of two stereoisomers. These stereoisomers were eluted at the retention time of 24 minutes and 28 minutes respectively in the following HPLC analysis, wherein the peak area ratio was about 1:2 (designated as diastereomer e and diastereomer f, respectively).

〈HPLC conditions〉

Column: Inertsil PREP ODS (6.0×250mm)
Mobile phase: 0.1% TFA aqueous solution:acetonitrile=7:3
Flow rate: 1 ml/min

The mixture of the stereoisomers was crystallized from a mixed solvent of a small amount of methanol and ether, the firstly precipitated crystals were filtered to give diastereomer f (0.22g).
m.p.: 209°C
$^1$H-NMR(DMSO-$d_6$) δ : 0.77 (3H, d), 0.84 (3H, d), 0.9 (1H, m), 1.45 (2H, m), 2.1 (3H, m), 2.4 (2H, m), 2.6 (1H, q), 3.0 (2H, m), 3.4 (4H, m), 3.9 (1H, dd), 4.7 (1H, dd), 4.81 (2H, q), 5.1 (1H, m), 7.2 (4H, m), 7.4 (5H, m), 8.2 (2H, m), 11.05 (1H, s).

(6) 4(S)-[4-(N-Hydroxyamino)-2(R)-isobutyl-3-morpholinomethylsuccinyl] amino-1,2,4,5-tetrahydro-3H-2-benzazepin-3-one [Compound 80]

A mixture of the diastereomer f obtained in the above (5) (0.2g), 10% Pd-C (0.1g) and methanol (20ml) was stirred under hydrogen atmosphere for two hours, and the catalyst was removed and the solution was concentrated in vacuo. The residue was crystallized from ether to give the title compound (0.14g).
m.p.: 190-191°C
$^1$H-NMR(DMSO-$d_6$) δ : 0.78 (3H, d), 0.84 (3H, d), 0.94 (1H, t), 1.44 (2H, m), 2.0 (1H, m), 2.1 (2H, m), 2.4 (3H, m), 2.5 (1H, m), 2.6 (1H, m), 3.0 (2H, m), 3.5 (4H, m), 3.9 (1H, dd), 4.7 (1H, dd), 5.1 (1H, m), 7.2 (4H, m), 8.2 (2H, m), 8.79 (1H, s), 10.41 (1H, s).

Preparation 14

Preparation of 4(S)-[4-N-hydroxyamino)-2(R)-isobutyl-3-(1,2,3,4-tetrahydroisoquinolin-2-yl)methylsuccinyl]amino-1,2,4,5-tetrahydro-3H-2-benzazepin-3-one (Compound 81)

(1) 4(S)-[4-Benzyloxyamino-2(R)-isobutyl-3-(1,2,3,4-tetrahydroisoquinolin-2-yl)methylsuccinyl]amino-1,2,4,5-tetrahydro-3H-2-benzazepin-3-one [Compound (Xb)]:

In the same manner as described in Preparation 13 (4) except using 1,2,3,4-tetrahydroisoquinoline instead of morpholine, the title compound (0.56g) was obtained from 4(S)-[3-benzyloxycarbonyl-2(R)-isobutyl-3-methylenesuccinyl]amino-1,2,4,5-tetrahydro-3H-2-benzazepin-3-one (0.62g). This product was a mixture of two stereoisomers which were eluted with the retention time of 8.8 minutes and 10.1 minutes respectively (the peak area ratio was about 4:1) in the following HPLC analysis.

〈HPLC conditions〉

Column: YMC-Pack ODS-A A-312 (4.6×150mm)
Mobile phase: 0.1% TFA aqueous solution:acetonitrile=3:2
Flow rate: 1 ml/min
The mixture of the stereoisomers showed the following properties.
m.p.: 65-70°C
$^1$H-NMR($CDCl_3$) δ : 0.8-0.9 (6H, m), 0.9-1.0 (1H, m), 1.5 (1H, m), 1.7 (1H.m), 2.5-3.0 (9H, m), 3.1 (1H, m), 3.4 (1H, m), 3.5 (1H, m), 3.7 (1H, m), 3.9 (1H, dd), 4.8 (1H, dd), 5.1 (2H, m), 5.2 (1H, m), 6.6-7.5 (15H, m).

(2) 4(S)-[4-(N-Benzyloxyamino)-2(R)-isobutyl-3-(1,2,3,4-tetrahydroisoquinolin-2-yl)methylsuccinyl]amino-1,2,4,5-tetrahydro-3H-2-benzazepin-3-one [Compound (XIb) in optically active form]:

The mixture of stereoisomers obtained in the above (1) (0.50g) was treated in the manner as described Preparation 13 (5) to give the compound (XIa) (0.44g) as a mixture of two stereoisomers. These stereoisomers were eluted with the retention time of 1.06 minute and 12.6 minutes respectively in the following HPLC analysis, wherein the peak area ratio was about 4:1 (which were designated as diostereomer g and diostereomer h respectively).

〈HPLC conditions〉

Column: Inertsil PREP-ODS (6.0×250mm)
Mobile phase: 0.1% TFA aqueous solution:acetonitrile=3:2
Flow rate: 1 ml/min

These stereoisomers were crystallized from a mixed solvent of a small amount of methanol and ether, and the firstly precipitated crystals were filtered to give diastereomer g (0.3g).
m.p.: 165-166°C
$^1$H-NMR(DMSO-$d_6$) $\delta$ : 0.77 (3H, d), 0.85 (3H, d), 0.93 (1H, m), 1.45 (2H, m), 2.3 (1H, m), 2.5-2.6 (2H, m), 2.7 (4H, m), 2.8 (1H, m), 3.0 (2H, m), 3.4 (1H, d), 3.6 (1H, d), 3.9 (1H, dd), 4.72 (1H, m), 4.73 (2H, s), 5.06 (1H, m), 7.1 (13H, m), 8.1 (2H, m), 11.06 (1H, s).

(3) 4(S)-[4-(N-Hydroxyamino)-2(R)-isobutyl-3-(1,2,3,4-tetrahydroisoquinolin-2-yl)methylsuccinyl]amino-1,2,4,5-tetrahydro-3H-2-benzazepin-3-one (Compound 81)

Compound 81 in optically active form (0.12g) was obtained from the optically active compound (diastereomer g) obtained in the above (2) (0.3g) in the same manner as described in Preparation 13 (6).
m.p.: 168°C
$^1$H-NMR(DMSO-$d_6$) $\delta$ : 0.79 (3H, d), 0.85 (3H, d), 0.97 (1H, t), 1.5 (2H, m), 2.29 (1H, d), 2.5 (2H, m), 2.7 (4H, m), 2.8 (1H, m), 3.0 (2H, m), 3.37 (1H, d), 3.62 (1H, d), 3.9 (1H, dd), 4.7 (1H, dd), 5.07 (1H, m), 7.0 (8H, m), 8.1 (2H, m), 8.7 (1H, d), 10.4 (1H, s).

Preparation 15

Preparation of 4(S)-[4-N-hydroxyamino)-2(R)-isobutyl-3-methylsuccinyl]amino-1,2,4,5-tetrahydro-3H-2-benzazepin-3-one (Compound 82)

(1) 4(S)-[4-Hydroxy-2(R)-isobutyl-3-methylsuccinyl]amino-1,2,4,5-tetrahydro-3H-2-benzazepin-3-one [Compound (Xc) in optically active form]:

The optically active Compound (VIII) (0.4g) (see Preparation 13 (2)) was dissolved in methanol (10ml), and thereto was added 10% PD-C (100mg), and the mixture was stirred under hydrogen atmosphere at room temperature for 3 hours to carry out the catalytic reduction. After the catalyst was filtered off, the solvent was removed to give 4(S)-[4-hydroxy-2(R)-isobutyl-3-methylsuccinyl] amino-1,2,4,5-tetrahydro-3H-2-benzazepin-3-one (a mixture of stereoisomers). These stereoisomers were eluted with the retention time of 6.3 minutes and 7.3 minutes respectively in the following HPLC analysis (designated as diostereomer i and diostereomer j respectively), wherein the peak area ratio was about 5:1.

〈HPLC conditions〉

Column: YMC-Pack ODS-A A-312 (6.0×150mm)
Mobile phase: 0.1% TFA aqueous solution:acetonitrile=3:2
Flow rate: 1 ml/min
The above product was purified by preparative HPLC to give an optically active Compound (Vc) (stereoisomer i) (230mg).
$^1$H-NMR(DMSO-$d_6$) $\delta$ : 0.81 (3H, d), 0.88 (3H, d), 0.93-1.07 (1H, m), 1.07 (3H, d), 1.43-1.60 (2H, m), 2.30-2.43 (1H, m), 2.53-2.63 (1H, m), 2.96 (1H, dd), 3.11 (1H, dd), 3.95 (1H, dd), 4.75 (1H, dd), 5.06-5.16 (1H, m), 7.10-7.25 (4H, m), 8.18-8.28 (2H, m), 12.2 (1H, bs).

(2) 4(S)-[4-(N-Benzyloxyamino)-2(R)-isobutyl-3-methylsuccinyl]amino-1,2,4,5-tetrahydro-3H-2-benzazepin-3-one [Compound (XIc) in optically active form]:

The optically active product (diastereomer i) (200mg) obtained in the above (1) was dissolved in DMF (5ml), and thereto were added O-benzylhydroxylamine hydrochloride (125mg), 4-methylmorpholine (80mg), WSC (160mg) and HOBt (110mg) in order, and the mixture was stirred at room temperature overnight. The reaction mixture was poured into diluted hydrochloric acid, and extracted with ethyl acetate. The ethyl acetate layer was washed with saturated aqueous potassium carbonate solution and saturated aqueous sodium chloride solution, and dried over magnesium sulfate.

The solvent was removed in vacuo to give an optically active Compound (XIc) (180mg).
$^1$H-NMR(DMSO-$d_6$) δ : 0.77 (3H, d), 0.85 (3H, d), 0.85-0.95 (1H, m), 0.96 (3H, d), 1.34-1.53 (2H, m), 2.10-2.20 (1H, m), 2.46-2.60 (1H, m), 2.94 (1H, dd), 3.09 (1H, dd), 3.95 (1H, dd), 4.74 (1H, dd), 4.79 (2H, s), 5.05-5.15 (1H, m), 7.10-7.25 (4H, m), 7.28-7.42 (5H, m), 8.15-8.27 (2H, m), 11.08 (1H, s).

(3) 4(S)-[4-(N-Hydroxyamino)-2(R)-isobutyl-3-methylsuccinyl]amino-1,2,4,5-tetrahydro-3H-2-benzazepin-3-one (Compound 82):

The optically active Compound (XIc) (170mg) obtained in the above (2) was dissolved in a mixed solvent of methanol (10ml) and THF (5mg), and the mixture was subjected to catalystic reduction with 10% Pd-C (70mg) under hydrogen atmosphere. After the catalyst was filtered off, the solvent was distilled off, and the residue was recrystallized from ethanol to give optically active Compound (82) (14mg).
m.p.: 215-217°C
Mass spectum (SIMS): 362 (M+1)
IR (KBr) cm-1: 3380, 3288, 1640.
$^1$H-NMR(DMSO-$d_6$) δ : 0.78 (3H, d), 0.85 (3H, d), 0.85-0.95 (1H, m), 0.97 (3H, d), 1.38-1.57 (2H, m), 2.12-2.24 (1H, m), 2.45-2.60 (1H, m), 3.00 (1H, dd), 3.09 (1H, dd), 3.95 (1H, dd), 4.75 (1H, dd), 5.06-5.16 (1H, m), 7.10-7.25 (4H, m), 8.15-8.23 (2H, m), 8.76 (1H, d), 10.47 (1H, d).

Preparation 16

Preparation of 4(S)-[4-N-hydroxyamino)-2(R)-isobutyl-3-phenylthiomethylsuccinyl]amino-1,2,4,5-tetrahydro-3H-2-benzazepin-3-one (Compound 83)

(1) 4(S)-[4-hydroxy-2(R)-isobutyl-3-phenylthiomethylsuccinyl]amino-1,2,4,5-tetrahydro-3H-2-benzazepin-3-one [Compound (Xd) in optically active form]:

Thiophenol (5ml) was added to the optically active Compound (VIII) [Prereparation 13 (2)] (0.40g), and mixture was reacted with shield of light at 60°C overnight. To the reaction mixture were added ether and n-hexane, and the precipitated white solid material was filtered. The desired stereoisomer eluted in the retention time of 9.0 minutes in HPLC analysis under the following conditions.

〈HPLC conditions〉

Column: YMC-Pack ODS-A A-312 (6.0×150mm)
Mobile phase: 0.1% TFA solution:acetonitrile=1:1
Flow rate: 1 ml/min
Retention time: 9.0 minutes
The above stereoisomer was purified by preparative HPLC to give optically active Compound (Xd) (0.12g).
$^1$H-NMR(DMSO-$D_6$) δ : 0.80 (3H, d), 0.86 (3H, d), 0.92-1.03 (1H, m), 1.42-1.63 (2H, m), 2.58-2.77 (2H, m), 2.95-3.19 (4H, m), 3.98 (1H, dd), 4.74 (1H, dd), 5.02-5.12 (1H, m), 7.09-7.33 (9H, m), 8.24 (1H, t), 8.44 (1H, d), 12.6 (1H, bs).

(2) 4(S)-[4-(N-Hydroxyamino)-2(R)-isobutyl-3-phenylthiomethylsuccinyl] amino-1,2,4,5-tetrahydro-3H-2-benzazepin-3-one (Compound (83)

The optically active compound (0.11g) obtained in the above (1) was dissolved in DMF (3ml), and thereto were added HOBt (45mg) and WSC (55mg), and the mixture was stirred for 1.5 hour under ice cooling. Hydroxylamine hydrochloride (25mg) and 4-methylmorpholine (37mg) were added to the mixture, and the resultant was stirred at room temperature overnight. After the reaction, ethyl acetate (50ml) was added thereto. The mixture was washed with 2N hydrochloric acid and a saturated aqueous sodium chloride solution, and dried over magnesium sulfate. The magnesium sulfate was filtered off, and the solvent was removed in vacuo, and the residue was purified by preparative HPLC to give the title Compound (48mg).

〈Preparative HPLC conditions〉

Column: Inertsil PREP-ODS (20.0×250mm)
Mobile phase: 0.1% TFA aqueous solution:acetonitrile=1:1
Flow rate: 10ml/min

The optically active Compound (83) showed the following properties.
m.p.: decomposed around 190°C
Mass spectrum (SIMS): 470 (M+H)+
IR (KBr) $cm^{-1}$: 3380, 1690, 1664, 1646.
$^1$H-NMR(DMSO-$d_6$) δ : 0.80 (3H, d), 0.86 (3H, d), 0.90-1.02 (1H, m), 1.40-1.57 (2H, m), 2.40-2.50 (1H, m), 2.60-2.70 (1H, m), 2.95-3.15 (4H, m), 3.90-4.03 (1H, m), 4.69-4.79 (1H, m), 5.01-5.11 (1H, m), 7.04-7.32 (9H, m), 8.22 (1H, t), 8.41 (1H, d), 10.64 (1H, s).

Preparation 17

Preparation of mouse anti-human FasL antibody

hFasL/L5178Y wherein a human FasL cDNA was introduced into mouse lymphoma L-5178Y was used as an immunogen. A female MRLlpr/lpr mouse (Crea) (4 weeks old) was immunized by administering intraperitoneally the immunogen ($1x10^7$) four times at an interval of 10 days. Three days after the final immunization, the mouse was killed and the spleen was obtained under sterilized condition. The obtained spleen was finely cut with scissors in RPMI 1640 medium to give a suspension of antibody-producing spleen cells in RPMI 1640 medium. This suspension was mixed with mouse myeloma cells P3U1 (ATCC CRL-1597), and thereto were added dropwise a RPMI 1640 medium (0.5ml) containing polyethylene glycol and dimethylsulfoxide (content: 42.5 w/v% and 15 v/v%, respectively) with gently stirring over a period of one minute. Thereafter, to the mixture was further added dropwise a RPMI 1640 medium (30ml) with gently stirring over a priod of 5 minutes to effect the cell fusion.

The mixture was centrifuged at 1000 rpm for 10 minutes and the supernatant was removed to give a mixture of fused cells.

The cell mixture thus obtained was suspended in HAT medium (a RPMI 1640 medium containing 100μ M hypoxanthine, 0.4μ M aminopterin and 16μ M thymidine, as well as 10% fetal bovine serum) and the mixture was poured into 96 well microplate (Falcon3072, manufactured by Bectone Deckinson) in an amount of 0.2 ml/well, which was cultured under 5% $CO_2$ atmosphere at 37°C. After cultivating for 11 days, the supernatant was subjected to the test of cytotoxic activity as mentioned in Experiment 4, by which the hybridoma was screened.

The hybrodoma which produced an antibody which strongly neutralized the cytotoxic activity was elected and subjected to cloning by limited dilution method to give NOK-1 (IgG1, κ) and NOK-3 (IgM, κ). The NOK-1 was purified by subjecting the mouse ascites to protein G affinity chromatography. The NOK-3 was purified by subjecting the mouse ascites to salting-out with ammonium sulfate and gel filtration.

Experiment 4

Test of cytotoxic activity

The culture supernatant (100μl) obtained in the above Preparation 17 was added to hFasL/WR19L ($1\times10^4$ cells) wherein a human FasL cDNA was introduced into mouse lymphoma WR19L, and the mixture was cultured under 5%$CO_2$ atmosphere at 37°C for 16 hours. To the mixture was added Alamar Blue™ (manufactured by Alamar Bioscience) (10μl), and the mixture was further cultured under 5%$CO_2$ atmosphere at 37°C for 4 hours. The fluorescence at 590 nm of the reduced Alamar Blue in the culture supernatant was measured at an excited wavelength of 544 nm with Titertek Fluoroskan (manufactured by Laboratory Systems).

Preparation 18

Preparation of biotin-labelled anti-FasL antibody (NOK-1)

The anti-FasL antibody (NOK-1) obtained in Preparation 17 was dissolved in 0.1M aqueous sodium hydrogen carbonate solution in a concentration of 1 mg/ml. The solution (2ml) thus prepared was dialyzed against 0.1M aqueous sodium hydrogen carbonate solution (1000ml) at 4°C for one hour. After the dialysis, to the NOK-1 solution was added a solution (100μl) of N-hydroxysuccinimide biotin (manufactured by Pearce Co.) (1 mg/ml) in dimethylsulfoxide and the mixture was stirred at room temperatutre for 3 hours. After the stirring, the solution was dialyzed three times against PBS at 4°C for one hour to give the desired biotin-labelled antibody.

Preparation 19

Preparation of [4-(hydroxyamino)-2(R)-isobutyl-3-methylsuccinyl]-L-phenylglycine-N-methylamide (Compound 4)

(1) [4-Hydroxy-2(R)-isobutyl-3(RS)-methylsuccinyl]-L-phenylglycine-N-methylamide:

[4-Hydroxy-2(R)-isobutyl-3-methylenesuccinyl]-L-phenylglycine-N-methylamide (11.1g) was dissoved in ethanol (700ml), and 10% Pd/C (1.0g) was added thereto, and the mixture was stirred for two hours under hydrogen atmosphere. The catalyst was filtered off and the filtrate was concentrated to dryness under reduced pressure to give [4-hydroxy-2(R)-isobutyl-3(RS)-methylsuccinyl]-L-phenylglycine-N-methylamide (a mixture of two kinds of diastereomers with asymmetric carbon at the 3-position) (11g). The solid material was a mixture of two kinds of stereoisomers (diastereomer a' and diastereomer b') which were eluted at a retention time of about 8.1 minutes and about 9.5 minutes respectively in the following HPLC analysis.

〈HPLC conditions〉

Column: Inertsil Prep ODS (6.0×250mm) manufactured by GL Sience Co., Ltd.
Mobile phase: 0.1% TFA aqueous solution:acetonitrile=6:4
Flow rate: 1 ml/min

A small amount of the mixture of the diastereomers was separated by HPLC under the above-mentioned conditions for the purpose of the anlysis. As a result, each diastereomer showed the following MNR spectram.

Diastereomer a'

$^1$H-NMR(DMSO-$d_6$) $\delta$ : 0.8 (3H, J=6.5Hz), 0.87 (6H, d, J=6.6Hz), 0.9-1.1 (1H, m), 1.3-1.6 (2H, m), 2.2-2.4 (1H, m), 2.57 (3H, d, J=4.6Hz), 2.6-2.8 (1H, m), 5.47 (1H, d, J=8.0Hz), 7.2-7.45 (5H, m), 8.12 (1H, d, J=4.6Hz), 8.73 (1H, d, J=8.0Hz), 12.16 (1H, bs).

Diastereomer b'

$^1$H-NMR(DMSO-$d_6$) $\delta$ : 0.82 (3H, d, J=6.5Hz), 0.86 (3H, d, J=6.4Hz), 0.95 (3H, d, J=7.1Hz), 1.0-1.15 (1H, m), 1.45-1.6 (2H, m), 2.57 (3H, d, J=4.6Hz), 2.5-2.6 (1H, m), 2.7-2.85 (1H, m), 5.39 (1H, d, J=7.8Hz), 7.2-7.4 (5H, m), 8.12 (1H, d, J=4.6Hz), 8.50 (1H, d, J=7.8Hz), 12.18 (1H, bs).

(2) [4-(N-Benzyloxyamino-2(R)-isobutyl-3-methylsuccinyl]-L-phenylglycine-N-methylamide (diastereomer a):

The compound obtained in the above (1) (11g) was dissolved in a mixture of DMF (150ml) and methylene chloride (200ml). To the mixture were added O-benzylhydroxyamine hydrochloride (8.0mg), hydroxybenzotriazole (5.5g), and triethylamine (5.1g), and further added WSC (7.8g), and the mixture was stirred overnight. The precipitated solid was collected by filtration and washed with diluted hydrochloric acid and then with a small amount of methanol to give one of optically active isomers of [4-(N-benzyloxyamino-2(R)-isobutyl-3-methylsuccinyl]-L-phenylglycine-N-methylamide (7.7g.)

Although in the reaction solution, there were obtained two stereoisomers (designated as diastereomer a and diastereomer b) which were eluted at a retention time of about 8.2 minutes and about 9.6 minutes respectively in the following HPLC analysis, the compound obtained in the above was one of them (diastereomer a).

〈HPLC conditions〉

Column: Inertsil Prep ODS (6.0×250mm) manufactured by GL Sience Co., Ltd.
Mobile phase: 0.1% TFA aqueous solution:acetonitrile=5:5
Flow rate: 1 ml/min
m.p.: 256-257°C (dec.)
$^1$H-NMR(DMSO-$d_6$) $\delta$ : 0.76 (3H, d, J=2.1Hz), 0.78 (3H, d, J=2.5Hz), 0.84 (3H, d, J=6.4Hz), 0.9 (1H, m), 1.3 (2H, m), 2.1 (1H, m), 2.56 (3H, d, J=4.5Hz), 2.66 (1H, m), 4.77 (2H, s), 5.46 (1H, d, J=8.0Hz), 7.23-7.42 (10H, m), 8.07 (1H, dd, J= 4.6Hz), 8.74 (1H, d, J=8.0Hz), 11.04 (1H, s).

| Analysis for $C_{25}H_{33}N_3O_4$ | | | |
|---|---|---|---|
| Calcud: | C, 68.31; | H, 7.57; | N, 9.56 |
| Found: | C, 68.08; | H, 7.49; | N, 9.51 |

(3) [4-Hydroxyamino-2(R)-isobutyl-3-methylsuccinyl]-L-phenylglycine-N-methylamide (Compound 4):

The diastereomer a obtained in the above (2) (7.7g) was suspended in DMF (500ml) and thereto was added 10% Pd/C (0.7g), and the mixture was stirred for four hours under hydrogen atmosphere. The catalyst was filtered off and the filtrate was concentrated in vacuo, and the residue was crystallized from methanol to give [4-hydroxyamino-2(R)-isobutyl-3-methylsuccinyl]-L-phenylglycine-N-methylamide (Compound 4) (5.2g).
m.p.: 217-219°C
$[\alpha]_D$=+136° (c=0.1, MeOH)

| Analysis for $C_{18}H_{27}N_3O_4$ | | | |
|---|---|---|---|
| Calcud: | C, 61.87; | H, 7.79; | N, 12.03 |
| Found: | C, 61.70; | H, 7.79; | N, 11.97 |

Preparation 20

Preparation of [4-(N-hydroxyamino)-2(R)-isobutyl-3(S)-methylsuccinyl]-L-phenylglycine-N-methylamide (Compound 4a).

(1) 4-tert-Butoxy-2(R)-isobutyl-3(S)-methylsuccinic acid:

N-(4-tert-Butoxy-2(R)-isobutyl-3(S)-methylsuccinyl)-4(S)-benzyl-2-oxazolidinone (0.98g) was dissolved in tetrahydrofuran (30ml) and thereto was added a solution of 30% aqueous hydrogen peroxide (1.1ml) and lithium hydroxide monohydrate (0.15g) in water (10ml) under ice cooling. The mixture was stirred under ice cooling for 13 hours and at room temperature for 4 hours. Sodium nitrite (1.1g) was added to the reaction mixture, and the mixture was stirred for 30 minutes. Tetrahydrofuran was distilled off under reduced pressure, and water (10ml) was added to the residue, and the mixture was washed with chloroform. Aqueous phase was adjusted to pH 4 by diluted hydrochloric acid, and the mixture was extracted by ethyl acetate (10ml) 3 times. The ethyl acetate solution was washed with saturated aqueous sodium chloride solution, and dried over magnesium sulfate. The mixture was concentrated in vacuo to give 4-tert-butoxy-2(R)-isobutyl-3(S)-methylsuccinic acid (0.45g) as a colorless oil.
$^1$H-NMR($CDCl_3$) $\delta$ : 0.90 (3H, d, J=6.5Hz), 0.91 (3H, d, J=6.5Hz), 1.16 (3H, d, J=6.9Hz), 1.2 (1H, m), 1.4 (9H, s), 1.6 (2H, m), 2.57 (1H, m), 2.7 (1H, m).

(2) [4-tert-Butoxy-2(R)-isobutyl-3(S)-methylsuccinyl]-L-phenylglycine-N-methylamide:

A mixture of N-benzyldxycarbonyl-L-phenylglycine-N-methylamide (cf. JP-A-101952/1995) (0.7g), methanol (30ml) and 10% Pd/C(0.3g) was stirred under hydrogen pressure (4kg/cm$^2$) for 1.5 hour. The catalyst was removed by filtration from the reaction mixture, and the mixture was concentrated in vacuo. The residue was dissolved in a mixture of methylene chloride (20ml) and dimethylformamide (10ml). To the mixture were added 4-tert-butoxy-2(R)-isobutyl-3(S)-methylsuccinic acid (0.45g) and HOBt (0.26g) , and further added WSC (0.42g) under ice cooling. The mixture was stirred for 30 minutes, and stirred further at room temperature overnight. The resulted solid material was filtered and washed with ether to give [4-tert-butoxy-2(R)-isobutyl-3(S)-methylsuccinyl]-L-phenylglycine-N-methylamide (0.3g) as a colorless solid. $^1$H-NMR($CDCl_3$) $\delta$ : 0.87 (3H, d, J=6.6Hz), 0.88 (3H, d, J=6.9Hz), 0.91 (3H, d, J=6.5Hz), 1.1 (1H, m), 1.42 (9H, s), 1.5 (1H, m), 1.7 (1H, m), 2.4 (1H, m), 2.5 (1H, m), 2.80 (3H, d, J=4.9Hz), 5.40 (1H, d, J=6.5Hz), 5.71 (1H, d, J=5.0Hz), 7.18 (1H, d, J=6.5Hz), 7.3 (5H, m).

(3) [4-(N-Benzyloxyamino)-2(R)-isobutyl-3(S)-methylsuccinyl]-L-phenylglycine-N-methylamide:

[4-tert-Butoxy-2(R)-isobutyl-3(S)-methylsuccinyl]-L-phenylglycine-N-methylamide (0.3g) was dissolved in formic acid (5ml), and the solution was stirred at room temperature for 2 hours and 40 minutes. Formic acid was distilled off under reduced pressure, and the residue was dissolved in a mixture of methylene chloride (10ml) and dimethylformamide (10ml). To the mixture were added O-benzylhydroxylamine hydrochloride (0.24g), triethylamine (0.15g) and WSC (0.17g) under ice cooling, and the mixture was stirred for 30 minutes, and further stirred at room temperature for 17 hours. The reaction mixture was concentrated in vacuo, and water added to the residue. The precipitated `solid material was filtered, and washed with water and ether to give [4-(N-benzyloxyamino)2-(R)-isobutyl-3(S)-methylsuccinyl]-L-phenylgliycine-N-methylamide (0.18g) as a colorless solid. The properties of this compound were the same as those of diastereomer a of Preparation 19 (2).

(4) [4-(N-Hydroxyamino)-2(R)-isobutyl-3(S)-methylsuccinyl]-L-phenylglycine-N-methylamide:

[4-(N-Benzyloxyamino)-2(R)-isobutyl-3(S)-methylsuccinyl]-L-phenylglycine-N-methylamide (0.17g), methanol (50ml) and 10% Pd/C (50mg) were stirred under hydrogen pressure ($4kg/cm^2$) at room temperature for 2 hours. The catalyst was removed by filtration from the reaction mixture, and the filtrate was concentrated in vacuo, and the residue was recrystallized from methanol to give [4-(N-hydroxyamino)-2(R)-isobutyl-3(S)-methylsuccinyl]-L-phenylglycine-N-methylamide (Compound 4a) (50mg) as colorless crystals.

The properties of this compound 4a were the same as those of the compound 4 of Preparation 19.

Preparation 21

Preparation of [4-(N-hydroxyamino)-2(R)-isobutyl-3(S)-methylsuccinyl]-L-cyclohexylglycine-N-methylamide (Compound 10).

(1) [4-Benzyloxy-3-benzyloxycarbonyl-2(R)-isobutylsuccinyl]-L-cyclohexylglycine-N-methylamide:

Methanol (50ml) and 10% Pd-C (300mg) were added to N-benzyloxycarbonyl-L-phenylglycine-N-methylamide (3.0g) (it was prepared by the method described in JP-A-101925/1995, and the mixture was stirred for one hour under hydrogen atmosphere. The catalyst was filtered off and to the filtrate was added 5% $Rh/Al_2O_3$ (1.0g), and the mixture was subjected to catalytic reduction under hydrogen pressure of $4.2kg/cm^2$. The catalyst was removed by filtration and methanol was distilled off. The residue was dissolved in DMF (20ml), and thereto were added 2-benzyloxycarbonyl-3(R)-hydroxycarbonyl-5-methylhexanic acid benzyl ester (4.4g), WSC (2.1g) and hydroxybenzotriazole (1.5g), and the mixture was stirred at room temperature overnight. A diluted HCl solution was added to the reaction mixture, and it was extracted with ethyl acetate. The organic layer was washed with water and a saturated aqueous sodium hydrogen carbonate solution and a saturated aqueous sodium chloride solution, and dried over magnesium sulfate. After removing magnesium sulfate, the solvent was distilled off. The residue was purified by silica gel column chromatography (chloroform:ethanol=20/1) to give [4-benzyloxy-3-benzyloxycarbonyl-2(R)-isobutylsuccinyl]-L-cyclohexylglycine-N-methylamide (3.0g).

$^1H$-NMR($CDCl_3$) $\delta$ : 0.75-0.85 (6H, m), 0.8-1.9 (14H, m), 2.7-2.8 (3H, m), 2.9-3.1 (1H, m), 3.80 (1H, d), 4.1-4.2 (1H, m), 5.0-5.2 (4H, m), 6.1-6.2 (1H, m), 6.5-6.6 (1H, d), 7.2-7.4 (10H, m).

(2) [4-Hydroxy-2(R)-isobutyl-3-methylenesuccinyl]-L-cyclohexylglycine-N-methylamide:

To a mixture of [4-benzyloxy-3-benzyloxycarbonyl-2(R)-isobutylsuccinyl]-L-cyclohexylglycine-N-methylamide (3.0g), ammonium formate (1.7g) and ethanol (30ml) was added 10% Pd-C (0.5g), and the mixture was stirred for 3 hours at room temparature. The catalyst was removed by filtering the reaction mixture, and to the filtrate were added piperidine (0.57g) and 37% formaldehyde solution (2.5ml), and the mixture was stirred at room temperature overnight. The reaction mixture was further refluxed for one hour, and the mixture was concentrated dryness in vacuo. 10% Citric acid solution was added to the residue, and it was extracted with ethyl acetate. The organic layer was extracted with an aqueous potassium carbonate solution and the extract was adjusted to pH 4 with diluted hydrochloric acid, and it was again extracted with ethyl acetate. The organic layer was washed with water and a saturated aqueous sodium chloride solution and dried over magnesium sulfate. After removing magnesium sulfate, the solvent was distilled off to give [4-hydroxy-2(R)-isobutyl-3-methylsuccinyl]-L-cyclohexylglycine-N-methylamide (1.4g).

$^1H$-NMR($CDCl_3$ + DMSO-$d_6$) $\delta$ : 0.86 (3H, d, J=6.2Hz), 0.90 (3H, d, J=6.2Hz), 0.8-1.8 (14H, m), 2.70 (3H, d, J=4.6Hz), 3.5-3.7 (1H, m), 4.18 (1H, t, J=7.4Hz), 5.76 (1H, s), 6.29 (1H, s), 7.25 (1H, bs), 7.66 (1H, bs).

(3) [4-(N-Hydroxyamino)-2(R)-isobutyl-3-methylsuccinyl]-L-cyclohexylglycine-N-methylamide:

To [4-hydroxy-2(R)-isobutyl-3-methylsuccinyl]-L-cyclohexylglycine-N-methylamide (1.4g) were added methanol (30ml) and 10% Pd-C (0.3g), and the mixture was stirred for 1.5 hour under hydrogen atmosphere. After the catalyst was removed by filtration, the solvent was distilled off. The residue was dissolved in DMF (30ml), and thereto were added O-benzylhydroxylamine hydrochloride (0.79g), triethylamine (0.50g), WSC (0.95g) and hydroxybenzotriazole (0.66g). The mixture was stirred at room temperature overnight, and the precipitated solid material was separated by filtration to give [4-(N-benzyloxyamino)-2(R)-isobutyl-3-methylsuccinyl]-L-cyclohexylglycine-N-methylamide (1.1g). To this solid material were added methanol (30ml), THF (30ml) and 10% Pd-C (0.2g), and the mixture was stirred for two hours under hydrogen atmosphere. After removing the catalyst by filtration, the solution was distilled off. The residue was recrystallized from methanol to give [4-(N-hydroxyamino)-2(R)-isobutyl-3-methylsuccinyl]-L-cyclohexylglycine-N-methylamide (0.12g).
$^1$H-NMR(DMSO-$d_6$) $\delta$ : 0.75 (3H, d, J=6.4Hz), 0.81 (3H, d, J=6.4Hz), 0.91 (3H, d, J=6.8Hz), 0.7-1.8 (14H, m), 2.0-2.2 (1H, m), 2.4-2.6 (1H, m), 2.55 (3H, d, J=4.5Hz), 4.03 (1H, t, J=8.4Hz), 7.6-7.7 (1H, m), 8.03 (1H, d, J=8.4Hz), 8.76 (1H, s), 10.45 (1H, s).

Preparation 22

Preparation of [4-(N-hydroxyamino)-2(R)-phenylethylsuccinyl]-L-phenylglycine-N-methylamide (Compound 88)

(1) 3-Phenylbutanoyl-4(s)-benzyl-2-oxazolidinone:

Thionyl chloride(15ml) was added to 4-phenylbutyric acid (5.0g), and the mixture was refluxed for one hour. After distilling off thionyl chloride, the residue was dissolved in tetrahydrofuran (10ml) to prepare an acid chloride solution. 4(S)-Benzyl-2-oxazolidinone (5.0g) was dissolved in tetrahydrofuran (50ml), and the solution was cooled to -78°C, and thereto was added dropwise n-butyllithium/n-hexane solution (1.6M) (18ml). After 30 minutes, to the mixture was added the acid chloride solution prepared above. The mixture was stirred at -78°C for 30 minutes and further at room temperature for 45 minutes. After the reaction, the reaction mixture was acidified with diluted hydrochloric acid, and the solution was extracted with ethyl acetate. The organic layer was washed with water, an aqueous potassium carbonate solution, water and a saturated aqueous sodium chloride solution, and dried over magnesium sulfate. The solvent was distilled off and the residue was purified by silica gel column chromatography (ethyl acetate:n-hexane=1:3) to give the title compound (7.4g).
$^1$H-NMR($CDCl_3$) $\delta$ : 1.96-2.10 (2H, m), 2.67-2.80 (3H, m), 2.87-3.09 (2H, m), 3.27 (1H, dd, J=3.3, 13.3Hz), 4.10-4.20 (2H, m), 4.59-4.68 (1H, m), 7.13-7.36 (10H, m).

(2) 3-[2(R)-t-Butoxycarbonylmethyl-4-phenylbutanoyl]-4(S)-benzyl-2-oxazolidinone:

Diisopropylamine (1.7g) was dissolved in tetrahydrofuran (30ml) and thereto was added dropwise n-butyllithium/n-hexane (1.6M) (10ml) at -78°C. To the mixture was further added dropwise a solution of 3-phenylbutanoyl-4(S)-benzyl-2-oxazolidinone (5.0g) in tetrahydrofuran (20ml), and the mixture was stirred for 40 minutes, and thereto was added bromoacetic acid t-butyl ester (4.0g), and the mixture was warmed to room temperature with stirring overnight. The reaction mixture was acidified with diluted hydrochloric acid and extracted with ethyl acetate. The organic layer was washed with water and a saturated aqueous sodium chloride solution and dried over magnesium sulfate. The solvent was distilled off and the residue was purified by silica gel column chromatography (ethyl acetate:n-hexane=1:3) to give the title compound (4.0g).
$^1$H-NMR ($CDCl_3$) $\delta$ : 1.43 (9H, s), 1.70-1.90 (1H, m), 1.97-2.12 (1H, m), 2.53 (1H, dd, J=4.5, 16.6Hz), 2.62-2.77 (3H, m), 2.87 (1H, dd, J=10.3, 16.6Hz), 3.32 (1H, dd, J=3.2, 13.4Hz), 4.03-4.18 (2H, m), 4.20-4.33 (1H, m), 4.48-4.59 (1H, m), 7.1-7.4 (10H, m).

(3) 4-t-Butoxy-2(R)-phenylethylsuccinic acid:

To 3-[2(R)-t-butoxycarbonylmethyl-4-phenylbutanoyl]-4(S)-benzyl-2-oxazolidinone (4.0g) were added tetrahydrofuran (100ml) and water (30ml), and thereto was further added a solution of 30% aqueous hydrogen peroxide solution (4.5ml) and lithium hydroxide monohydrate (650mg) in water (30ml), and the mixture was stirred for 1.5 hour. To the reaction mixture was added sodium nitrite (2.7g) and the mixture was stirred for 30 minutes, and tetrahydrofuran was distilled off, and the aqueous layer was washed with chloroform. The aqueous layer was acidified with diluted hydrochloric acid and extracted with ethyl acetate. The organic layer was washed with water and a saturated aqueous sodium chloride solution and dried over magnesium sulfate. The solvent was distilled off to give the title compound (2.5g).

$^1$H-NMR($CDCl_3$) δ : 1.43 (9H, s), 1.8-1.9 (1H, m), 1.9-2.1 (1H, m), 2.44 (1H, dd, J=5.2, 16.3Hz), 2.6-2.8 (3H, m), 2.8-2.9 (1H, m), 7.1-7.3 (5H, m).

(4) [4-t-Butoxy-2(R)-phenylethylsuccinyl]-L-phenylglycine-N-methylamide:

To N-benzyloxycarbonyl-L-phenylglycine-N-methylamide (860mg) (prepared by the method described in JP-A-101925/1995) were added methanol (20ml) and 10% Pd-C (300mg), and the mixture was stirred under hydrogen atmosphere for one hour. The catalyst was removed by filtration and the solvent was distilled off. To the residue were added DMF (10ml), 4-t-butoxy-2(R)-phenylethylsuccinic acid (800mg), WSC(550mg) and HOBt (390mg), and the mixture was stirred at room temperature overnight. The reaction mixture was acidified with diluted hydrochloric acid and extracted with ethyl acetate. The organic layer was washed with water, aqueous potassium carbonate solution, water and saturated aqueous sodium chloride solution successively and dried over magnesium sulfate. The solvent was distilled off and the residue was purified by silica gel column chromatography (ethyl acetate:n-hexane=1:1) to give the title compound (0.84g).
$^1$H-NMR($CDCl_3$) δ : 1.32 (9H, s), 1.6-1.8 (1H, m), 1.9-2.1 (1H, m), 2.33 (1H, dd, J=4.8, 16.4Hz), 2.5-2.8 (4H, m), 2.81 (3H, d, J=4.9Hz), 5.43 (1H, d, J=6.9Hz), 5.8-5.9 (1H, m), 7.0-7.4 (11H, m).

(5) [4-Hydroxy-2(R)-phenylethylsuccinyl]-L-phenylglycine-N-methylamide:

To [4-t-butoxy-2(R)-phenylethylsuccinyl]-L-phenylglycine-N-methylamide (0.84g) was added 90% aqueous trifluoroacetic acid solution (3ml), and the mixture was stirred under ice cooling for one hour. Trifluoroacetic acid was distilled off and the residue was extracted with chloroform. The organic layer was washed with water and saturated aqueous sodium chloride solution and dried over magnesium sulfate. The solvent was distilled off to give the title compound (0.58g).
$^1$H-NMR(DMSO-$d_6$) δ : 1.58-1.85 (2H, m), 2.30 (1H, dd, J=5.5, 16.4Hz), 2.44-2.70 (6H, m), 2.83-2.97 (1H, m), 5.43 (1H, d, J=7.7Hz), 7.10-7.44 (10H, m), 8.14-8.23 (1H, m), 8.47 (1H, d, J=7.8Hz), 12.0 (1H, bs).

(6) [4-(N-Hydroxyamino)-2(R)-phenylethylsuccinyl]-L-phenylglycine-N-methylamide:

To a mixture of [4-hydroxy-2(R)-phenylethylsuccinyl]-L-phenylglycine-N-methylamide (0.58g) and N-methylmorpholine (195mg) was added tetrahydrofuran (5ml) and further added ethyl chlorocarbonate (210mg) at - 10°C, and the mixture was stirred for 15 minutes. To the mixture was added a suspension of hydroxylamine hydrochloride (330mg) and N-methylmorpholine (480mg) in DMF (3ml), and the mixture was stirred for 1.5 hour. The reaction mixture was warmed to room temperature and acidified with diluted hydrochloric acid and then extracted with ethyl acetate. The organic layer was washed with water and saturated aqueous sodium chloride solution and dried over magnesium sulfate. The solvent was distilled off and the residue was purified by HPLC to give [4-(N-hydroxyamino)-2(R)-phenylethylsuccinyl]-L-phenylglycine-N-methylamide (137mg).
m.p.: 191°C-194°C
$^1$H-NMR(DMSO-$d_6$) δ : 1.55-1.80 (2H, m), 2.06 (1H, dd, J=7.5, 14.4Hz), 2.23 (1H, dd, J=7.0, 14.4Hz), 2.40-2.70 (5H, m), 2.82-2.98 (1H, m), 5.41 (1H, d, J=7.7Hz), 7.10-7.50 (10H, m), 8.12-8.22 (1H, m), 8.49 (1H, d, J=7.7Hz), 8.73 (1H, s), 10.40 (1H, s).

| Analysis for $C_{21}N_{25}N_{3}0_{4} \cdot 0.7H_2O$ | | | |
|---|---|---|---|
| Calcud: | C, 63.68; | H, 6.72; | N, 10.61 |
| Found: | C, 63.82; | H, 6.63; | N, 10.72 |

Preparation 23

Preparation of [4-(N-hydroxyamino)-2(R)-phenylethylsuccinyl]-L-3-(1-naphthyl)alanine-N-methylamide (Compound 89)

(1) [4-t-Butoxy-2(R)-phenylethylsuccinyl]-L-3-(1-naphthyl)alanine-N-methylamide:

To BOC-L-3-(1-naphthyl)alanine-N-methylamide (1.1g) which was prepared in the same manner as in Preparation 4) was added 90% aqueous trifluoroacetic acid solution (10ml) under ice cooling, and the mixture was stirred for 40 minutes. Trifluoroacetic acid was distilled off under reduced pressure and to the residue was added chloroform, and the mixture was washed with an aqueous potassium carbonate solution and dried over magnesium sulfate. The solvent was distilled off under reduced pressure and to the residue were added DMF (10ml), 4-t-butoxy-2(R)-phenylethylsuccinic acid (800mg), WSC (550mg) and HOBt (390mg), and the mixture was stirred at room temperature overnight. The reaction mixture was acidified with diluted hydrochloric acid and then extracted with ethyl acetate. The organic layer was washed with water, an aqueous potassium carbonate solution, water and a saturated aqueous sodium chloride solution and dried over magnesium sulfate. The solvent was distilled off and the residue was purified by silica gel column chromatography (ethyl acetate:n-hexane=1:1) to give the title compound (1.0g).
$^1$H-NMR($CDCl_3$) δ : 1.43 (9H, s), 1.60-1.80 (1H, m), 1.86-2.02 (1H, m), 2.36-2.70 (5H, m), 2.57 (3H, d, J=4.9Hz), 3.37 (1H, dd, J=9.2, 13.7Hz), 3.70 (1H, dd, J=5.9, 13.7Hz), 4.60-4.72 (1H, m), 5.22-5.33 (1H, m), 6.54 (1H, d, J=7.5Hz), 7.08-7.40 (7H, m), 7.47-7.55 (1H, m), 7.56-7.63 (1H, m), 7.75 (1H, d, J=7.7Hz), 7.85 (1H, d, J=8.1Hz), 8.31 (1H, J=8.1Hz).

(2) [4-Hydroxy-2(R)-phenylethylsuccinyl]-L-3-(1-naphthyl)alanine-N-methylamide:

The title compound (0.72g) was prepared from [4-t-butoxy-2(R)-phenylethylsuccinyl]-L-3-(1-naphthyl)alanine-N-methylamide (1.0g) in the same manner as described in Preparation 22 (5).
$^1$H-NMR(DMSO-$d_6$) δ : 1.55-1.75 (2H, m), 2.44 (1H, dd, J=6.4, 16.1Hz), 2.35-2.60 (6H, m), 2.60-2.75 (1H, m), 3.23-3.40 (1H, m), 3.46 (1H, dd, J=6.1, 13.9Hz), 4.53-4.67 (1H, m), 7.05-7.20 (3H, m), 7.20-7.30 (2H, m), 7.30-7.40 (2H, m), 7.46-7.62 (2H, m), 7.72-7.82 (2H, m), 7.87-7.94 (1H, m), 8.18-8.30 (2H, m), 12.16 (1H, s).

(3) [4-(N-Hydroxyamino)-2(R)-phenylethylsuccinyl]-L-3-(1-naphthyl)alanine-N-methylamide:

[4-Hydroxy-2(R)-phenylethylsuccinyl]-L-3-(1-naphthyl)alanine-N-methylamide (0.40g) was reacted in the same manner as described in Preparation 22 (6), followed by crystallizing from a mixed solvent of ethanol and ether to give the title compound (40mg) as colorless crystals.
m.p.: 212°C-214°C
$^1$H-NMR(DMSO-$d_6$) δ : 1.45-1.72 (2H, m), 1.99 (1H, dd, J=7.7, 14.6Hz), 2.13 (1H, dd, J=7.0, 14.6Hz), 2.25-2.40 (2H, m), 2.40-2.65 (1H, m), 2.58 (3H, d, J=4.5Hz), 3.20-3.40 (1H, m), 3.57 (1H, dd, J=5.2, 14.0Hz), 4.50-4.65 (1H, m), 7.00-7.40 (7H, m), 7.45-7.62 (2H, m), 7.70-7.80 (1H, m), 7.91 (1H, d, J=7.0Hz), 7.95-8.03 (1H, m), 8.23 (2H, d, J=8.1Hz), 8.79 (1H, s), 10.44 (1H, s).

Preparation 24

Preparation of [4-(N-hydroxyamino)-2(R)-phenylpropylsuccinyl]-L-3-(1-naphthyl)alanine-N-methylamide (Compound 90)

(1) 3-Phenylpentanoyl-4(S)-benzyl-2-oxazolidinone:

Thionyl chloride (15ml) was added to 5-phenylpentanoic acid (3.9g) and the mixture was refluxed from one hour. Thionyl chloride was distilled off and the residue was dissolved in tetrahydrofuran (20ml) to prepare an acid chloride solution. 4(S)-Benzyl-2-oxazolidinone (3.9g) was dissolved in tetrahydrofuran (50ml) and cooled to -78°C and thereto was added dropwise n-butyllithium/n-hexane (1.6 M) (14ml). After 10 minutes, to the mixture was added the acid chloride solution prepared above, and the mixture was stirred at -78°C for 25 minutes and further at room temperature for 30 minutes. The reaction mixture was acidified with diluted hydrochloric acid and extracted with ethyl acetate. The organic layer was washed with water, an aqueous potassium carbonate solution, water and a saturated aqueous sodium chloride solution and dried over magnesium sulfate. The solvent was distilled off and the residue was purified by silica gel column chromatography (ethyl acetate:n-hexane=1:3) to give the title compound (4.7g).
$^1$H-NMR($CDCl_3$) δ : 1.80-2.00 (4H, m), 2.75-2.95 (3H, m), 3.00-3.20 (2H, m), 3.41 (1H, dd, J=3.3, 13.3Hz), 4.25-4.35 (2H, m), 4.75-4.85 (1H, m), 7.20-7.50 (10H, m).

(2) 3-[2(R)-t-Butoxycarbonylmethyl-4-phenylpentanoyl]-4(S)-benzyl-2-oxazolidinone:

Diisopropylamine (1.5g) was dissolved in tetrahydrofuran (30ml) and thereto was added dropwise n-butyllithium/n-hexane (1.6M) (9.3ml) at -78°C. To the mixture was further added dropwise a solution of 3-phenylpentanoyl-4(S)-benzyl-2-oxazolidinone (4.7g) in tetrahydrofuran (10ml), and the mixture was stirred for 40 minutes, and thereto was added bromoacetic acid t-butyl ester (3.5g), and the mixture was warmed to room temperature with stirring overnight. After the reaction, the reaction mixture was acidified with diluted hydrochloric acid and extracted with ethyl acetate. The organic

layer was washed with water and a saturated aqueous sodium chloride solution and dried over magnesium sulfate. The solvent was distilled off and the residue was purified by silica gel column chromatography (ethyl acetate:n-hexane=1:3) to give the title compound (3.3g).
$^1$H-NMR($CDCl_3$) δ : 1.41 (9H, s), 1.20-1.80 (4H, m), 2.40-2.87 (5H, m), 3.34 (1H, dd, J=3.0, 13.5Hz), 4.00-4.27 (3H, m), 4.58-4.70 (1H, m), 7.10-7.40 (10H, m).

(3) 4-t-Butoxy-2(R)-phenylpropylsuccinic acid:

To 3-[2(R)-t-butoxycarbonylmethyl-4-phenylpentanoyl]-4(S)-benzyl-2-oxazolidinone (3.3g) were added tetrahydrofuran (75ml) and water (25ml), and thereto was further added a solution of 30% aqueous hydrogen peroxide solution (3.6ml) and lithium hydroxide monohydrate (520mg) in water (30ml), and the mixture was stirred for 1.5 hour. To the reaction mixture was added sodium nitrite (2.5g) and the mixture was stirred for 30 minutes, and tetrahydrofuran was distilled off, and the aqueous layer was washed with chloroform. The aqueous layer was acidified with diluted hydrochloric acid and extracted with ethyl acetate. The organic layer was washed with water and a saturated aqueous sodium chloride solution and dried over magnesium sulfate. After removing magnesium sulfate, the solvent was distilled off to give the title compound (1.6g).
$^1$H-NMR($CDCl_3$) δ : 1.41 (9H, s), 1.50-1.80 (4H, m), 2.36 (1H, d, J=5.2, 16.4Hz), 2.55-2.68 (3H, m), 2.75-2.90 (1H, m), 7.10-7.30 (5H, m).

(4) [4-t-Butoxy-2(R)-phenylpropylsuccinyl]-L-3-(1-naphthyl)alanine-N-methylamide:

To BOC-L-3-(1-naphthyl)alanine-N-methylamide (950mg) was added 90% aqueous trifluoroacetic acid solution (10ml) under ice cooling, and the mixture was stirred for 40 minutes. Trifluoroacetic acid was distilled off under reduced pressure and to the residue was added chloroform, and the mixture was washed with an aqueous potassium carbonate solution and dried over magnesium sulfate. After removing magnesium sulfate, the solvent was distilled off and to the residue were added DMF (10ml), 4-t-butoxy-2(R)-phenylpropylsuccinic acid (700mg), WSC (460mg) and HOBt (325mg), and the mixture was stirred at room temperature overnight. The reaction mixture was acidified with diluted hydrochloric acid and then extracted with ethyl acetate. The organic layer was washed with water, an aqueous potassium carbonate solution, water and a saturated aqueous sodium chloride solution and dried over magnesium sulfate. The solvent was distilled off and the residue was purified by silica gel column chromatography (ethyl acetate:n-hexane=1:1) to give the title compound (510mg).
$^1$H-NMR($CDCl_3$) δ : 1.42 (9H, s), 1.50-1.70 (4H, m), 2.32-2.66 (5H, m), 2.52 (3H, d, J=4.8Hz), 3.35 (1H, dd, J=9.1, 13.7Hz), 3.69 (1H, dd, J=5.8, 13.7Hz), 4.58-4.70 (1H, m), 5.20-5.30 (1H, m), 6.51 (1H, d, J=7.5Hz), 7.10-7.40 (7H, m), 7.47-7.63 (2H, m), 7.75 (1H, d, J=7.5Hz), 7.86 (1H, d, J=8.0Hz), 8.32 (1H, d, J=8.4Hz).

(5) [4-Hydroxy-2(R)-phenylpropylsuccinyl]-L-3-(1-naphthyl)alanine-N-methylamide:

The title compound (400mg) was prepared from [4-t-butoxy-2(R)-phenylpropylsuccinyl]-L-3-(1-naphthyl)alanine-N-methylamide (510mg) in the same manner as described in Preparation 22 (5).
$^1$H-NMR(DMSO-$d_6$) δ : 1.20-1.52 (4H, m), 2.17 (1H, dd, J=6.4, 16.2Hz), 2.36 (1H, dd, J=7.9, 16.2Hz), 2.40-2.60 (5H, m), 2.60-2.70 (1H, m), 3.20-3.60 (2H, m), 4.48-4.62 (1H, m), 7.08-7.20 (3H, m), 7.20-7.30 (2H, m), 7.30-7.42 (2H, m), 7.46-7.62 (2H, m), 7.67-7.81 (2H, m), 7.87-7.92 (1H, m), 8.17-8.28 (2H, m).

(6) [4-(N-Hydroxyamino)-2(R)-phenylpropylsuccinyl]-L-3-(1-naphthyl)alanine-N-methylamide:

[4-Hydroxy-2(R)-phenylpropylsuccinyl]-L-3-(1-naphthyl)alanine-N-methylamide (400mg) was reacted in the same manner as described in Preparation 22 (6), followed by crystallizing from isopropyl alcohol to give the title compound (183mg) as colorless crystals.
m.p.: 193°C-194°C
$^1$H-NMR(DMSO-$d_6$) δ : 1.20-1.45 (4H, m), 1.95 (1H, dd, J=7.3, 14.5Hz), 2.12 (1H, dd, J=7.0, 14.5Hz), 2.30-2.60 (6H, m), 3.25 (1H, dd, J=9.1, 14.0Hz), 3.57 (1H, dd, J=4.3, 14.0Hz), 4.42-4.58 (1H, m), 7.04-7.30 (5H, m), 7.30-7.40 (2H, m), 7.45-7.62 (2H, m), 7.70-7.80 (1H, m), 7.80-8.00 (2H, m), 8.10-8.30 (2H, m), 8.79 (1H, s), 10.43 (1H, s).

| Analysis for $C_{27}H_{31}N_{3}O_{4}$ | | | |
|---|---|---|---|
| Calcud: | C, 70.26; | H, 6.77; | N, 9.10 |

(continued)

| Analysis for $C_{27}H_{31}N_{3}0_{4}$ | | | |
|---|---|---|---|
| Found: | C, 70.39; | H, 6.86; | N, 9.10 |

Preparation 25

Preparation of [4-(N-hydroxyamino)-2(R)-phenylpropylsuccinyl]-L-phenylglycine-N-methylamide (Compound 91)

(1) [4-t-Butoxy-2(R)-phenylpropylsuccinyl]-L-phenylglycine-N-methylamide:

To N-benzyloxycarbonyl-L-phenylglycine-N-methylamide (1.06g) were added methanol (30ml) and 10% Pd-C (200mg), and the mixture was stirred under hydrogen atmosphere. After 3 hours, Pd-C was removed and the solvent was distilled off. To the residue were added DMF (7ml), 4-t-butoxy-2(R)-phenylpropylsuccinic acid (870mg), WSC (580mg) and HOBt (410mg), and the mixture was stirred at room temperature overnight. The reaction mixture was acidified with diluted hydrochloric acid and then extracted with ethyl acetate. The organic layer was washed with water, an aqueous potassium carbonate solution, water and a saturated aqueous sodium chloride solution and dried over magnesium sulfate. The solvent was distilled off and the residue was purified by silica gel column chromatography (ethyl acetate:n-hexane=1:1) to give the title compound (0.83g).
$^{1}$H-NMR($CDCl_3$) $\delta$ : 1.31 (9H, s), 1.40-1.52 (1H, m), 1.55-1.80 (3H, m), 2.29 (1H, dd, J=4.9, 16.5Hz), 2.50 (1H, dd, J=9.1, 16.5Hz), 2.53-2.70 (3H, m), 2.78 (3H, d, J=4.9Hz), 5.41 (1H, d, J=6.9Hz), 5.90-6.00 (1H, m), 7.02-7.38 (11H, m).

(2) [4-Hydroxy-2(R)-phenylpropylsuccinyl]-L-phenylglycine-N-methylamide:

The title compound (0.56g) was prepared from [4-t-butoxy-2(R)-phenylpropylsuccinyl]-L-phenylglycine-N-methylamide (0.83g) in the same manner as described in Preparation 22 (5).
$^{1}$H-NMR(DMSO-$d_6$) $\delta$ : 1.32-1.62 (4H, m), 2.22 (1H, dd, J=5.8, 16.4Hz), 2.43 (1H, dd, J=8.7, 16.4Hz), 2.45-2.70 (2H, m), 2.57 (3H, d, J=4.5Hz), 2.80-2.92 (1H, m), 5.41 (1H, d, J=7.6Hz), 7.12-7.42 (10H, m), 8.12-8.20 (1H, m), 8.48 (1H, d, J=8.3Hz), 12.04 (1H, s).

(3) [4-(N-Hydroxyamino)-2(R)-phenylpropylsuccinyl]-L-phenylglycine-N-methylamide:

[4-Hydroxy-2(R)-phenylpropylsuccinyl]-L-phenylglycine-N-methylamide (0.56g) was reacted in the same manner as described in Preparation 22 (6), followed by crystallizing from isopropyl alcohol to give the title compound (0.12g) as colorless crystals.
m.p.: 176°C-179°C
$^{1}$H-NMR(DMSO-$d_6$) $\delta$ : 1.30-1.60 (4H, m), 1.99 (1H, dd, J=7.2, 14.5Hz), 2.17 (1H, dd, J=7.1, 14.5Hz), 2.45-2.65 (2H, m), 2.56 (3H, d, J=4.4Hz), 2.80-2.93 (1H, m), 5.39 (1H, d, J=7.8Hz), 7.10-7.43 (10H, m), 8.08-8.20 (1H, m), 8.46 (1H, d, J=7.8Hz), 8.71 (1H, s), 10.36 (1H, s).

| Analysis for $C_{22}H_{27}N_{3}0_{4}$ | | | |
|---|---|---|---|
| Calcud: | C, 66.48; | H, 6.85; | N, 10.57 |
| Found: | C, 66.48; | H, 6.99; | N, 10.54 |

Example 1

Preparation of tablets

Tablets containing [4-(hydroxyamino)-2(R)-isobutyl-3(S)-methylsnccinyl]-L-phenylglycine-N-methylamide (100mg) are prepared as follows.

[Formulation]

| Components | Amount |
|---|---|
| Active substance (Compound 4a) | 100 parts by weight |
| Corn starch | 46 parts by weight |
| Microcrystalline Cellulose | 98 parts by weight |
| Hydroxypropyl Cellulose | 2 parts by weight |
| Magnesium Stearate | 4 parts by weight |

[Procedure]

The active substance, corn starch and microcrystalline cellulose are mixed and thereto is added a solution of hydroxypropyl cellulose in water (50 parts by weight), and the mixture is kneeded well. The kneeded mixture is passed through a screen to form granules and dried. The granules thus obtained are mixed with magnesium stearate and the mixture is tableted to give tablets (weight of each tablet: 250 mg).

Example 2

Preparation of granules

Granules containing [4-(hydroxyamino)-2(R)-isobutyl-3(S)-methylsuccinyl]-L-phenylglycine-N-metylamide (200 mg) are prepared as follows.

[Formulation]

| Components | Amount |
|---|---|
| Active substance (Compound 4a) | 200 parts by weight |
| Lactose | 185 parts by weight |
| Corn starch | 109 parts by weight |
| Hydroxypropyl Cellulose | 6 parts by weight |

[Procedure]

The active substance, lactose and corn starch are mixed and thereto is added a solution of hydroxypropyl cellulose in water (120 parts by weight), and the mixture is kneeded well. The kneeded mixture is passed through a 20 mesh screen to granulate, dried and regulate the shape and size to give the desired granules.

Example 3

Preparation of capsules

Capsules containing [4-(hydroxyamino)-2(R)-isobutyl-3(S)-methylsuccinyl]-L-phenylglycine-N-metylamide (100mg) per one capsule are prepared as follows.

| Components | Amount |
|---|---|
| Active substance (Compound 4a) | 100 parts by weight |
| Lactose | 35 parts by weight |
| Corn starch | 60 parts by weight |
| Magnesium Stearate | 5 parts by weight |

[Procedure]

The above components are mixed well, and the mixed powder is fulfilled in a capsule (content: 200mg per each capsule).

Example 4

Preparation of injections

A mixture of [4-(hydroxyamino)-2(R)-isobutyl-3(S)-methylsuccinyl]-L-phenylglycine-N-metylamide (0.5 parts by weight) and sorbitol (5 parts by weight) is dissolved in distilled water for injection to give an aqueous solution (100 parts by weight) and the aqueous solution is filtered with a membrane filter. The filtrate (each 5 g) is fulfilled in an ampoule discharged with nitrogen gas. After melt sealing the ampoule, it is sterilized at 120°C for 15 minutes to give an injection preparation containing [4-(hydroxyamino)-2(R)-isobutyl-3(S)-methylsuccinyl]-L-phenylglycine-N-metylamide (25 mg per ampoule).

**Claims**

1. A Fas ligand solubilization inhibitor which comprises a compound having a matrix metalloproteinase inhibitory activity as an active ingredient.

2. A Fas ligand solubilization inhibitor comprising, as an active ingredient, a compound of the generic formula (I)

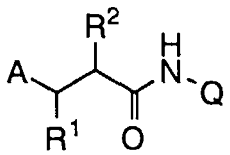

(I)

wherein Q is a group selected from the following formula (II)-(V)

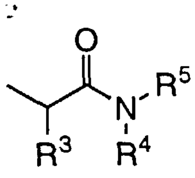

(II)

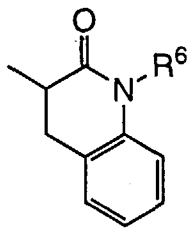

(III)

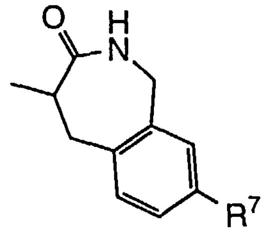

(IV)

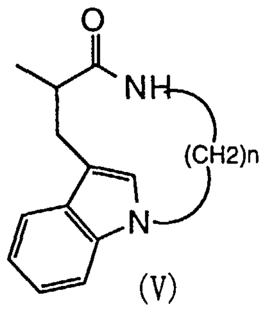

(V)

wherein A is N-hydroxyaminocarbonyl, carboxyl, mercapto, or phosphono; $R^1$ is hydrogen, amino, hydroxy, mercapto, $C_1$-$C_6$ alkoxy, $C_1$-$C_6$ alkylthio, $C_1$-$C_6$ alkyl, $C_2$-$C_6$ alkenyl, $C_6$-$C_{12}$ aryl, $C_6$-$C_{12}$ aryl($C_1$-$C_6$ alkyl), ($C_1$-$C_6$ alkyl)thio($C_1$-$C_6$ alkyl), ($C_1$-$C_6$ alkanoyl)thio($C_1$-$C_6$ alkyl), $C_6$-$C_{12}$ arylthio( $C_1$-$C_6$ alkyl), heterocyclylthio($C_1$-$C_6$ alkyl), ($C_1$-$C_6$ alkyl)sulfinyl($C_1$-$C_6$ alkyl), $C_6$-$C_{12}$ arylsulfinyl($C_1$-$C_6$ alkyl), heterocyclylsulfinyl ($C_1$-$C_6$ alkyl), ($C_1$-$C_6$ alkyl)sulfonyl($C_1$-$C_6$ alkyl), ($C_6$-$C_{12}$ aryl)sulfonyl($C_1$-$C_6$ alkyl), heterocyclylsulfonyl($C_1$-$C_6$ alkyl), $C_1$-$C_6$ alkyl substituted by -$NR^{11}R^{12}$, wherein $R^{11}$ and $R^{12}$ are the same or different and are hydrogen or $C_1$-$C_6$ alkyl, or may constitute a saturated or unsaturated heterocyclic ring with the adjacent nitrogen atom, or $C_1$-$C_6$ alkyl which may be substituted by a functional group selected from a group consisting of amino, hydroxy, mercapto and carboxyl of which the functional group may be chemically protected; $R^2$ is hydrogen, $C_1$-$C_6$ alkyl, $C_2$-$C_6$ alkenyl, $C_6$-$C_{12}$ aryl, $C_6$-$C_{12}$ aryl($C_1$-$C_6$ alkyl), heterocyclyl($C_1$-$C_6$ alkyl), cycloalkyl($C_1$-$C_6$ alkyl), $C_1$-$C_6$ alkylthio, $C_1$-$C_6$ alkylsulfinyl, $C_1$-$C_6$ alkylsulfonyl, $C_6$-$C_{12}$ aryl($C_1$-$C_6$ alkyl)thio, ($C_1$-$C_6$ alkyl)thio($C_1$-$C_6$ alkyl), or ($C_1$-$C_6$ alkoxy) $C_1$-$C_6$ alkyl; $R^3$ is $C_1$-$C_6$ alkyl, $C_2$-$C_6$ alkenyl, $C_6$-$C_{12}$ aryl($C_1$-$C_6$ alkyl), $C_6$-$C_{12}$ aryl, heterocyclyl, heterocyclyl($C_1$-$C_6$ alkyl), bicycloaryl, bicycloaryl($C_1$-$C_6$ alkyl), cycloalkyl($C_1$-$C_6$ alkyl), cycloalkyl, glycosyloxy($C_1$-$C_6$ alkyl), or tetrahydronaphthylmethyl; $R^4$ is hydrogen, $C_1$-$C_6$ alkyl, $C_1$-$C_6$ alkoxy($C_1$-$C_6$ alkyl), [di($C_1$-$C_6$ alkoxy)$C_1$-$C_6$ alkyl]methyl, [tri($C_1$-$C_6$ alkoxy)$C_1$-$C_6$ alkyl]methyl, hydroxy($C_1$-$C_6$ alkyl), [dihydroxy($C_1$-$C_6$ alkyl)]methyl, [trihydroxy($C_1$-$C_6$ alkyl)]methyl, amino($C_1$-$C_6$ alkyl), substituted amino($C_1$-$C_6$ alkyl), carboxy($C_1$-$C_6$ alkyl), $C_1$-$C_6$ alkoxycarbonyl($C_1$-$C_6$ alkyl), carbamoyl($C_1$-$C_6$ alkyl), substituted carbamoyl($C_1$-$C_6$ alkyl), dihydroxyphosphonyl($C_1$-$C_6$ alkyl), di($C_1$-$C_6$ alkoxy)phosphonyl($C_1$-$C_6$)alkyl, $C_6$-$C_{12}$ aryl, $C_6$-$C_{12}$ aryl($C_1$-$C_6$ alkyl), pyrrolidino($C_1$-$C_6$ alkyl), piperidino($C_1$-$C_6$ alkyl), morpholino($C_1$-$C_6$ alkyl), glycosyloxy($C_1$-$C_6$ alkyl), -$CH(R^{13})COOR^{14}$, wherein $R^{13}$ is a side chain of $\alpha$-amino acid, and $R^{14}$ is $C_1$-$C_6$ alkyl, -$CH(R^{13})CONR^{15}R^{16}$, wherein $R^{13}$ is as defined above, $R^{15}$ and $R^{16}$ are the same or different and are hydrogen, or $C_1$-$C_6$ alkyl, $C_1$-$C_6$ alkoxy which may be substituted by hydroxy, amino or carboxyl, morpholinosulfonylamino($C_1$-$C_6$ alkyl), ($C_1$-$C_6$ alkyl)aminosulfonylamino($C_1$-$C_6$ alkyl), di($C_1$-$C_6$ alkyl) aminosulfonylamino($C_1$-$C_6$ alkyl), or aminosulfonyl $C_6$-$C_{12}$ aryl ($C_1$-$C_6$ alkyl); $R^5$ is hydrogen, or $C_1$-$C_6$ alkyl; and $R^4$ and $R^5$ may constitute a saturated or unsaturated cyclic amine with the adjacent nitrogen atom, of which the cyclic amine may contain an oxygen or sulfur atom; $R^6$ is hydrogen, hydroxy, $C_1$-$C_6$ alkoxy, or ($C_1$-$C_6$ alkoxy) $C_1$-$C_6$ alkoxy; and $R^7$ is hydrogen, hydroxy, or methoxy; and n is interger 5-7, or its pharmaceutically acceptable salt.

**3.** A Fas ligand solubilization inhibitor clamed in the claim 2 wherein N is hydroxyaminocarbonyl, or its pharmaceutically acceptable salt.

**4.** A Fas ligand solubilization inhibitor clamed in the claim 2 wherein Q is a group (II), and $R^1$ is hydrogen, $C_1$-$C_6$ alkyl, phenylthio $C_1$-$C_6$ alkyl, or $C_1$-$C_6$ alkyl substituted by -$NR^{11}R^{12}$, wherein $R^{11}$ and $R^{12}$ are the same or different and are hydrogen or $C_1$-$C_6$ alkyl, or may constitute a saturated or unsaturated heterocyclic ring with the adjacent nitrogen atom; $R^2$ is $C_1$-$C_6$ alkyl, or $C_6$-$C_{12}$ aryl($C_1$-$C_6$ alkyl); $R^3$ is cyclohexyl, $C_6$-$C_{12}$ aryl, ($C_6$-$C_{12}$ aryl) $C_1$-$C_6$ alkyl,

benzothienyl, or tetrahydronaphthylmethyl; and $R^2$ and $R^5$ are the same or different and are hydrogen, $C_1$-$C_6$ alkyl, or aminosulfonyl($C_6$-$C_{12}$ aryl) $C_1$-$C_6$ alkyl,
or its pharmaceutically acceptable salt.

5. A pharmaceutical composition for preventing and treating the diseases caused by the excessively activated T cell, comprising, as an active ingredient, a Fas ligand solubilization inhibitor clamed in any of the claims 1-4.

6. A pharmaceutical composition for preventing and treating hepatitis, comprising, as an active ingredient, a Fas ligand solubilization inhibitor clamed in any of the claims 1-4.

7. A pharmaceutical composition for preventing and treating graft-versus-host diseases (GVHD), comprising, as an active ingredient, a Fas ligand solubilization inhibitor clamed in any of the claims 1-4.

8. A pharmaceutical composition for preventing and treating autoimmune diseases, comprising, as an active ingredient, a Fas ligand solubilization inhibitor clamed in any of the claims 1-4.

9. A pharmaceutical composition for preventing and treating acquired immunodeficiency syndrome (AIDS), comprising, as an active ingredient, a Fas ligand solubilization inhibitor clamed in any of the claims 1-4.

10. A reagent for the inhibition of a Fas ligand solubilization for the test and study of Fas ligand, comprising a matrix metalloproteinase as an active ingredient.

11. A reagent for the inhibition of a Fas ligand solubilization claimed in claim 10, comprising, as an active ingredient, a compound of the following formula (VIa)

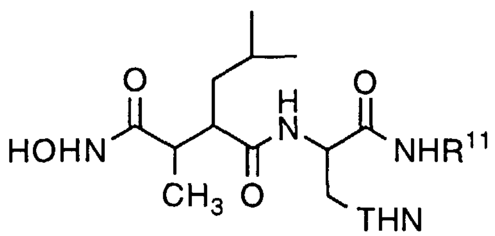

(VIa)

wherein $R^{11}$ is a $C_1$-$C_6$ alkyl, and THN is 5,6,7,8-tetrahydro-1-naphthyl or 5,6,7,8-tetrahydro-2-naphthyl, or its salt.

12. A compound of the following formula (VIa)

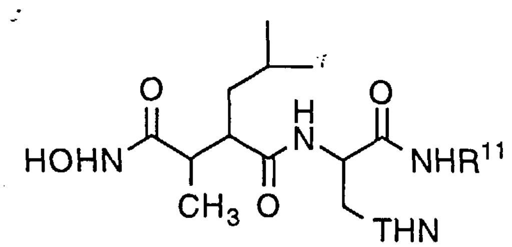

(VIa)

wherein $R^{11}$ is a $C_1$-$C_6$ alkyl, and THN is 5,6,7,8-tetrahydro-1-naphthyl or 5,6,7,8-tetrahydro-2-naphthyl, or its salt.

**INTERNATIONAL SEARCH REPORT**

International application No.
PCT/JP96/02492

A. CLASSIFICATION OF SUBJECT MATTER
Int. Cl$^6$ A61K45/00, A61K31/165, A61K31/47, A61K31/55, C07D487/04, C07C239/18
According to International Patent Classification (IPC) or to both national classification and IPC

B. FIELDS SEARCHED
Minimum documentation searched (classification system followed by classification symbols)
Int. Cl$^6$ C61K45/00, A61K31/165, A61K31/47, A61K31/55, C07D487/04, C07C239/18

Documentation searched other than minimum documentation to the extent that such documents are included in the fields searched

Electronic data base consulted during the international search (name of data base and, where practicable, search terms used)
CAS ONLINE

C. DOCUMENTS CONSIDERED TO BE RELEVANT

| Category* | Citation of document, with indication, where appropriate, of the relevant passages | Relevant to claim No. |
|---|---|---|
| P,X | KAYAGAKI, NOBUHIKO. Metalloproteinase-mediated release of Fas ligand. J. Exp. Med. December 1995, Vol. 182, No. 6, pages 1777-1783 | 1, 10 |
| P,X | KAYAGAKI, NOBUHIKO. Matrix-metalloproteinase mediated of human Fas ligand. Immunol. Front. 1996, Vol. 6, No. 3, pages 173-177 | 1 - 11 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| December 2, 1996 (02. 12. 96) | December 17, 1996 (17. 12. 96) |
| Name and mailing address of the ISA/ Japanese Patent Office | Authorized officer |
| Facsimile No. | Telephone No. |

Form PCT/ISA/210 (second sheet) (July 1992)